# EUROPÄISCHE PATENTSCHRIFT

(11) **EP 3 169 674 B1**
(45) Veröffentlichungstag und Bekanntmachung des Hinweises auf die Patenterteilung: **20.01.2021**
(21) Anmeldenummer: 15735973.8
(22) Anmeldetag: 13.07.2015
(51) Int. Cl.: C07D 401/04, A01N 43/647, A61P 33/00

(54) **ARYL-TRIAZOLYL-PYRIDINE ALS SCHÄDLINGSBEKÄMPFUNGSMITTEL**
ARYL-TRIAZOLYL-PYRIDINES AS PESTICIDES
ARYLE-TRIAZOLYLE-PYRIDINES EN TANT QUE PESTICIDES

(30) Priorität: 15.07.2014 EP 14177112
(43) Veröffentlichungstag der Anmeldung: 24.05.2017
(73) Patentinhaber: Bayer Animal Health GmbH, 51373 Leverkusen (DE)
(72) Erfinder: HALLENBACH, Werner, 40789 Monheim (DE); SCHWARZ, Hans-Georg, 46282 Dorsten (DE); GÖRGENS, Ulrich, 40882 Ratingen (DE); ILG, Kerstin, 50670 Köln (DE); TURBERG, Andreas, 42781 Haan (DE); HORSTMANN, Sebastian, 51381 Leverkusen (DE); KÖHLER, Adeline, 40764 Langenfeld (DE)
(74) Vertreter: Tier 1 Patents
(86) Internationale Anmeldenummer: PCT/EP2015/065933
(87) Internationale Veröffentlichungsnummer: WO 2016/008830

(56) Entgegenhaltungen:
- WO-A1-2012/175474

## Beschreibung

Die vorliegende Anmeldung betrifft neue Pyrazolyl-triazolyl-pyridine, Verfahren zu ihrer Herstellung und ihre Verwendung zur Bekämpfung von tierischen Schädlingen, vor allem von Arthropoden und insbesondere von Insekten und Spinnentieren.

WO2012175474-A1 beschreibt bestimmte Phenyl-triazolyl-pyridine als insektizide Verbindungen. Dabei umfasst die allgemeine Formel (A) in ihren Definitionen für A¹ bis A⁴ CX (X steht für Wasserstoff, Halogen, Cyano, C₁-C₄-Alkyloxy, C₁-C₄-Alkyl oder C₁-C₄-Haloalkyl) oder Stickstoff, wobei Q² bestimmte Phenyl-Substituenten bedeuten. Allerdings wird in den Vorzugsbereichen und in den Beispielen A¹, A² oder A³ = Stickstoff nicht weiter ausgeführt.

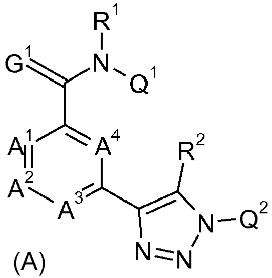

Moderne Pflanzenschutzmittel und veterinärmedizinische Ectoparasitizide müssen vielen Anforderungen genügen, beispielsweise in Bezug auf Dosierung, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln oder veterinärmedizinisch wirksamen Parasitiziden nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass Phenyl-triazolyl-pyridine sowie deren *N-*Oxide und Salze dem Stand der Technik überlegene biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

### Zusammenfassung

Ein Aspekt der vorliegenden Erfindung bezieht sich auf Verbindungen der Formel (I) 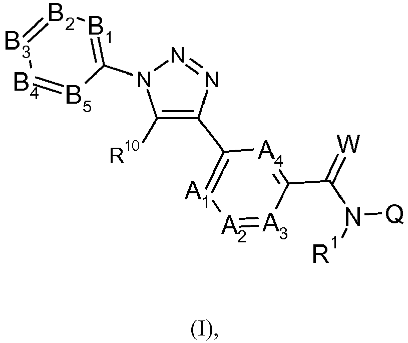 in denen
- R¹: für Wasserstoff, oder eine gegebenenfalls substituierte Gruppe ausgewählt aus C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl-C₁-C₆-alkyl, Heteroaryl-C₁-C₆-alkyl steht;
die chemischen Gruppierungen
- A₁: für CR²
- A₂: für Stickstoff,
- A₃: für CR³ und
- A₄: für CR⁴ stehen,
- B₁: für CR⁵ oder N,
- B₂: für CR⁶ oder N,
- B₃: für CR⁷ oder N,
- B₄: für CR⁸ oder N, und
- B₅: für CR⁹ oder N stehen,
wobei aber nicht mehr als zwei der Gruppierungen B₁ bis B₅ gleichzeitig für Stickstoff stehen;
- R² und R⁴: unabhängig voneinander für Wasserstoff oder C₁-C₄-Alkyl stehen,
- R³: für Halogen, C₁-C₄-Alkyl, C₁-C₄-Halogenalkyl oder C₁-C₄-Alkoxy steht,
- R⁵, R⁶, R⁸ und R⁹: unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino oder *N,N*-Di-(C₁-C₆-alkyl)amino, C₁-C₆-Alkylsulfonyl-amino, *N-*(C₁-C₆-Alkyl)-C₁-C₆-alkylsulfonyl-amino, Phenyl stehen;
- R⁷: für Halogen, Cyano, Nitro, jeweils gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, bevorzugt für Halogen, Cyano, Nitro oder C₁-C₆-Halogenalkyl, Phenyl steht;
- R¹⁰: unabhängig voneinander für Wasserstoff, Halogen, Cyano, Nitro, Amino oder ein gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl steht;
- W: für Sauerstoff oder Schwefel steht;
- Q: für Wasserstoff, Formyl, Hydroxy, Amino oder eine der gegebenenfalls substituierten Gruppierungen C₁-C₆-Alkyl, C₁-C₆-Alkyloxy, C₃-C₆-Alkenyl, C₃-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl oder für eine Gruppierung *N*-(C₁-C₆-Alkyl)amino, *N*-(C₁-C₆-Alkylcarbonyl)amino, *N,N*-Di(C₁-C₆-alkyl)amino steht; oder
- Q: für einen gegebenenfalls mehrfach mit V substituierten ungesättigten 6-gliedrigen Carbozyklus steht, oder für einen gegebenenfalls mehrfach mit V substituierten ungesättigten 5- bzw. 6-gliedrigen heterozyklischen Ring steht, wobei
- V: für Halogen, Cyano, Nitro, oder eine der gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-alkyl, C₁-C₆-Alkylsulfanyl, C₁-C₆-Alkylsulfinyl, C₁-C₆-Alkylsulfonyl, *N,N*-Di(C₁-C₆-alkyl)amino steht;
sowie Salze, N-Oxide und tautomere Formen der Verbindungen der Formel (I),
wobei die genannten substituierten Gruppen mit einem oder mehreren Substituent(en) M¹, vorzugsweise 1, 2 oder 3 Resten M¹, substituiert sind und der/die Substituenten M¹ jeweils unabhängig voneinander ausgewählt aus der Gruppe bestehend aus Halogen, Hydroxy, Nitro, Formyl, Carboxy, Cyano, Amino, Isocyano, Azido, (C₁-C₄)-Alkyl, (C₁-C₄)-Halogenalkyl, (C₂-C₄)-alkenyl, (C₂-C₄)-alkinyl, (C₃-C₆)-Cycloalkyl (C₁-C₄)-Alkoxy, (C₁-C₄)-Halogenalkoxy, (C₁-C₄)-Alkoxy-(C₁-C₄)-alkoxy, (C₁-C₄)-Alkoxy-(C₁-C₆)-alkyl, *N*-(C₁-C₄)-Alkoxy-imino-(C₁-C₃)-alkyl, (C₁-C₄)-Alkylsulfanyl, (C₁-C₄)-Halogenalkylsulfanyl, (C₁-C₄)-Alkoxycarbonyl, (C₁-C₄)-Alkylcarbonyl, Carbamoyl, C₁-C₄-Alkylcarbamoyl, C₃-C₇-Cycloalkylcarbamoyl, Mono- und *N,N-*Di(C₁-C₄)-alkylaminocarbonyl, Amino, (C₁-C₆)-Acylamino, Mono- und *N,N-*Di(C₁-C₄)-alkylamino, Tri(C₁-C₄)-alkylsilyl, (C₃-C₆)-Cycloalkyl, C₆-Aryl, Heterocyclyl mit 3 bis 6 Ringatomen, wobei jeder der letztgenannten cyclischen Gruppen auch über Heteroatome oder eine divalente funktionelle CH₂-, oder C₂H₄-Gruppe gebunden sein kann, (C₁-C₄)-Alkylsulfinyl, wobei beide Enantiomere der (C₁-C₄)-Alkylsulfinylgruppe umfasst sind, (C₁-C₄)-Alkylsulfonyl, (C₁-C₄)-Alkylphosphinyl, (C₁-C₄)-Alkylsulfanyl-(C₁-C₄)-alkyl, (C₁-C₄)-Alkoxy-(C₁-C₄)-alkyl, Mono- und *N,N*-Di(C₁-C₄)-alkyl-amino(C₁-C₄)-alkyl und Hydroxy(C₁-C₄)-alkyl ist/sind und die genannten Reste M¹ unsubstituiert oder gegebenenfalls (wie z. B. Alkyl oder Amino), sofern sie kohlenwasserstoffhaltige oder stickstoffwasserstoffhaltige Anteile enthalten, mit einem oder mehreren, vorzugsweise 1, 2 oder 3 Resten M² substituiert sein können, wobei M² unabhängig voneinander ausgewählt ist aus der Gruppe bestehend aus Amino, Hydroxy, Halogen, Nitro, Cyano, Isocyano, Mercapto, Isothiocyanato, Carboxy und Carbonamid.

Hierin beschrieben sind weiterhin Verbindungen der Formel (I), worin
- R¹: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
- A₁: für CR² oder N,
- A₂: für N,
- A₃: für CR³ oder N,
- A₄: für CR⁴ oder N,
- B₁: für CR⁵ oder N,
- B₂: für CR⁶ oder N,
- B₃: für CR⁷ oder N,
- B₄: für CR⁸ oder N, und
- B₅: für CR⁹ oder N stehen,
wobei aber nicht mehr als zwei der chemischen Gruppierungen A₁ bis A₄ gleichzeitig für Stickstoff stehen und nicht mehr als zwei der Gruppierungen B₁ bis B₅ gleichzeitig für Stickstoff stehen;
- R² und R⁴: unabhängig voneinander für Wasserstoff oder Methyl stehen; und
- R³: für Fluor, Chlor, Brom, Iod, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy steht;
- R⁵, R⁶, R⁸ und R⁹: unabhängig voneinander für Wasserstoff, Halogen, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(N-Methoxyimino)-ethyl, Methylsulfanyl, Trifluormethylsulfanyl, Methylsulfonyl, Methylsulfinyl, Trifluormethylsulfonyl, Trifluormethylsulfinyl, wobei aber R⁶ und R¹⁰ nicht gleichzeitig für Wasserstoff stehen,
- R⁷: für perfluoriertes C₁-C₆-Alkyl, perfluoriertes C₃-C₆-Cycloalkyl, C₁-C₆-Alkoxy, *N-*Alkoxyiminoalkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆alkylamino, sowie für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht; oder
- R⁷: für perhalogeniertes, bevorzugt perfluoriertes, C₁-C₆-Alkyl perhalogeniertes, bevorzugt perfluoriertes, C₃-C₆-Cycloalkyl, C₁-C₆-Alkoxy, *N*-Alkoxyiminoalkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆alkylamino, sowie für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N-*Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino, Phenyl steht;
- R¹⁰: unabhängig voneinander für Wasserstoff, Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl steht;
- W: für Sauerstoff oder Schwefel steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1 -Methoxycarbonyl-cyclopropyl, 1-(*N*-Methylcarbamoyl)cyclopropyl, 1 -(N-Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N-*Diethylamino, 2-oxo-2-(2,2,2-trifluoroethylamino)ethyl steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht.

Eine weitere bevorzugte Ausführungsform bezieht sich auf eine erfindungsgemäße Verbindung der Formel (I), worin
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
- A₁: für CH,
- A₂: für N,
- A₃: für CR³, und
- A₄: für CH stehen;
- B₁: für CR⁵ oder N,
- B₂: für CH,
- B₃: für CR⁷,
- B₄: für CR⁸, und
- B₅: für CR⁹ oder N stehen,
- R³: für Fluor, Chlor, Brom, Iod, Methyl, Ethyl, Fluormethyl, Difluormethyl, Chlordifluormethyl, Trifluormethyl, 2,2,2-Trilfluorethyl, Methoxy, Ethoxy, n-Propoxy, 1-Methylethoxy steht;
- R⁵, R⁶, R⁸ und R⁹: unabhängig voneinander für Wasserstoff, Halogen, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(N-Methoxyimino)-ethyl, Methylsulfanyl, Trifluormethylsulfanyl, Methylsulfonyl, Methylsulfinyl, Trifluormethylsulfonyl, Trifluormethylsulfinyl, wobei aber R⁶ und R¹⁰ nicht gleichzeitig für Wasserstoff stehen;
- R⁷: für Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Trifluormethylsulfonyl, Trifluormethylsulfinyl, Trifluormethylsulfanyl, steht; oder
- R⁷: für Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Trifluormethylsulfonyl, Trifluormethylsulfinyl, Trifluormethylsulfanyl, Phenyl steht;
- R¹⁰: unabhängig voneinander für Wasserstoff, Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl steht;
- W: für Sauerstoff steht; und
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1 -Methoxycarbonyl-cyclopropyl, 1-(*N*-Methylcarbamoyl)cyclopropyl, 1-(*N-*Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl" 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N*-Diethylamino, 2-oxo-2-(2,2,2-trifluoroethylamino)ethyl steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N-*Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
- R⁵: für Wasserstoff, Methyl, Ethyl, 2-Methylethyl, tert.-Butyl, Fluor, Chlor, Brom, Iod, Nitro, Trifluormethyl, Difluormethoxy, Trifluormethoxy, Amino steht.

Eine weitere bevorzugte Ausführungsform bezieht sich auf eine erfindungsgemäße Verbindung der Formel (I), worin
- W: für Sauerstoff steht;
- R¹⁰: für Wasserstoff und R¹ für Wasserstoff oder Methyl steht;
- B₃: für CR⁷ steht und R⁷ für perhalogeniertes C₁-C₄-Alkyl steht; oder
- B₃: für CR⁷ steht und R⁷ für perhalogeniertes C₁-C₄-Alkyl oder Phenyl steht;
- Q: für gegebenenfalls unabhängig voneinander mit F, Cl, Br, I oder CN substituiertes Cyclopropyl steht
und alle weiteren Parameter wie in Paragraph [0006] definiert sind.

Eine weitere bevorzugte Ausführungsform bezieht sich auf eine erfindungsgemäße Verbindung der Formel (I), worin
- W: für Sauerstoff steht;
- R¹⁰: für Wasserstoff und R¹ für Wasserstoff oder Methyl steht;
- B₃: für CR⁷ steht und R⁷ für -C₃F₇ steht; oder
- B₃: für CR⁷ steht und R⁷ für -C₃F₇ oder Phenyl steht; und
- Q: für Cyclopropyl oder 1-Cyano-Cyclopropyl steht;
und alle weiteren Parameter wie in Paragraph [0006] definiert sind.

Eine weitere bevorzugte Ausführungsform bezieht sich auf eine erfindungsgemäße Verbindung der Formel (I), worin
- B₁: für CR⁵ steht, und R⁵ für Halogen, Ci-Cö-Alkoxy, C₁-C₆-Alkyl, mit Halogen (bevorzugt mit F) substituiertes C₁-C₆-Alkoxy oder mit Halogen (bevorzugt mit F) substituiertes C₁-C₆-Alkyl steht;
- B₂: für CR⁶ steht, wobei R⁶ für Wasserstoff oder C₁-C₆-Alkyl steht;
- B₄: für CR⁸ oder N steht, wobei R⁸ für Wasserstoff oder C₁-C₆-Alkyl steht;
- B₅: für CR⁹ oder N steht wobei R⁹ für Halogen, C₁-C₆-Alkoxy oder C₁-C₆-Alkyl steht; und
und alle weiteren Parameter wie in einem der Paragraphen [0006], [0009] oder [0010] definiert sind.

Eine weitere bevorzugte Ausführungsform bezieht sich auf eine erfindungsgemäße Verbindung der Formel (I), worin
- B₁: für CR⁵ steht, und R⁵ für Cl, Br, I, C₁-C₄-Alkyl, mit F substituiertes C₁-C₄-Alkyl oder mit F substituiertes C₁-C₄-Alkoxy steht,
- B₂: für CR⁶ steht, wobei R⁶ für Wasserstoff steht,
- B₄: für CR⁸ oder N steht, wobei R⁸ für Wasserstoff steht,
- B₅: für CR⁹ oder N steht wobei R⁹ für Halogen oder C₁-C₄-Alkyl steht
und alle weiteren Parameter wie in einem der Paragraphen [0006], [0009] oder [0010] oder [0011] definiert sind.

Eine weitere bevorzugte Ausführungsform bezieht sich auf eine erfindungsgemäße Verbindung der Formel (I), worin
A₁ für CR² steht wobei R² für Wasserstoff oder C₁-C₄-Alkyl steht;
A₂ für Stickstoff steht;
A₃ für CR³ steht wobei R³ für Halogen, C₁-C₄-Alkyl, C₁-C₄-Halogenalkyl oder C₁-C₄-Alkoxy steht; und
A₄ für CR⁴ steht wobei R⁴ für Wasserstoff oder C₁-C₄-Alkyl steht
und alle weiteren Parameter wie in einem der Paragraphen [0006], [0009], [0010], [0011] oder [0012] definiert sind.

Eine weitere bevorzugte Ausführungsform bezieht sich auf eine erfindungsgemäße Verbindung der Formel (I), worin
A₁ für CR² steht wobei R² für Wasserstoff steht;
A₂ für Stickstoff steht;
A₃ für CR³ steht wobei R³ für Halogen, C₁-C₄-Alkyl, C₁-C₄-Halogenalkyl oder C₁-C₄-Alkoxy steht; und
A₄ für CR⁴ steht wobei R⁴ für Wasserstoff steht;
und alle weiteren Parameter wie in Paragraph [0013] definiert sind.

Eine weitere bevorzugte Ausführungsform bezieht sich auf eine erfindungsgemäße Verbindung der Formel (I), worin
W für Sauerstoff steht,
R¹⁰ für Wasserstoff und R¹ für Wasserstoff oder Methyl steht,
A₁ für CR² steht wobei R² für Wasserstoff oder C₁-C₄-Alkyl steht,
A₂ für Stickstoff steht,
A₃ für CR³ steht wobei R³ für Halogen, C₁-C₄-Alkyl, C₁-C₄-Halogenalkyl oder C₁-C₄-Alkoxy steht,
A₄ für CR⁴ wobei R⁴ für Wasserstoff oder C₁-C₄-Alkyl steht,
B₁ für CR⁵ steht, und R⁵ für Cl, Br, I (bevorzugt Cl), C₁-C₄-Alkyl, mit F substituiertes (bevorzugt perfluoriertes) C₁-C₄-Alkyl, oder mit F substituiertes (bevorzugt mit ein, zwei oder drei F substituiertes, mehr bevorzugt mit zwei oder drei F substituiertes) C₁-C₄-Alkyl steht,
B₂ für CR⁶ steht, wobei R⁶ für Wasserstoff oder C₁-C₆-Alkyl steht,
B₃ für CR⁷ steht wobei R⁷ für perhalogeniertes C₁-C₄-Alkyl oder Phenyl steht,
B₄ für CR⁸ steht, wobei R⁸ für Wasserstoff oder C₁-C₆-Alkyl steht,
B₅ für CR⁹ steht wobei R⁹ für Halogen, C₁-C₆-Alkoxy oder C₁-C₆-Alkyl steht,
Q für Cyclopropyl oder 1-Cyano-Cyclopropyl, steht.

Eine weitere bevorzugte Ausführungsform bezieht sich auf eine erfindungsgemäße Verbindung der Formel (I), worin
W für Sauerstoff steht,
R¹⁰ für Wasserstoff und R¹ für Wasserstoff oder Methyl steht,
A₁ für CR² steht wobei R² für Wasserstoff steht;
A₂ für Stickstoff steht,
A₃ für CR³ steht wobei R³ für C₁-C₄-Alkyl (-CH₃, -C₂H₅, -C₃H₇, -C₄H₉), Cl, F, I oder C₁-C₄-Alkoxy (-O-CH₃, -O-C₂H₅, -O-C₃H₇, -O-C₄H₉) steht, bevorzugt für C₁-C₄-Alkyl (-CH₃, -C₂H₅, -C₃H₇, -C₄H₉), Cl oder C₁-C₄-Alkoxy (-O-CH₃, -O-C₂H₅, -O-C₃H₇, -O-C₄H₉), mehr bevorzugt für Methyl, Methoxy oder Cl steht,
A₄ für CR⁴ wobei R⁴ für Wasserstoff steht,
B₁ für CR⁵ steht, wobei R⁵ für F, Cl, Br, C₁-C₄-Alkyl (-CH₃, -C₂H₅, -C₃H₇, -C₄H₉), mit F substituiertes (bevorzugt perfluoriertes) C₁-C₄-Alkyl (-CF₃, -C₂F₅, -C₃F₇, -C₄F₉), oder mit F substituiertes C₁-C₄-Alkoxy (bevorzugt OCFH₂, OCF₂H oder OCF₃, mehr bevorzugt OCF₂H oder OCF₃) steht, bevorzugt wobei R⁵ für Cl, CF₃, OCF₃, OCF₂H, Methyl oder Ethyl steht,
B₂ für CR⁶ steht, wobei R⁶ für Wasserstoff steht,
B₃ für CR⁷ steht wobei R⁷ für perfluoriertes C₁-C₄-Alkyl oder Phenyl, bevorzugt für -C₃F₇ oder Phenyl steht,
B₄ für CR⁸ steht, wobei R⁸ für Wasserstoff steht,
B₅ für CR⁹ steht wobei R⁹ für F, Cl, Br, I, C₁-C₄-Alkyl steht, bevorzugt für Cl, Br, I oder C₁-C₄-Alkylsteht, besonders bevorzugt für Cl, I, Br oder Methyl steht,
Q für Cyclopropyl, 1-Cyano-Cyclopropyl, C₁-C₄-Alkyl (-CH₃, -C₂H₅, -C₃H₇, -C₄H₉), oder fluoriertes C₁-C₄-Alkyl (z. B. -CHF₂, CH₂F, -CF₃, -C₂H₄F, -C₂H₃F₂, -C₂H₂F₃, -C₂HF₄, -C₂F₅, -C₃F₇, -C₄F₉) steht, bevorzugt für Methyl, Ethyl, Propyl, C₂H₂F₃ (wie -CH₂CF₃), Cyclopropyl oder 1-Cyano-Cyclopropyl, steht.

Ein weiterer Aspekt der vorliegenden Erfindung bezieht sich auf eine pharmazeutische Zusammensetzung, enthaltend wenigstens eine erfindungsgemäße Verbindung der Formel (I).

Ein weiterer Aspekt der vorliegenden Erfindung bezieht sich auf eine erfindungsgemäße Verbindung der Formel (I) zur Verwendung als Arzneimittel.

Weiterhin hierin beschrieben ist die Verwendung einer erfindungsgemäßen Verbindung der Formel (I) zur Herstellung pharmazeutischer Zusammensetzungen zur Bekämpfung von Parasiten auf Tieren.

Ein weiterer Aspekt der vorliegenden Erfindung bezieht sich auf die Verwendung einer erfindungsgemäßen Verbindung der Formel (I) zum Schutz des Vermehrungsmaterials von Pflanzen, bevorzugt zum Schutz von Saatgut.

Weiterhin hierin beschrieben sind Intermediate der Formel (II).

Weiterhin hierin beschrieben sind Intermediate der Formel (III).

Weiterhin hierin beschrieben ist die Verwendung eines Intermediates der Formel (II) zur Synthese einer erfindungsgemäßen Verbindung der Formel (I).

Weiterhin hierin beschrieben ist die Verwendung eines Intermediates der Formel (III) zur Synthese einer erfindungsgemäßen Verbindung der Formel (I).

### Definitionen

Der Fachmann ist sich bewusst, dass die Ausdrücke "ein", "eine" oder "eines" wie in dieser Anmeldung genutzt je nach Situation "ein/eine/eines (1)", "ein/eine/eines (1) oder mehr" oder "mindestens ein/eine/eines (1)" bedeuten kann.

Der Ausdruck "gegebenenfalls substituiert", soweit keine spezifischen Substituenten angegeben sind, heißt, dass die entsprechende Gruppe einfach oder mehrfach mit einem Substituenten M¹ substituiert sein kann, wobei bei Mehrfachsubstitutionen die Substituenten M¹ gleich oder verschieden sein können.

Dem Fachmann ist klar, dass in dieser Anmeldung genannte Beispiele nicht als beschränkend anzusehen sind sondern lediglich einige Ausführungsformen näher beschreiben.

Die Ausdrücke "(Cₙ-Cₘ)" bzw. "Cₙ-Cₘ-" sind gegeneinander austauschbar und beziehen sich auf die minimale und maximale Anzahl von Kohlenstoffatomen in einer organischen Gruppe. "(C₁-C₆)" bzw. "C₁-C₆-"Alkyl bezieht sich z. B. auf eine Alkylgruppe mit 1, 2, 3, 4, 5, oder 6 Kohlenstoffatomen. Gleichfals sind die Ausdrücke "(Cₙ)" bzw. "Cₙ-" gegeneinander austauschbar und beziehen sich auf die Anzahl von Kohlenstoffatomen in einer organischen Gruppe. Z. B. beziehen sich die Ausdrücke "C₃-Cycloalkyl" bzw. "(C₃)Alkyl" auf Cyclopropyl.

Bei den in den vorstehenden Formeln angegebenen Definitionen der Symbole wurden Sammelbegriffe verwendet, die allgemein repräsentativ für die folgenden Substituenten stehen:

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen besonders bevorzugt mit 1, 2, 3 oder 4 Kohlenstoffatomen, wie beispielsweise Methyl, Ethyl, n-Propyl, Isopropyl, n-Butyl, Isobutyl, s-Butyl, t-Butyl Die erfindungsgemäßen Alkyle können mit einem oder mehreren, gleichen oder verschiedenen Resten M¹ gegebenenfalls substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen besonders bevorzugt mit 2, 3 oder 4 Kohlenstoffatomen, und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, etc. Die erfindungsgemäßen Alkenyle können mit einem oder mehreren, gleichen oder verschiedenen Resten M¹ gegebenenfalls substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen besonders bevorzugt mit 2, 3 oder 4 Kohlenstoffatomen, und mindestens einer Dreifachbindung wie beispielsweise Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, etc.. Die erfindungsgemäßen Alkinyle können mit einem oder mehreren, gleichen oder verschiedenen Resten M¹ gegebenenfalls substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Bicyclo[2.2.1]heptyl, Bicyclo[2.2.2]octyl oder Adamantyl, besonders bevorzugt für Cycloalkyle mit 3, 4, 5, 6 oder 7 Kohlenstoffatomen, wie z. B. Cyclopropyl oder Cyclobutyl. Die erfindungsgemäßen Cycloalkyle können mit einem oder mehreren, gleichen oder verschiedenen Resten M¹ gegebenenfalls substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, Besonders brzugt für Alkylcycloalkyle mit 4, 5 oder 7 Kohlenstoffatomen wie z. B. Ethylcyclopropyl oder 4-Methyl-cyclohexyl, wobei das Alkylcycloalkyl über das Cycloalkyl mit der Grundstruktur verbunden ist. Die erfindungsgemäßen Alkylcycloalkyle können mit einem oder mehreren, gleichen oder verschiedenen Resten M¹ gegebenenfalls substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, besonders bevorzugt für Cycloalkylalkyle mit 4, 5 oder 7 Kohlenstoffatomen wie unter anderen Cyclopropylmethyl oder Cyclobutylmethyl, wobei das Alkylcycloalkyl über das Alkyl mit der Grundstruktur verbunden ist. Die erfindungsgemäßen Cycloalkylalkyle können mit einem oder mehreren, gleichen oder verschiedenen Resten M¹ gegebenenfalls substituiert sein.

Erfindungsgemäß steht "Alkoxy" für geradkettiges oder verzweigtes O-Alkyl, vorzugsweise mit 1 bis 6 Kohlenstoffatomen, mehr bevorzugt für Alkoxygruppen mit 1 bis 4 Kohlenstoffatomen wie beispielsweise Methoxy, Ethoxy, n-Propoxy, Isopropoxy, n-Butoxy, Isobutoxy, s-Butoxy oder t-Butoxy. Die erfindungsgemäßen Alkoxygruppen können mit einem oder mehreren, gleichen oder verschiedenen Resten M¹ gegebenenfalls substituiert sein.

Erfindungsgemäß steht "Alkylsulfanyl" für geradkettiges oder verzweigtes S-Alkyl, vorzugsweise mit 1 bis 6 Kohlenstoffatomen, mehr bevorzugt für Alkylsulfanylgruppen mit 1 bis 4 Kohlenstoffatomen, wie beispielsweise Methylthio, Ethylthio, n-Propylthio, Isopropylthio, n-Butylthio, Isobutylthio, s-Butylthio und t-Butylthio. Die erfindungsgemäßen Alkylsulfanylgruppen können mit einem oder mehreren, gleichen oder verschiedenen Resten M¹ gegebenenfalls substituiert sein.

Erfindungsgemäß steht "Alkylsulfinyl" für geradkettiges oder verzweigtes Alkylsulfinyl, vorzugsweise mit 1 bis 6 Kohlenstoffatomen, mehr bevorzugt für Alkylsulfinylgruppen mit 1 bis 4 Kohlenstoffatomen, wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Die erfindungsgemäßen Alkylsulfinylgruppen können mit einem oder mehreren, gleichen oder verschiedenen Resten M¹ gegebenenfalls substituiertsein.

Erfindungsgemäß steht "Alkylsulfonyl" für geradkettiges oder verzweigtes Alkylsulfonyl, vorzugsweise mit 1 bis 6 Kohlenstoffatomen, mehr bevorzugt für Alkylsulfonylgruppen mit 1 bis 4 Kohlenstoffatomen, wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Die erfindungsgemäßen Alkylsulfonylgruppen können mit einem oder mehreren, gleichen oder verschiedenen Resten M¹ gegebenenfalls substituiert sein.

Erfindungsgemäß steht "Acyl" für Reste, die eine X¹-C(=O)-X² Gruppe enthalten, wobei X¹ und X² unabhängig voneinander für einen organischen Rest wie in dieser Anmeldung definiert oder für Wasserstoff oder für eine Bindung an die Grundstruktur einer Verbindung der Formel (I) steht. Insbesondere werden unter "Acyl" organische Säuren, Ester, Aldehyde, Alkylcarbonyl (alkyl-C(=O)-) und Amide verstanden. Bevorzugt handelt es sich bei X¹ und X² jeweils unabhängig voneinander um eine gegebenenfalls mit einem oder mehreren, gleichen oder verschiedenen Resten M¹ substituierte Gruppe ausgewählt aus Alkyl, Alkylen (-CₙH₂ₙ-), Alkoxy, Alkoxylen (-O-CₙH₂ₙ-), Amino, Mono- oder Di-Alkylamino oder Wasserstoff oder ein Rest X¹ oder X² stellt eine Bindung an die Grundstruktur einer Verbindung der Formel (I) dar.

Erfindungsgemäß steht "Alkylcarbonyl" für geradkettiges oder verzweigtes Alkyl-C(=O)-, vorzugsweise mit 2 bis 7 Kohlenstoffatomen (inklusive des C-Atoms der C(=O)-Gruppe), mehr bevorzugt für Alkylcarbonyle mit 2 bis 5 Kohlenstoffatomen ((C₁-C₄)Alkyl-C(=O)-), wie Methylcarbonyl, Ethylcarbonyl, n-Propylcarbonyl, Isopropylcarbonyl, s-Butylcarbonyl und t-Butylcarbonyl. Die erfindungsgemäßen Alkylcarbonyle können mit einem oder mehreren, gleichen oder verschiedenen Resten M¹ gegebenenfalls substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, mehr bevorzugt für Cycloalkylcarbonyl mit 3, 5 oder 7 Kohlenstoffatomen im Cycloalkylteil wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Die erfindungsgemäßen Cycloalkylcarbonylgruppen können mit einem oder mehreren, gleichen oder verschiedenen Resten M¹ gegebenenfalls substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen, mehr bevorzugt mit 1, 2, 3 oder 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten M¹ gegebenenfalls substituiert sein.

Erfindungsgemäß steht "Halogen" für Fluor (F), Chlor (Cl), Brom (Br) oder Iod (I).

Die Ausdrücke "Halogenalkyl", "Halogenalkenyl", "Halogenalkinyl", "Halogenalkylcarbonyl" "Halogenalkoxy", "Halogenalkoxycarbonyl", "Halogenalkylsulfanyl", "Halogenalkylsulfinyl" oder "Halogenalkylsulfonyl" wie hierin benutzt beziehen sich auf mit mindestens einem Halogen substituierte chemische Alkyl, Alkenyl, Alkinyl, Alkylcarbonyl, Alkoxy, Alkoxycarbonyl, Alkylsulfanyl, Alkylsulfinyl oder Alkylsulfonyl Gruppe (jeweils bevorzugt mit eins bis 6 Kohlenstoffatomen oder mehr bevorzugt mit eins, zwei, drei oder vier Kohlenstoffatomen). Die Halogengruppen können einfach oder mehrfach bis zur maximal möglichen Substituentenzahl (perhalogeniert) mit Halogen substituiert sein. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor oder Chlor und besonders bevorzugt für Fluor. In einer bevorzugten Ausführungsform sind perhalogenierte Gruppen mit nur einer Sorte Halogenen maximal substituiert, z. B. perfluoriertes Methyl (Trifluormethyl; CF₃) oder perfluoriertes Ethyl (Pentafluorethyl; C₂F₅). Einige Beispiele für "Halogenalkyl", "Halogenalkenyl", "Halogenalkinyl", "Halogenalkylcarbonyl" "Halogenalkoxy", "Halogenalkoxycarbonyl", "Halogenalkylsulfanyl", "Halogenalkylsulfinyl" oder "Halogenalkylsulfonyl" sind Trichlormethyl (CCl₃), Trifluormethyl (CF₃), Chlordifluormethyl (CClF₂), Dichlorfluormethyl (CCl₂F), 2,2-Difluorethyl (F₂HCCH₂), 2,2,2-Trifluorethyl (F₃CCH₂), Pentafluorethyl (C₂F₅), 2,2-Difluorethenyl (CHCF₂), 2-Chlorethinyl (CHCCl), Trifluormethoxy -OCF₃, Difluormethoxy -OCHF₂, 1,1,2,2-Tetrafluorethylthio, 2-Chlor-1,1,2-trifluorethylsulfinyl, Trichlormethylsulfonyl, etc. Die erfindungsgemäßen Halogengruppen können, wenn angegeben, mit einem oder mehreren, gleichen oder verschiedenen Resten M¹ gegebenenfalls substituiert sein, solange wenigstens ein Wasserstoffatom an einem Kohlenstoffatom der Halogengruppe durch ein Halogen ersetzt ist. Ein Beispiel für ein mit einem M¹ substituierten Halogenalkyl ist 2-Cyano-2,2-Difluoroethyl (C(CN)F₂CH₂).

Eine Aminogruppe (-NH₂) kann mit mit einem oder mehreren, gleichen oder verschiedenen Resten M¹ gegebenenfalls substituiert sein.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise *N*-Mono- und *N,N*-Dialkylamino, (z.B. Methylamino, Ethylamino, *N,N*-Dimethylamino, *N,N-*Diethylamino, *N*,*N-*Di-n-propylamino, *N,N*-Diisopropylamino oder *N,N*-Dibutylamino), *N*-Mono-oder *N,N*-Dialkoxyalkylaminogruppen (z.B. *N*-Methoxymethylamino, *N*-Methoxyethylamino, *N,N*-Di-(methoxymethyl)-amino oder *N,N*-Di-(methoxyethyl)-amino), *N*-Mono- und *N,N-*Diarylamino, wie gegebenenfalls substituierte Aniline, Acylamino, *N,N-*diacylamino, *N*-Alkyl-*N*-arylamino, *N*-Alkyl-*N-*acylamino sowie gesättigte *N*-Heterocyclen; dabei sind Alkylreste mit 1 bis 4 C-Atomen bevorzugt; Aryl ist dabei vorzugsweise Phenyl oder gegebenenfalls substituiertes Phenyl; für Acyl gilt dabei die weiter oben genannte Definition, vorzugsweise (C₁-C₄)Alkyl-C(=O)-.

Substituiertes Amino schließt auch quartäre Ammoniumverbindungen (Salze) mit vier organischen Substituenten am Stickstoffatom ein.

Erfindungsgemäß steht "Hydroxyalkyl" für geradkettigen oder verzweigten Alkohol, vorzugsweise mit 1 bis 6 Kohlenstoffatomen, mehr bevorzugt mit 1, 2, 3 oder 4 Kohlenstoffatomen, wie beispielsweise Methanol, Ethanol, n-Propanol, Isopropanol, n-Butanol, Isobutanol, s-Butanol und t-Butanol. Die erfindungsgemäßen Hydroxyalkylgruppen können mit einem oder mehreren, gleichen oder verschiedenen Resten M¹ substituiert sein

Erfindungsgemäß steht "Alkylaminocarbonyl" für geradkettiges oder verzweigtes Alkylaminocarbonyl mit vorzugsweise 1 bis 6 Kohlenstoffatomen, mehr bevorzugt 1, 2, 3 oder 4 Kohlenstoffatomen im Alkylteil, wie beispielsweise Methylaminocarbony (-CONHCH₃), Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten M¹ gegebenenfalls substituiert sein.

Erfindungsgemäß steht "*N,N*-Dialkylamino-carbonyl" (-C(=O)N(alkyl)₂)für geradkettiges oder verzweigtes *N,N*-Dialkylaminocarbonyl mit vorzugsweise 1 bis 6 Kohlenstoffatomen pro alkyl, mehr bevorzugt 1, 2, 3, oder 4 Kohlenstoffatomen pro alkyl, wie beispielsweise *N,N*-Dimethylamino-carbonyl (-C(=O)N(CH₃)₂), N.N-Diethylamino-carbonyl, *N,N*-Di(n-propylamino)-carbonyl, *N,N*-Di-(isopropylamino)-carbonyl und *N,N*-Di-(s-butylamino)-carbonyl. Die erfindungsgemäßen *N,N-*Dialkylamino-carbonylgruppen können mit einem oder mehreren, gleichen oder verschiedenen Resten M¹ gegebenenfalls substituiert sein.

"Carbozyklus", soweit nicht an anderer Stelle anders definiert ist insbesondere ein Cycloalkyl, Cycloalkenyl, oder Aryl. Insbesonder ist ein Carbozyklus ein C₆ bis C₁₄ mono- bi- oder tricyclisches Aryl. Ein Carbozyklus kann mit einem oder mehreren, gleichen oder verschiedenen Resten M¹ gegebenenfalls substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten M¹ gegebenenfalls substituiert sein.

Erfindungsgemäß steht "Arylalkyl" für ein mit einem Aryl substituierten Alkylrest mit vorzugsweise 6 bis 14, insbesondere 6 bis 10 Ring-Kohlenstoffatomen im Arylteil und 1 bis 6, insbesondere 1 bis 4 Kohlenstoffatomen im Alkylteil. Arylalkyl kann mit einem oder mehreren, gleichen oder verschiedenen Resten im Alkyl- und/oder Arylteil substituiert sein. Beispiele solcher Arylalkyle sind unter anderem Benzyl und 1-Phenylethyl. Die erfindungsgemäßen Arylalkyl ruppen können mit einem oder mehreren, gleichen oder verschiedenen Resten M¹ gegebenenfalls substituiert sein.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl. Die erfindungsgemäßen Gruppen "Heterocyclus", "heterocyclischer Ring" oder "heterocyclisches Ringsystem" können mit einem oder mehreren, gleichen oder verschiedenen Resten M¹ gegebenenfalls substituiert sein.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen, vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten M¹ gegebenenfalls substituiert sein.

"Substituierte" Gruppe bzw. "mit mindestens einem Rest M¹ substituierten" Gruppe im Sinne der vorliegenden Erfindung, wie ein gegebenenfalls substituierter Alkyl-, Alkenyl-, Alkinyl-, Cycloalkyl-, Aryl-, Phenyl-, Benzyl-, Heterocyclyl-, Heteroaryl-, oder Amino-Rest etc., ist allgemein eine Gruppe die mindestens einen kohlenwasserstoffhaltigen oder stickstoffwasserstoffhaltigen Anteile enthält, in dem das Wasserstoff durch ein anderes Atom oder eine Atomgruppe M¹ ersetzt ist. In anderen Worten, eine derartige Gruppe ist eine vom unsubstituierten Grundkörper abgeleitete substituierte Gruppe, wobei der Grundkörper mit einem oder mehreren Substituent(en) M¹, vorzugsweise 1, 2 oder 3 Resten M¹, substituiert ist und der/die Substituenten M¹ jeweils unabhängig voneinander ausgewählt aus der Gruppe bestehend aus Halogen, Hydroxy, Nitro, Formyl, Carboxy, Cyano, Amino, Isocyano, Azido, (C₁-C₄)-Alkyl, (C₁-C₄)-Halogenalkyl, (C₂-C₄)-alkenyl, (C₂-C₄)-alkinyl, (C₃-C₆)-Cycloalkyl (C₁-C₄)-Alkoxy, (C₁-C₄)-Halogenalkoxy, (C₁-C₄)-Alkoxy-(C₁-C₄)-alkoxy, (C₁-C₄)-Alkoxy-(C₁-C₆)-alkyl, *N*-(C₁-C₄)-Alkoxy-imino-(C₁-C₃)-alkyl, (C₁-C₄)-Alkylsulfanyl, (C₁-C₄)-Halogenalkylsulfanyl, (C₁-C₄)-Alkoxycarbonyl, (C₁-C₄)-Alkylcarbonyl, Carbamoyl, C₁-C₄-Alkylcarbamoyl, C₃-C₇-Cycloalkylcarbamoyl, Mono- und *N,N-*Di(C₁-C₄)-alkylaminocarbonyl, Amino, (C₁-C₆)-Acylamino, Mono- und *N,N*-Di(C₁-C₄)-alkylamino, Tri(C₁-C₄)-alkylsilyl, (C₃-C₆)-Cycloalkyl, C₆-Aryl, Heterocyclyl mit 3 bis 6 Ringatomen, wobei jeder der letztgenannten cyclischen Gruppen auch über Heteroatome oder eine divalente funktionelle CH₂-, oder C₂H₄-Gruppe gebunden sein kann, (C₁-C₄)-Alkylsulfinyl, wobei beide Enantiomere der (C₁-C₄)-Alkylsulfinylgruppe umfasst sind, (C₁-C₄)-Alkylsulfonyl, (C₁-C₄)-Alkylphosphinyl, (C₁-C₄)-Alkylsulfanyl-(C₁-C₄)-alkyl, (C₁-C₄)-Alkoxy-(C₁-C₄)-alkyl, Mono- und *N,N*-Di(C₁-C₄)-alkyl-amino(C₁-C₄)-alkyl und Hydroxy(C₁-C₄)-alkyl ist/sind. Die beispielhaft genannten Reste M¹ können unsubstituiert sein oder gegebenenfalls (wie z. B. Alkyl oder Amino), sofern sie kohlenwasserstoffhaltige oder stickstoffwasserstoffhaltige Anteile enthalten, mit einen oder mehrere, vorzugsweise 1, 2 oder 3 Resten M² substituiert sein, wobei M² unabhängig voneinander ausgewählt ist aus der Gruppe bestehend aus Amino, Hydroxy, Halogen, Nitro, Cyano, Isocyano, Mercapto, Isothiocyanato, Carboxy und Carbonamid. Substituierte Gruppen umfassen auch explizit genannte substituierte Gruppen. "Halogenalkyl" ist z. B. von dem Ausdruck "substituiertes" Alkyl umfasst und stellt eine bevorzugte Ausführungsform eines substituierten Alkyls dar. Dies gilt analog auch für alle anderen substituierten Gruppen.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Gegebenenfalls substituiertes Phenyl ist vorzugsweise Phenyl, das unsubstituiert oder ein- oder mehrfach, vorzugsweise einfach, zweifach oder dreifach durch gleiche oder verschiedene Reste ausgewählt aus der Gruppe Halogen, Cyano, Isocyano, Nitro, gegebenenfalls mit mindestens einem Rest M¹ substituiertes (C₁-C₄)Alkyl, C₂-C₄-Alkenyl, C₂-C₄-Alkinyl, (C₁-C₄)Alkoxy, (C₁-C₄)Alkoxy-(C₁-C₄)alkoxy, (C₁-C₄)Alkoxy-(C₁-C₄)alkyl, (C₃-C₆)-Cycloalkyl, (C₁-C₄)Halogenalkyl, (C₁-C₄)Halogenalkoxy, (C₁-C₄)Alkylsulfanyl, (C₁-C₄)Halogenalkylsulfanyl, substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl substituiert ist.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste ausgewählt aus der Gruppe Halogen, Haloaklyl, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl und (C₁-C₄)Halogenalkoxy substituiert ist.

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für mit Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl,, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Detaillierte Beschreibung

Hierin beschrieben sind Aryl-triazolyl-pyridine der allgemeinen Formel (I) 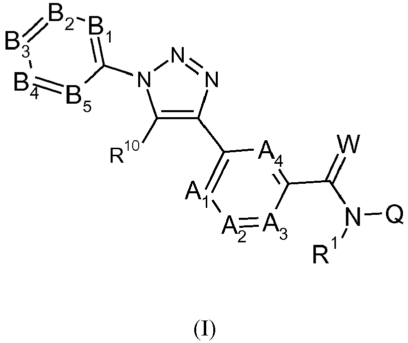 , in denen
- R¹: für Wasserstoff, oder eine gegebenenfalls substituierte Gruppe ausgewählt aus C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl-C₁-C₆-alkyl, Heteroaryl-C₁-C₆-alkyl steht;
die chemischen Gruppierungen
- A₁: für CR² oder Stickstoff,
- A₂: für Stickstoff,
- A₃: für CR³ oder Stickstoff und
- A₄: für CR⁴ oder Stickstoff stehen,
- B₁: für CR⁵ oder N,
- B₂: für CR⁶ oder N,
- B₃: für CR⁷ oder N,
- B₄: für CR⁸ oder N, und
- B₅: für CR⁹ oder N stehen,
wobei aber nicht mehr als zwei der chemischen Gruppierungen A₁ bis A₄ gleichzeitig für Stickstoff und nicht mehr als zwei der Gruppierungen B₁ bis B₅ gleichzeitig für Stickstoff stehen;
- R², R³, R⁴, R⁵, R⁶, R⁸ und R⁹: unabhängig voneinander für Wasserstoff, Halogen, Cyano, Nitro, gegebenenfalls substituiertes C₁-C₆-Alkyl, C₃-C₇-Cycloalkyl, C₁-C₆-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino oder *N*,*N*-Di-(C₁-C₆-alkyl)amino, C₁-C₆-Alkylsulfonyl-amino, *N*-(C₁-C₆-Alkyl)-C₁-C₆-alkylsulfonyl-amino stehen; oder
- R², R³, R⁴, R⁵, R⁶, R⁸ und R⁹: unabhängig voneinander für Wasserstoff, Halogen, Cyano, Nitro, gegebenenfalls substituiertes C₁-C₆-Alkyl, C₃-C₇-Cycloalkyl, Ci-Cö-Alkoxy, *N*-(C₁-C₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino oder *N,N*-Di-(C₁-C₆-alkyl)amino, C₁-C₆-Alkylsulfonyl-amino, *N*-(C₁-C₆-Alkyl)-C₁-C₆-alkylsulfonyl-amino, Phenyl stehen;
- R⁷: für Halogen, Cyano, Nitro, jeweils gegebenenfalls substituiertes C₁-C₆-Alkyl, C₃-C₆-Cycloalkyl, Ci-Cö-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, bevorzugt für Halogen, Cyano, Nitro oder C₁-C₆-Halogenalkyl steht; oder
- R⁷: für Halogen, Cyano, Nitro, jeweils gegebenenfalls substituiertes C₁-C₆-Alkyl, C₃-C₆-Cycloalkyl, Ci-Cö-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, bevorzugt für Halogen, Cyano, Nitro oder C₁-C₆-Halogenalkyl Phenyl steht;
- R¹⁰: unabhängig voneinander für Wasserstoff, Halogen, Cyano, Nitro, Amino oder ein gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl steht;
- W: für Sauerstoff oder Schwefel steht;
- Q: für Wasserstoff, Formyl, Hydroxy, Amino oder eine der gegebenenfalls substituierten Gruppierungen C₁-C₆-Alkyl, C₁-C₆-Alkyloxy, C₃-C₆-Alkenyl, C₃-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl oder für eine Gruppierung *N*-(C₁-C₆-Alkyl)amino, *N*-(C₁-C₆-Alkylcarbonyl)amino, *N,N*-Di(C₁-C₆-alkyl)amino steht; oder
- Q: für einen gegebenenfalls mehrfach mit V substituierten ungesättigten 6-gliedrigen Carbozyklus steht, oder für einen gegebenenfalls mehrfach mit V substituierten ungesättigten 5- bzw. 6-gliedrigen heterozyklischen Ring steht, wobei
- V: für Halogen, Cyano, Nitro, oder eine der gegebenenfalls substituierten Gruppierungen C₁-C₆-Alkyl, C₂-C₆-Alkenyl, C₂-C₆-Alkinyl, C₃-C₇-Cycloalkyl, Ci-Cö-Alkoxy, *N*-(C₁-C₆-Alkoxy)-imino-C₁-C₆-alkyl, C₁-C₆-Alkylsulfanyl, C₁-C₆-Alkylsulfinyl, C₁-C₆-Alkylsulfonyl, *N,N*-Di(C₁-C₆-alkyl)amino steht;
sowie Salze, N-Oxide und tautomere Formen der Verbindungen der Formel (I).

Eine bevorzugte Ausführungsform bezieht sich auf Verbindungen der Formel (I) in der B₁ für CR⁵ steht und R⁵ für Halogen, gegebenenfalls mit Halogen (bevorzugt mit F) substituiertes C₁-C₆-Alkoxy oder gegebenenfalls mit Halogen (bevorzugt mit F) substituiertes C₁-C₆-Alkyl steht, bevorzugt für Cl, Br, I, C₁-C₄-Alkyl, mit F substituiertes (bevorzugt perfluoriertes) C₁-C₄-Alkyl, oder mit F substituiertes (bevorzugt mit einem, zwei oder drei F substituiertes) C₁-C₄-Alkoxy steht, mehr bevorzugt für Cl, C₁-C₄-Alkyl mit einem, zwei oder drei F substituiertes C₁-C₄-Alkoxy oder perfluoriertes C₁-C₄-Alkyl steht und besonders bevorzugt für Cl, CF₃, OCHF₂, OCF₃, Methyl oder Ethyl steht.

Eine weitere bevorzugte Ausführungsform bezieht sich auf Verbindungen der Formel (I) in der B₂ für CR⁶ steht und R⁶ für Wasserstoff oder C₁-C₆-Alkyl steht, bevorzugt für Wasserstoff steht.

Eine weitere bevorzugte Ausführungsform bezieht sich auf Verbindungen der Formel (I) in der B₄ für CR⁸ steht und R⁸ für Wasserstoff oder C₁-C₆-Alkyl steht, bevorzugt für Wasserstoff steht.

Eine weitere bevorzugte Ausführungsform bezieht sich auf Verbindungen der Formel (I) in der B₃ für CR⁷ steht und R⁷ für Halogen, Cyano, Nitro oder C₁-C₆-Halogenalkyl steht, bevorzugt für perhalogeniertes C₁-C₄-Alkyl, besonders bevorzugt für perfluoriertes C₁-C₄-Alkyl, ganz besonders bevorzugt für -C₃F₇ steht oder R⁷ für Halogen, Cyano, Nitro, C₁-C₆-Halogenalkyl oder Phenyl steht, bevorzugt für perhalogeniertes C₁-C₄-Alkyl oder Phenyl, besonders bevorzugt für perfluoriertes C₁-C₄-Alkyl oder Phenyl, ganz besonders bevorzugt für -C₃F₇ oder Phenyl steht.

Eine bevorzugte Ausführungsform bezieht sich auf Verbindungen der Formel (I) in der B₅ für CR⁹ steht und R⁹ für Halogen, C₁-C₆-Alkoxy oder C₁-C₆-Alkyl steht, bevorzugt für Halogen oder C₁-C₄-Alkyl steht und besonders bevorzugt für Cl, Br, I oder Methyl steht.

Eine weitere bevorzugte Ausführungsform bezieht sich auf Verbindungen der Formel (I) in der W für Sauerstoff steht.

Eine weitere bevorzugte Ausführungsform bezieht sich auf Verbindungen der Formel (I) in der R¹ und R¹⁰ unabhängig voneinander für Wasserstoff oder C₁-C₄-Alkyl stehen, bevorzugt in der R¹⁰ für Wasserstoff und R¹ für Wasserstoff oder Methyl steht, mehr bevorzugt in der R¹ und R¹⁰ für Wasserstoff stehen.

Eine weitere bevorzugte Ausführungsform bezieht sich auf Verbindungen der Formel (I) in der A₁ für CR² steht und R² für Wasserstoff oder C₁-C₄-Alkyl steht, bevorzugt für Wasserstoff steht.

Eine weitere bevorzugte Ausführungsform bezieht sich auf Verbindungen der Formel (I) in der A₄ für CR⁴ steht und R⁴ für Wasserstoff oder C₁-C₄-Alkyl steht, bevorzugt für Wasserstoff steht.

Eine weitere bevorzugte Ausführungsform bezieht sich auf Verbindungen der Formel (I) in der A₃ für CR³ steht und R³ für Halogen, C₁-C₄-Alkyl, C₁-C₄-Halogenalkyl oder C₁-C₄-Alkoxy steht, besonders bevorzugt für Cl, Methyl oder Methoxy steht.

Eine weitere bevorzugte Ausführungsform bezieht sich auf Verbindungen der Formel (I) in der Q für Wasserstoff, Hydroxy, Formyl oder gegebenenfalls substituiertes C₃-C₆-Cycloalkyl steht, bevorzugt gegebenenfalls unabhängig voneinander mit Nitro, Amino, Halogen, C₁-C₄-Alkoxy, Cyano oder Hydroxycarbonyl substituiertes C₃-C₆-Cycloalkyl, mehr bevorzugt für gegebenenfalls unabhängig voneinander mit F, Cl, Br, I oder CN substituiertes Cyclopropyl, besonders bevorzugt für Cyclopropyl, mit Fluor oder Chlor monosubstituiertes Cyclopropyl oder Cyano-Cyclopropyl (1-Cyano-Cyclopropyl oder 2-Cyano-Cyclopropyl), ganz besonders bevorzugt für Cyclopropyl oder 1-Cyano-Cyclopropyl, steht. Eine weitere bevorzugte Ausführungsform richtet sich auf Verbindungen der Formel (I) in der Q für Wasserstoff, Hydroxy, Formyl oder gegebenenfalls substituiertes C₃-C₆-Cycloalkyl oder gegebenenfalls substituiertes C₁-C₄-Alkyl steht, bevorzugt gegebenenfalls unabhängig voneinander mit Nitro, Amino, Halogen, C₁-C₄-Alkoxy, Cyano oder Hydroxycarbonyl substituiertes C₃-C₆-Cycloalkyl oder gegebenenfalls mit F substituiertes C₁-C₄-Alkyl steht, mehr bevorzugt für gegebenenfalls unabhängig voneinander mit F, Cl, Br, I oder CN substituiertes Cyclopropyl oder C₁-C₄-Alkyl oder mit F substituiertes C₁-C₄-Alkyl steht, besonders bevorzugt für Cyclopropyl, mit Fluor oder Chlor monosubstituiertes Cyclopropyl oder Cyano-Cyclopropyl (1-Cyano-Cyclopropyl oder 2-Cyano-Cyclopropyl) oder jeweils gegebenfalls mit F substituiertes Methyl, Ethyl Propyl oder Butyl, ganz besonders bevorzugt für Cyclopropyl oder 1-Cyano-Cyclopropyl oder Methyl oder Ethyl oder mit 1,2 oder 3 F substituiertes Methyl oder mit 1,2, 3, 4 oder 5 F substituiertes Ethyl, steht.

Eine weitere bevorzugte Ausführungsform bezieht sich auf Verbindungen der Formel (I) in der
- W: für Sauerstoff steht,
- R¹⁰: für Wasserstoff und R¹ für Wasserstoff oder Methyl steht,
- A₁: für CR² wobei R² für Wasserstoff oder C₁-C₄-Alkyl steht, bevorzugt für Wasserstoff steht,
- A₂: für Stickstoff steht,
- A₃: für CR³ steht wobei R³ für Halogen, C₁-C₄-Alkyl, C₁-C₄-Halogenalkyl oder C₁-C₄-Alkoxy steht,
- A₄: für CR⁴ wobei R⁴ für Wasserstoff oder C₁-C₄-Alkyl steht, bevorzugt für Wasserstoff steht,
- B₁: für CR⁵ oder N steht, und R⁵ für Halogen, gegebenenfalls mit F substituiertes C₁-C₆-Alkoxy oder C₁-C₆-Alkyl steht, bevorzugt für Cl oder gegebenenfalls mit F substituiertes C₁-C₄-Alkyl oder mit F substituiertem Alkoxy steht und besonders bevorzugt für Cl, CF₃, OCHF₂, OCF₃, Methyl oder Ethyl steht,
- B₂: für CR⁶ oder N steht, wobei R⁶ für Wasserstoff oder C₁-C₆-Alkyl steht, bevorzugt für Wasserstoff steht,
- B₃: für CR⁷ oder N steht wobei R⁷ für Halogen, Cyano, Nitro oder C₁-C₆-Halogenalkyl steht, bevorzugt für perhalogeniertes C₁-C₄-Alkyl, besonders bevorzugt für perfluoriertes C₁-C₄-Alkyl, ganz besonders bevorzugt für -C₃F₇ steht, oder
- B₃: für CR⁷ oder N steht wobei R⁷ für Halogen, Cyano, Nitro, Phenyl oder C₁-C₆-Halogenalkyl steht, bevorzugt für perhalogeniertes C₁-C₄-Alkyl oder Phenyl, besonders bevorzugt für perfluoriertes C₁-C₄-Alkyl oder Phenyl, ganz besonders bevorzugt für -C₃F₇ oder Phenyl steht,
- B₄: für CR⁸ oder N steht, wobei R⁸ für Wasserstoff oder C₁-C₆-Alkyl steht, bevorzugt für Wasserstoff steht,
- B₅: für CR⁹ oder N steht wobei R⁹ für Halogen, C₁-C₆-Alkoxy oder C₁-C₆-Alkyl steht, bevorzugt für Halogen oder C₁-C₄-Alkyl steht und besonders bevorzugt für Cl, Br, I oder Methyl steht,
- Q: für Wasserstoff, Hydroxy, Formyl oder gegebenenfalls substituiertes C₃-C₆-Cycloalkyl steht, bevorzugt gegebenenfalls unabhängig voneinander mit Nitro, Amino, Halogen, C₁-C₄-Alkoxy, Cyano oder Hydroxycarbonyl substituiertes C₃-C₆-Cycloalkyl, mehr bevorzugt für gegebenenfalls unabhängig voneinander mit F, Cl, Br, I oder CN substituiertes Cyclopropyl, besonders bevorzugt für Cyclopropyl, mit Fluor oder Chlor monosubstituiertes Cyclopropyl oder Cyano-Cyclopropyl (1-Cyano-Cyclopropyl oder 2-Cyano-Cyclopropyl), ganz besonders bevorzugt für Cyclopropyl oder 1-Cyano-Cyclopropyl, steht, oder
- Q: für Cyclopropyl, mit Fluor oder Chlor monosubstituiertes Cyclopropyl oder Cyano-Cyclopropyl (1-Cyano-Cyclopropyl oder 2-Cyano-Cyclopropyl) oder jeweils gegebenfalls mit F substituiertes Methyl, Ethyl Propyl oder Butyl, ganz besonders bevorzugt für Cyclopropyl oder 1-Cyano-Cyclopropyl oder Methyl oder Ethyl oder mit ein, zwei oder drei F substituiertes Methyl oder mit ein, zwei, drei, vier oder fünf F substituiertes Ethyl, steht,
wobei aber nicht mehr als zwei der Gruppierungen B₁ bis B₅ gleichzeitig für Stickstoff stehen.

Weiterhin hierin beschrieben sind Verbindungen der Formel (I) in der
- W: für Sauerstoff steht,
- R¹⁰: für Wasserstoff und R¹ für Wasserstoff oder Methyl steht,
- A₁: für CR² oder Stickstoff steht wobei R² für Wasserstoff oder C₁-C₄-Alkyl steht, bevorzugt für Wasserstoff steht,
- A₂: für Stickstoff steht,
- A₃: für CR³ oder Stickstoff steht wobei R³ für Halogen oder eine gegebenenfalls substituierte Gruppe ausgewählt aus C₁-C₆-Alkyl und C₁-C₆-Alkoxy steht, bevorzugt für Halogen, C₁-C₄-Alkyl, C₁-C₄-Halogenalkyl oder C₁-C₄-Alkoxy steht,
- A₄: für CR⁴ oder Stickstoff wobei R⁴ für Wasserstoff oder C₁-C₄-Alkyl steht, bevorzugt für Wasserstoff steht,
- B₁: für CR⁵ steht, und R⁵ für Halogen, gegebenenfalls mit F substituiertes C₁-C₆-Alkoxy oder C₁-C₆-Alkyl steht, bevorzugt für Halogen oder gegebenenfalls mit F substituiertem C₁-C₄-Alkyl oder mit F substituiertem C₁-C₄-Alkoxy steht und besonders bevorzugt für Cl, Brom, Trifluormethyl, Difluormethyl, Difluormethoxy, Trifluormethoxy, Ethyl oder Methyl steht,
- B₂: für CR⁶ steht, wobei R⁶ für Wasserstoff oder C₁-C₆-Alkyl steht, bevorzugt für Wasserstoff steht,
- B₃: für CR⁷ steht wobei R⁷ für Halogen, Cyano, Nitro oder C₁-C₆-Halogenalkyl steht, bevorzugt für perhalogeniertes C₁-C₄-Alkyl, besonders bevorzugt für perfluoriertes C₁-C₄-Alkyl, ganz besonders bevorzugt für -C₃F₇ steht, oder
- B₃: für CR⁷ steht wobei R⁷ für Halogen, Cyano, Nitro, Phenyl oder C₁-C₆-Halogenalkyl steht, bevorzugt für perhalogeniertes C₁-C₄-Alkyl oder Phenyl, besonders bevorzugt für perfluoriertes C₁-C₄-Alkyl oder Phenyl, ganz besonders bevorzugt für -C₃F₇ oder Phenyl steht,
- B₄: für CR⁸ steht, wobei R⁸ für Wasserstoff oder C₁-C₆-Alkyl steht, bevorzugt für Wasserstoff steht,
- B₅: für CR⁹ steht wobei R⁹ für Halogen, C₁-C₆-Alkoxy oder C₁-C₆-Alkyl steht, bevorzugt für Halogen oder C₁-C₄-Alkyl steht und besonders bevorzugt für Cl, Br, I oder Methyl steht,
- Q: für Wasserstoff, Hydroxy, Formyl oder gegebenenfalls substituiertes C₃-C₆-Cycloalkyl steht, bevorzugt gegebenenfalls unabhängig voneinander mit Nitro, Amino, Halogen, C₁-C₄-Alkoxy, Cyano oder Hydroxycarbonyl substituiertes C₃-C₆-Cycloalkyl, mehr bevorzugt für gegebenenfalls unabhängig voneinander mit F, Cl, Br, I oder CN substituiertes Cyclopropyl, besonders bevorzugt für Cyclopropyl, mit Fluor oder Chlor monosubstituiertes Cyclopropyl oder Cyano-Cyclopropyl (1-Cyano-Cyclopropyl oder 2-Cyano-Cyclopropyl), ganz besonders bevorzugt für Cyclopropyl oder 1-Cyano-Cyclopropyl, steht, oder
- Q: für Cyclopropyl, mit Fluor oder Chlor monosubstituiertes Cyclopropyl oder Cyano-Cyclopropyl (1-Cyano-Cyclopropyl oder 2-Cyano-Cyclopropyl) oder jeweils gegebenfalls mit F substituiertes Methyl, Ethyl Propyl oder Butyl, ganz besonders bevorzugt für Cyclopropyl oder 1-Cyano-Cyclopropyl oder Methyl oder Ethyl oder mit ein, zwei oder drei F substituiertes Methyl oder mit ein, zwei, drei, vier oder fünf F substituiertes Ethyl, steht,
wobei aber nicht mehr als zwei der chemischen Gruppierungen A₁ bis A₄ gleichzeitig für Stickstoff stehen.

Eine weitere bevorzugte Ausführungsform bezieht sich auf Verbindungen der Formel (I) in der
- W: für Sauerstoff steht,
- R¹⁰: für Wasserstoff und R¹ für Wasserstoff oder Methyl steht,
- A₁: für CR² steht wobei R² für Wasserstoff oder C₁-C₄-Alkyl steht, bevorzugt für Wasserstoff steht,
- A₂: für Stickstoff steht,
- A₃: für CR³ steht wobei R³ für Halogen, C₁-C₄-Alkyl, C₁-C₄-Halogenalkyl oder C₁-C₄-Alkoxy steht,
- A₄: für CR⁴ wobei R⁴ für Wasserstoff oder C₁-C₄-Alkyl steht, bevorzugt für Wasserstoff steht,
- B₁: für CR⁵ steht, und R⁵ für Halogen, gegebenfalls mit F substituiertem C₁-C₆-Alkoxy oder C₁-C₆-Alkyl steht, bevorzugt für Halogen oder gegebenenfalls mit F substituiertem C₁-C₄-Alkyl oder mit F substituiertem C₁-C₄-Alkoxy steht und besonders bevorzugt für Cl, Brom, Trifluormethyl, Difluormethyl, Difluormethoxy, Trifluormethoxy, Ethyl oder Methyl steht,
- B₂: für CR⁶ steht, wobei R⁶ für Wasserstoff oder C₁-C₆-Alkyl steht, bevorzugt für Wasserstoff steht,
- B₃: für CR⁷ steht wobei R⁷ für Halogen, Cyano, Nitro oder C₁-C₆-Halogenalkyl steht, bevorzugt für perhalogeniertes C₁-C₄-Alkyl, besonders bevorzugt für perfluoriertes C₁-C₄-Alkyl, ganz besonders bevorzugt für -C₃F₇ steht, oder
- B₃: für CR⁷ steht wobei R⁷ für Halogen, Cyano, Nitro, Phenyl oder C₁-C₆-Halogenalkyl steht, bevorzugt für perhalogeniertes C₁-C₄-Alkyl oder Phenyl, besonders bevorzugt für perfluoriertes C₁-C₄-Alkyl oder Phenyl, ganz besonders bevorzugt für -C₃F₇ oder Phenyl steht,
- B₄: für CR⁸ steht, wobei R⁸ für Wasserstoff oder C₁-C₆-Alkyl steht, bevorzugt für Wasserstoff steht,
- B₅: für CR⁹ steht wobei R⁹ für Halogen, C₁-C₆-Alkoxy oder C₁-C₆-Alkyl steht, bevorzugt für Halogen oder C₁-C₄-Alkyl steht und besonders bevorzugt für Cl, Br, I oder Methyl steht,
- Q: für Wasserstoff, Hydroxy, Formyl oder gegebenenfalls substituiertes C₃-C₆-Cycloalkyl steht, bevorzugt gegebenenfalls unabhängig voneinander mit Nitro, Amino, Halogen, C₁-C₄-Alkoxy, Cyano oder Hydroxycarbonyl substituiertes C₃-C₆-Cycloalkyl, mehr bevorzugt für gegebenenfalls unabhängig voneinander mit F, Cl, Br, I oder CN substituiertes Cyclopropyl, besonders bevorzugt für Cyclopropyl, mit Fluor oder Chlor monosubstituiertes Cyclopropyl oder Cyano-Cyclopropyl (1-Cyano-Cyclopropyl oder 2-Cyano-Cyclopropyl), ganz besonders bevorzugt für Cyclopropyl oder 1 -Cyano-Cyclopropyl, steht oder
- Q: für Cyclopropyl, mit Fluor oder Chlor monosubstituiertes Cyclopropyl oder Cyano-Cyclopropyl (1-Cyano-Cyclopropyl oder 2-Cyano-Cyclopropyl) oder jeweils gegebenfalls mit F substituiertes Methyl, Ethyl Propyl oder Butyl, ganz besonders bevorzugt für Cyclopropyl oder 1-Cyano-Cyclopropyl oder Methyl oder Ethyl oder mit ein, zwei oder drei F substituiertes Methyl oder mit ein, zwei, drei, vier oder fünf F substituiertes Ethyl, steht.

Eine weitere bevorzugte Ausführungsform bezieht sich auf Verbindungen der Formel (I) in der
- W: für Sauerstoff steht,
- R¹⁰: für Wasserstoff und R¹ für Wasserstoff oder Methyl steht,
- A₁: für CR² steht wobei R² für Wasserstoff steht,
- A₂: für Stickstoff steht,
- A₃: für CR³ steht wobei R³ für F, Cl, Br, I, C₁-C₄-Alkyl, C₁-C₄-Halogenalkyl (bevorzugt perhalogeniertes C₁-C₄-Alkyl, ganz besonders bevorzugt perfluoriertes C₁-C₄-Alkyl (CF₃, C₂F₅, C₃F₇, C₄F₉), oder C₁-C₄-Alkoxy steht,
- A₄: für CR⁴ wobei R⁴ für Wasserstoff steht,
- B₁: für CR⁵ steht, und R⁵ für Halogen, gegebenenfalls mit F substituiertem C₁-C₆-Alkoxy oder C₁-C₆-Alkyl steht, bevorzugt für Halogen oder gegebenenfalls mit F substituiertem C₁-C₄-Alkyl oder mit F substituiertem C₁-C₄-Alkoxy steht und besonders bevorzugt für F, Cl, Br, Methyl oder Ethyl steht, ganz besonders bevorzugt für Cl, Brom, Trifluormethyl, Difluormethyl, Difluormethoxy, Trifluormethoxy, Ethyl oder Methyl steht,
- B₂: für CR⁶ steht, wobei R⁶ für Wasserstoff steht,
- B₃: für CR⁷ steht wobei R⁷ für perfluoriertes C₁-C₄-Alkyl, ganz besonders bevorzugt für C₃F₇ steht, oder
- B₃: für CR⁷ steht wobei R⁷ für perhalogeniertes C₁-C₄-Alkyl oder Phenyl, besonders bevorzugt für perfluoriertes C₁-C₄-Alkyl oder Phenyl, ganz besonders bevorzugt für -C₃F₇ oder Phenyl steht,
- B₄: für CR⁸ steht, wobei R⁸ für Wasserstoff steht,
- B₅: für CR⁹ steht wobei R⁹ für Halogen, C₁-C₆-Alkoxy oder C₁-C₆-Alkyl steht, bevorzugt für Halogen oder C₁-C₄-Alkyl steht und besonders bevorzugt für Cl, Br, I oder Methyl steht,
- Q: für gegebenenfalls unabhängig voneinander mit Nitro, Amino, Halogen, C₁-C₄-Alkoxy, Cyano oder Hydroxycarbonyl substituiertes C₃-C₆-Cycloalkyl, mehr bevorzugt für gegebenenfalls unabhängig voneinander mit F, Cl, Br, I oder CN substituiertes Cyclopropyl, besonders bevorzugt für Cyclopropyl, mit Fluor oder Chlor monosubstituiertes Cyclopropyl oder Cyano-Cyclopropyl (1-Cyano-Cyclopropyl oder 2-Cyano-Cyclopropyl), ganz besonders bevorzugt für Cyclopropyl oder 1-Cyano-Cyclopropyl, steht oder
- Q: für Cyclopropyl, mit Fluor oder Chlor monosubstituiertes Cyclopropyl oder Cyano-Cyclopropyl (1-Cyano-Cyclopropyl oder 2-Cyano-Cyclopropyl) oder jeweils gegebenfalls mit F substituiertes Methyl, Ethyl Propyl oder Butyl, ganz besonders bevorzugt für Cyclopropyl oder 1-Cyano-Cyclopropyl oder Methyl oder Ethyl oder mit ein, zwei oder drei F substituiertes Methyl oder mit ein, zwei, drei, vier oder fünf F substituiertes Ethyl, steht.

Weiterhin hierin beschrieben sind Verbindungen der Formel (I) 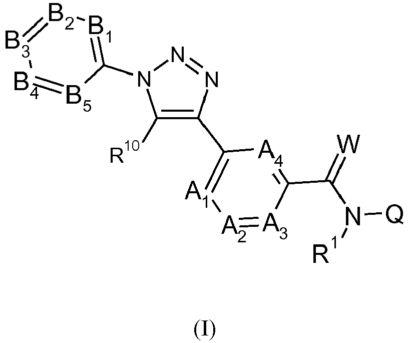 worin
- R¹: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl, bevorzugt für Wasserstoff oder Methyl, steht;
die chemischen Gruppierungen
- A₁: für CR² oder N,
- A₂: für N,
- A₃: für CR³ oder N, und
- A₄: für CR⁴ oder N stehen,
- B₁: für CR⁵ oder N,
- B₂: für CR⁶ oder N,
- B₃: für CR⁷ oder N,
- B₄: für CR⁸ oder N, und
- B₅: für CR⁹ oder N stehen,
wobei aber nicht mehr als zwei der chemischen Gruppierungen A₁ bis A₄ gleichzeitig für Stickstoff stehen und nicht mehr als zwei der Gruppierungen B₁ bis B₅ gleichzeitig für Stickstoff stehen;
- R² und R⁴: unabhängig voneinander für Wasserstoff, Methyl, Fluor und Chlor stehen; und
- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl, *N*-Methylamino, *N,N-*Dimethylamino, *N*-Ethylamino, *N,N*-Diethylamino, Methylsulfonylamino, *N*-Methyl-methylsulfonylamino steht;
- R⁵, R⁶, R⁸ und R⁹: unabhängig voneinander für Wasserstoff, Halogen, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(N-Methoxyimino)-ethyl, Methylsulfanyl, Trifluormethylsulfanyl, Methylsulfonyl, Methylsulfinyl, Trifluormethylsulfonyl, Trifluormethylsulfinyl, wobei aber R⁶ und R¹⁰ nicht gleichzeitig für Wasserstoff stehen,
- R⁷: für perfluoriertes C₁-C₆-Alkyl, perfluoriertes C₃-C₆-Cycloalkyl, C₁-C₆-Alkoxy, *N-*Alkoxyiminoalkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆alkylamino, sowie für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N-*Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht oder
- R⁷: für perhalogeniertes, bevorzugt perfluoriertes, C₁-C₆-Alkyl, perhalogeniertes, bevorzugt perfluoriertes, C₃-C₆-Cycloalkyl, C₁-C₆-Alkoxy, *N*-Alkoxyiminoalkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆alkylamino, sowie für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1 -Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino, Phenyl steht;
- R¹⁰: unabhängig voneinander für Wasserstoff, Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl, bevorzugt für Wasserstoff, steht;
- W: für Sauerstoff oder Schwefel, bevorzugt Sauerstoff, steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N-*Methylcarbamoyl)cyclopropyl, 1-(*N-*Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N-*Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;

Weiterhin hierin beschrieben sind Verbindungen der Formel (I)
worin
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
- A₁: für CH,
- A₂: für Stickstoff,
- A₃: für CR³, und
- A₄: für CH stehen;
- B₁: für CR⁵ oder N,
- B₂: für CH,
- B₃: für CR⁷,
- B₄: für CR⁸, und
- B₅: für CR⁹ oder N stehen;
- R³: für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, steht;
- R⁵, R⁶, R⁸ und R⁹: unabhängig voneinander für Wasserstoff, Halogen, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(N-Methoxyimino)-ethyl, Methylsulfanyl, Trifluormethylsulfanyl, Methylsulfonyl, Methylsulfinyl, Trifluormethylsulfonyl, Trifluormethylsulfinyl, wobei aber R6 und R10 nicht gleichzeitig für Wasserstoff stehen;
- R⁷: für Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Trifluormethylsulfonyl, Trifluormethylsulfinyl, Trifluormethylsulfanyl, steht; oder
- R⁷: für perfluoriertes, C₁-C₆-Alkyl, oder Phenyl steht;
- R¹⁰: unabhängig voneinander für Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl steht;
- W: für Sauerstoff steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N-*Methylcarbamoyl)cyclopropyl, 1-(*N-*Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht; wobei
- V: unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N-*Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht; und
- R⁵: für Wasserstoff, Methyl, Ethyl, 2-Methylethyl, tert.-Butyl, Fluor, Chlor, Brom, Iod, Nitro, Trifluormethyl, Difluormethoxy, Trifluormethoxy, Amino steht.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Weiterhin hierin beschrieben sind auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Weiterhin hierin beschrieben istdie Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Comitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella omata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Weiterhin hierin beschrieben sind Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Be-tracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

Die Verbindungen der Formel (I) können in Formulierungen bzw. in den aus diesen Formulierungen bereiteten Anwendungsformen in Mischung mit weiteren Verbindungen vorliegen, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothiophosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,
weitere Wirkstoffe wie beispielsweise Afidopyropen, Azadirachtin, Benclothiaz, Benzoximate, Bifenazate, Bromopropylate, Chinomethionat, Cryolite,

Dicofol, Diflovidazin, Fluensulfone, Flometoquin, Flufenerim, Flufenoxystrobin, Flufiprole, Fluopyram, Flupyradifurone, Fufenozide, Heptafluthrin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis von Bacillus firmus (I-1582, BioNeem, Votivo), sowie folgende Verbindungen:3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{5-[3-Chlor-5-(trifluormethyl)phenyl]-5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1), (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb,, (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl{3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3-methoxy-4-(prop-2-yn-1-yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)imino][6-(difluormethoxy)-2,3-difluorphenyl]methyl}-2-phenylacetamid, (15.85) N-{(Z)-[(Cyclopropylmethoxy)imino] [6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)methylene]amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl{6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134)1-{[rel(2R,3R)-3-(2-Chlorphenyl)-2-(2,4-difluorphenyl)oxiran-2-yl]methyl}-1H-1,2,4-triazol-5-yl thiocyanat, (15.135)5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl}-N-ethyl-N-methylimidoformamid, (15.155) N'-{5-Brom-6-[(cis-4-isopropylcyclohexyl)oxy]-2-methylpyridin-3-yl}-N-ethyl-N-methylimidoformamid, (15.156) N'- {5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis,* insbesondere *B. thuringiensis* subspecies *israelensis* (serotype H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii,* (ehemals bekannt als *Verticillium lecanii*), insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea),* insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als ,Inokulant' Pflanzen oder Pflanzenteilen oder Pflanzenorganen beigegeben werden und durch ihre besonderen Eigenschaften das Pflanzenwachstum und die Pflanzengesundheit fordern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia), Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,,,Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanzen, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie-Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin hierin beschrieben ist Saatgut, welches zum Schutz vor tierischen Schädlingen mit einer Verbindung der Formel (I) behandelt wurde. Ebenfalls beschrieben wird Saatgut, welches zur gleichen Zeit mit einer Verbindung der Formel (I) und Mischungspartner behandelt wurde. Weiterhin wird hierin Saatgut beschrieben, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren hierin beschrieben wird Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder, in einer weiteren Ausführungsform auch Endoparasiten. Der Begriff Endoparasiten schließt insbesondere Helminthen wie Cestoden, Nematoden oder Trematoden, und Protozoen wie Kokzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen sowie aquatische Ektoparasiten wie Copepoden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu diesen Parasiten gehören:
Aus der Ordnung der Anoplurida z.B. Haematopinus spp., Linognathus spp., Pediculus spp., Phthirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Stemostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroajacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.
Aus Unterklasse der Copepoden mit der Ordnung der Siphonostomatoida insbesondere die Gattungen Lepeophtheirus und Caligus, beispielhaft und besonders bevorzugt seien die Arten Lepeophtheirus salmonis, Caligus elongatus und Caligus clemensi genannt

Im Allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10 000 facher Verdünnung) anwenden oder sie als chemisches Bad verwenden.

Beim Einsatz im Bereich Tiergesundheit können die erfindungsgemäßen Wirkstoffe zur Erweiterung des Wirkspektrums in Kombination mit geeigneten Synergisten, Repellentien oder anderen Wirkstoffen wie beispielsweise Akariziden, Insektiziden, Anthelmintika, Mitteln gegen Protozoen, verwendet werden. Potentielle Mischpartner für erfindungsgemäße Verbindungen der Formel (I) können bei Anwendungen in der Tiergesundheit eine oder mehrere Verbindungen der Gruppen (In-1) bis (In-25) sein.
(In-1) Acetylcholinesterase (AChE) Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; besonders bevorzugt seien hier für Anwendungen gegen Ektoparasiten Bendiocarb, Carbaryl, Methomyl, Promacyl und Propoxur genannt; oder
   Organophosphate, z. B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion; besonders bevorzugt seien hier für Anwendungen gegen Ektoparasiten Azamethiphos, Chlorfenvinphos, Chlorpyrifos, Coumaphos, Cythioate, Diazinon (Dimpylate), Dichlorvos (DDVP), Dicrotophos, Dimethoate, Ethion (Diethion), Famphur (Famophos), Fenitrothion, Fenthion (MPP), Heptenophos, Malathion, Naled, Phosmet (PMP, Phtalofos) Phoxim, Propetamphos, Temephos, Tetrachlorvinphos (CVMP) und Triclorfon/Metrifonate genannt.
(In-2) GABA-gesteuerte Chlorid-Kanal-Antagonisten, wie beispielsweise Organochlorine, z. B. Bromocyclene, Chlordane und Endosulfan (alpha-), Heptachlor, Lindan, und Toxaphene; besonders bevorzugt seien hier für Anwendungen gegen Ektoparasiten Endosulphan (alpha-) und Lindan genannt; oder
   Fiprole (Phenylpyrazole), z. B. Acetoprole, Ethiprole, Fipronil, Pyrafluprole und Pyriprole, Rizazole; besonders, bevorzugt seien hier für Anwendungen gegen Ektoparasiten Fipronil und Pyriprole genannt; oder
   Arylisoxazoline, Arylpyrroline, Arylpyrrolidine z. B. Fluralaner (bekannt aus WO2009/2024541, Bsp . 11-1; aber auch Verbindungen aus WO2012007426, WO2012042006, WO2012042007, WO2012107533, WO2012120135, WO2012165186, WO2012155676, WO2012017359, WO2012127347, WO2012038851, WO2012120399, WO2012156400, WO2012163959, WO2011161130, WO2011073444, WO2011092287, WO2011075591, WO2011157748, WO 2007/075459, WO 2007/125984, WO 2005/085216, WO 2009/002809), Afoxolaner (z.B. in WO2011149749) und strukturell verwandte Arylpyrroline (bekannt z.B. aus WO20091072621, WO 2010020522, WO 2009112275, WO 2009097992, WO 2009072621, JP 2008133273, JP 2007091708), oder Arylpyrrolidine (z.B. in WO2012004326, WO2012035011, WO2012045700, WO 2010090344, WO 2010043315, WO 2008128711, JP 2008110971), und Verbindungen aus der Gruppe der sogenannten Metadiamide (bekannt z.B. aus WO2012020483, WO2012020484, WO2012077221, WO2012069366, WO2012175474, WO2011095462, WO2011113756, WO2011093415, WO2005073165) besonders bevorzugt sei hier für Anwendungen gegen Ektoparasiten Afoxolaner und Fluaralaner genannt.
(In-3) Natrium-Kanal-Modulatoren / Spannungsabhängige Natrium-Kanal-Blocker, wie beispielsweise Pyrethroide, z. B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1R)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)-Isomere], Tralomethrin, Transfluthrin und ZXI 8901; besonders bevorzugt seien hier für Anwendungen gegen Ektoparasiten die Typ I Pyrethroide Allethrin, Bioallethrin, Permethrin, Phenothrin, Resmethrin, Tetramethrin und die Typ II Pyrethroide (Alphacyanopyrethroide) Alpha-cypermethrin, Cyfluthrin (beta-), Cyhalothrin (lambda-), Cypermethrin (alpha-, zeta-), Deltamethrin, Fenvalerate, Flucythrinate, Flumethrin, Fluvalinate (tau-), und die esterfreien Pyrethroide Etofenprox und Silafluofen genannt; oder Organochlorverbindungen z. B. DDT; oder Methoxychlor. Wirkstoffe aus dieser Klasse eignen sich ganz besonders als Mischpartner da sie eine längeranhaltende Kontakt-repellierende Wirkung haben und somit das Wirkspektrum um diese Komponente erweitern.
(In-4) Nikotinerge Acetylcholin-Rezeptor-Agonisten, wie beispielsweise Neonikotinoide, z. B. Acetamiprid, Clothianidin, Dinotefuran, Imidacloprid, Imidaclothiz, Nitenpyram, Thiacloprid, Thiamethoxam; besonders bevorzugt seien hier für Anwendungen gegen Ektoparasiten Chlothianidin, Dinotefuran, Imidacloprid, Nitenpyram, und Thiacloprid genannt; oder Nikotin.
(In-5) Allosterische Acetylcholin-Rezeptor-Modulatoren (Agonisten), wie beispielsweise Spinosyne, z. B. Spinetoram und Spinosad; besonders bevorzugt seien hier für Anwendungen gegen Ektoparasiten Spinosad und Spinetoram genannt.
(In-6) Chlorid-Kanal-Aktivatoren, wie beispielsweise Avermectine/Milbemycine, z. B. Abamectin, Doramectin, Emamectin-benzoate, Eprinomectin, Ivermectin, Latidectin, Lepimectin, Milbemycin oxime, Milbemectin, Moxidectin, und Selamectin; Indolterpenoide wie z.B. Nodulisporinsäurederivate insbesondere Nodulisporinsäure A; besonders bevorzugt seien hier für Anwendungen gegen Ektoparasiten Doramectin, Eprinomectin, Ivermectin, Milbemycin oxime, Moxidectin, Selamectin und Nodulisporinsäure A genannt.
(In-7) Juvenilhormon-Analoge, z. B. Hydroprene (S-), Kinoprene, Methoprene (S-); oder Fenoxycarb; Pyriproxyfen; besonders bevorzugt seien hier für Anwendungen gegen Ektoparasiten Methoprene (S-) und Pyriproxyfen genannt.
(In-8) Milbenwachstumsinhibitoren, z. B. Clofentezine, Diflovidazin, Hexythiazox, Etoxazole; besonders bevorzugt sei hier für Anwendungen gegen Ektoparasiten Etoxazole genannt.
(In-9) Slo-1- und Latrophilin-Rezeptor Agonisten, wie beispielsweise zyklische Depsipeptide, z. B. Emodepsid sowie seine Ausgangsverbindung PF1022A (bekannt aus EP 382173, compound I); besonders bevorzugt sei hier für Anwendungen gegen Ektoparasiten Emodepsid genannt.
(In-10) Inhibitoren der oxidativen Phosphorylierung, ATP-Disruptoren, wie beispielsweise Diafenthiuron.
(In-12) Nikotinerge Acetylcholin-Rezeptor-Antagonisten, wie beispielsweise Bensultap, Cartap (-Hydrochlorid), Thiocylam, und Thiosultap (-sodium).
(In-13) Inhibitoren der Chitinbiosynthese, Typ 0, wie beispielsweise Benzoylharnstoffe, z. B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron; besonders bevorzugt seien hier für Anwendungen gegen Ektoparasiten Diflubenzuron, Fluazuron, Lufenuron und Triflumuron genannt.
(In-14) Inhibitoren der Chitinbiosynthese, Typ 1, wie beispielsweise Buprofezin.
(In-15) Häutungsstörende Wirkstoffe, wie beispielsweise Cyromazine und Dicyclanil; besonders bevorzugt seien hier für Anwendungen gegen Ektoparasiten Cyromazin und Dicyclanil genannt.
(In-16) Ecdysonagonisten/-disruptoren, wie beispielsweise Diacylhydrazine, z. B. Chromafenozide, Halofenozide, Methoxyfenozide und Tebufenozide.
(In-17) Oktopaminerge Agonisten, wie beispielsweise Amitraz, Cymiazole, Chlordimeform und Demiditraz; besonders bevorzugt seien hier für Anwendungen gegen Ektoparasiten Amitraz, Cymiazole und Demiditraz genannt.
(In-18) Komplex-III-Elektronentransportinhibitoren, wie beispielsweise Hydramethylnon; Acequinocyl; Fluacrypyrim.
(In-19) Komplex-I-Elektronentransportinhibitoren, wie beispielsweise aus der Gruppe der METI-Akarizide, z. B. Fenazaquin, Fenpyroximate, Pyrimidifen, Pyridaben, Tebufenpyrad, Tolfenpyrad; besonders bevorzugt seien hier für Anwendungen gegen Ektoparasiten Fenpyroximate, Pyrimidifen und Tolfenpyrad genannt;
(In-20) Blocker des spannungsabhängigen Natriumkanals, wie beispielsweise Indoxacarb und Metaflumizone; besonders bevorzugt seien hier für Anwendungen gegen Ektoparasiten Indoxacarb und Metaflumizone genannt.
(In-21) Inhibitoren der Acetyl-CoA-Carboxylase, wie beispielsweise Tetronsäure-Derivate, z. B. Spirodiclofen und Spiromesifen; oder Tetramsäure-Derivate, z. B. Spirotetramat.
(In-22) Komplex-II-Elektronentransportinhibitoren, wie beispielsweise Cyenopyrafen.
(In-23) Ryanodinrezeptor-Effektoren, wie beispielsweise Diamide, z. B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).
(In-24) Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen 4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)-amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor (ebenfalls bekannt aus WO 2007/149134), 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911), 1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/ 043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO 2006/129714), 2-Cyano-3-(difluoromethoxy)-N-ethylbenzenesulfonamide (bekannt aus WO 2005/035486), N-[1-(2,3-Dimethylphenyl)-2-(3,5-dimethylphenyl)ethyl]-4,5-dihydro-2-thiazolamine (bekannt aus WO 2008/104503); Penigequinolone A (bekannt aus EP 2248422 (compound I) und WO 2009/060015 (compound No. 11).
(In-25) Als geeignete Synergisten bei Verwendung mit Ektoparasitiziden seien hier MGK264 (*N-*Octylbicycloheptencarboxamid), Piperonylbutoxid (PBO) und Verbutin genannt; besonders bevorzugt seien hier Piperonylbutoxid und MGK264 genannt.

Zusätzlich zu diesen Gruppen können in Mischungen oder in Kombinationsanwendung auch Kurzzeitrepellentien verwendet werden. Beispiele sind DEET (N,N-diethyl-3-methylbenzamide), icaridin (1-Piperidine carboxylic acid), (1S, 20S)-2-methylpiperidinyl-3-cyclohexene-1-carboxamide (SS220), indalone (butyl 3,4-dihydro-2, 2-dimethyl-4-oxo-2H-pyran-6-carboxylate), dihydronepetalactones, nootkatone, IR3535 (3-[N-butyl-N-acetyl]-aminopropionic acid ethyl ester), 2-Ethylhexane-1,3-diol, (1R,2R,5R)-2-(2-Hydroxypropan-2-yl)-5-methyl-cyclohexan-1-ol, Dimethyl benzene-1,2-dicarboxylate, Dodecanoic acid, Undecan-2-one, N,N-Diethyl-2-phenyl-acetamide und aetherische Öle oder andere Pflanzeninhaltsstoffe mit bekannter repellierender Wirkung wie z.B. Borneol, Callicarpenal, 1,8-Cineol (eucalyptol), Carvacrol, b-Citronellol, a-Copaene, Coumarin (oder seine synthetischen Derviate bekannt aus US20120329832), Beim Einsatz gegen Ectoparasiten besonders bevorzugt sind icaridin, indalone und IR3535 (3-[N-butyl-N-acetyl]-aminopropionic acid ethyl ester)

Von den vorstehend genannten Gruppen (In-1) bis (In-25) sind die folgenden Gruppen als Mischpartner bevorzugt: (In-2), (In-3), (In-4), (In-5), (In-6), (In-17), (In-25).

Besonders bevorzugte Beispiele für insektizid oder akarizid wirksame Verbindungen, Synergisten oder Repellentien als Mischpartner der erfindungsgemäßen Verbindungen der Formel (I) sind Afoxolaner, Allethrin, Amitraz, Bioallethrin, Chlothianidin, Cyfluthrin (beta-), Cyhalothrin (lambda-), Cymiazole, Cypermethrin (alpha-, zeta-), Cyphenothrin, Deltamethrin, Demiditraz, Dinotefuran, Doramectin, Eprinomectin, Etofenprox, Fenvalerate, Fipronil, Fluazuron, Flucythrinate, Flumethrin, Fluralaner, Fluvalinate (tau-), Icaridin, Imidacloprid, Ivermectin, MGK264, Milbemycin oxime, Moxidectin, Nitenpyram, Permethrin, Phenothrin, Piperonylbutoxid, Pyriprole, Resmethrin, Selamectin, Silafluofen, Spinetoram, Spinosad, Tetramethrin, Thiacloprid.

Beispiele für endoparasitizide Wirkstoffe als Mischungs- und Kombinationspartner sind zum Beispiel (ohne, dass diese oder die folgenden Aufzählungen einschränkend wirken sollen):

Anthelmintische Wirkstoffe sind zum Beispiel die folgenden gegen Nematoden, Trematoden und/oder Cestoden wirksamen Stoffe:
- aus der Klasse der makrozyklischen Lactone, zum Beispiel: Abamectin, Emamectin, Ivermectin, Milbemectin, Latidectin, Lepimectin, Selamectin, Doramectin, Eprinomectin, Moxidectin, Milbemycin, Nemadectin;
- aus der Klasse der Benzimidazole und Probenzimidazole, zum Beispiel: Albendazole, Albendazolsulfoxid, Cambendazole, Cyclobendazole, Febantel, Fenbendazole, Flubendazole, Mebendazole, Netobimin, Oxfendazole, Oxibendazole, Parbendazole, Thiabendazole, Thiophanate, Triclabendazole;
- aus der Klasse der Depsipeptide, bevorzugt zyklische Depsipetide, insbesondere 24-gliedrige zyklische Depsipetide, zum Beispiel: Emodepside, PF1022A (bekannt aus EP 382173, Verbindung I);
- aus der Klasse der Tetrahydropyrimidine, zum Beispiel: Morantel, Pyrantel, Oxantel;
- aus der Klasse der Imidazothiazole, zum Beispiel: Butamisole, Levamisole, Tetramisole;
- aus der Klasse der Aminophenylamidine, zum Beispiel: Amidantel, Deacyliertes Amidantel (dAMD), Tribendimidine;
- aus der Klasse der Aminoacetonitrile, zum Beispiel: Monepantel;
- aus der Klasse der Paraherquamide, zum Beispiel: Derquantel, Paraherquamide;
- aus der Klasse der Salicylanilide, zum Beispiel: Bromoxanide, Brotianide, Clioxanide, Closantel, Niclosamide, Oxyclozanide, Rafoxanide, Tribromsalan;
- aus der Klasse der substitutierten Phenole, zum Beispiel: Bithionol, Disophenol, Hexachlorophen, Niclofolan, Meniclopholan, Nitroxynil;
- aus der Klasse der Organophosphate, zum Beispiel: Coumaphos, Haloxon, Crufomate, Dichlorvos, Naphthalofos, Trichlorfon;
- aus der Klasse der Piperazinone / Chinoline, zum Beispiel: Praziquantel, Epsiprantel;
- aus der Klasse der Piperazine, zum Beispiel: Piperazine, Hydroxyzin;
- aus der Klasse der Tetrazykline, zum Beispiel: Chlortetracyclin, Doxycyclin, Oxytetracyclin, Rolitetracyclin, Tetracyclin;
- aus der Klasse der verschiedenen anderen Klassen, zum Beispiel: Amoscanate, Bephenium, Bunamidine, Clonazepam, Clorsulon, Diamfenetide, Dichlorophen, Diethylcarbamazine, Emetine, Hetolin, Hycanthone, Lucanthone, Miracil, Mirasan, Niridazole, Nitroxynile, Nitroscanate, Oltipraz, Omphalotin, Resorantel, Oxamniquine;

Beispiele für Wirkstoffe gegen Protozoen als Mischungs- und Kombinationsspartner sind zum Beispiel:
- aus der Klasse der Triazine, zum Beispiel: Toltrazuril, Diclazuril, Ponazuril, Letrazuril;
- aus der Klasse der Polyletherionophore, zum Beispiel: Salinomycin, Maduramicin, Narasin, Monensin;
- aus der Klasse der makrozyklischen Lactone, zum Beispiel: Erythromycin, Milbemycin;
- aus der Klasse der Chinolone, zum Beispiel: Enrofloxacin, Pradofloxacin;
- aus der Klasse der Chinine, zum Beispiel: Chloroquin;
- aus der Klasse der Pyrimidine, zum Beispiel: Pyrimethamin;
- aus der Klasse der Sulfonamide, zum Beispiel: Sulfaquinoxaline, Trimethoprim, Sulfaclozin;
- aus der Klasse der Thiamine, zum Beispiel: Amprolium;
- aus der Klasse der Lincosamide, zum Beispiel: Clindamycin;
- aus der Klasse der Carbanilide, zum Beispiel: Imidocarb;
- aus der Klasse der Nitrofurane, zum Beispiel: Nifurtimox;
- aus der Klasse der Chinazolinon Alkaloide, zum Beispiel: Halofuginon;
- aus der Klasse der verschiedenen anderen Klassen, zum Beispiel: Oxamniquine, Paromomycin; aus der Klasse der Vaccinen oder Antigene von Mikroorganismen, zum Beispiel: Babesia canis canis, Babesia canis rossi, Babesia canis vogeli, Dictyocaulus viviparus, Eimeria acervulina, Eimeria brunetti, Eimeria maxima, Eimeria mitis, Eimeria necatrix, Eimeria praecox, Eimeria tenella, Leishmania infantum;

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid. Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenzbrechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Intermediate

Hierin beschrieben sind Intermediate der Formel (II), 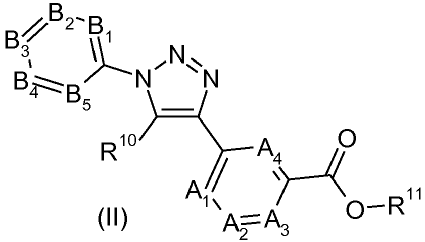 worin
A₁, A₂, A₃, A₄, B₁, B₂, B₃, B₄, B₅, und R¹⁰ die hierin genannten Bedeutungen haben und
R¹¹ für Wasserstoff, Methyl und Ethyl steht.

Weiterhin hierin beschrieben sind Intermediate der Formel (III) 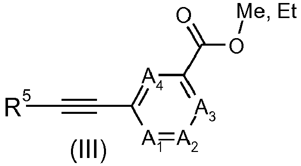 worin
A₁, A₂, A₃, A₄, und R⁵ die hierin genannten Bedeutungen haben.

### Herstellungsmethoden

Erfindungsgemäße Verbindungen der allgemeinen Struktur (I) können mit den entsprechenden Carbonsäureestern **(III)** über die Intermediate **(IIa** (Formel II, R¹¹ = Methyl, Ethyl)) nach dem im Reaktionsschema 1 angegebenen Verfahren hergestellt.

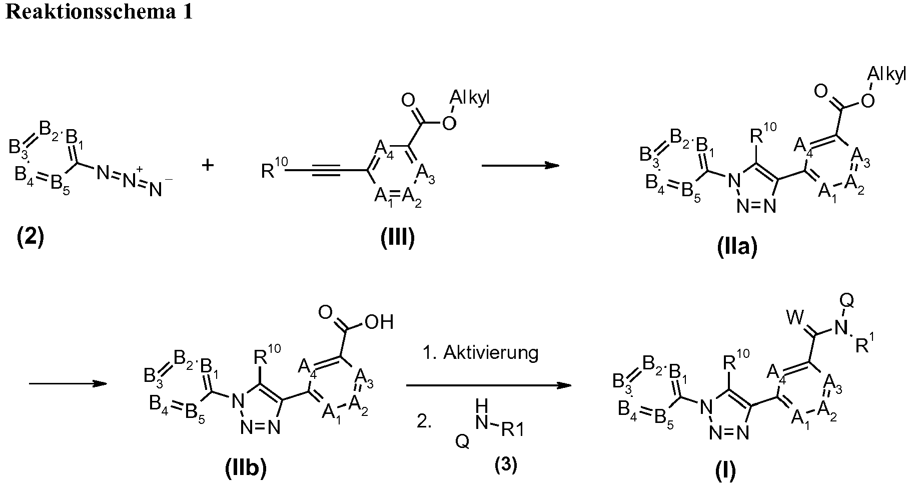

Die Reste A₁ bis A₄, B₁ bis B₅, Alkyl, Q, W, R¹, und R¹⁰ haben die oben beschriebenen Bedeutungen.

Erfindungsgemäße Verbindungen der allgemeinen Struktur (I) können mit den entsprechenden Carbonsäureestern **(III)** über die Intermediate **(IIa** (Formel II, R11 = Methyl, Ethyl)) hergestellt werden, aus denen die Verbindungen **(I)** nach dem Fachmann bekannten Verfahren sukzessiv gewonnen werden können. Verbindungen der Struktur **(IIa)** können nach literaturbekannten Verfahren aus den jeweiligen Halogencarbonsäureestern und Acetylenen hergestellt werden (z.B. WO2012/175474 A1, S. 117-118).

### Ringschluss mit Ester

### Stufe 1: Ester Ringschluss

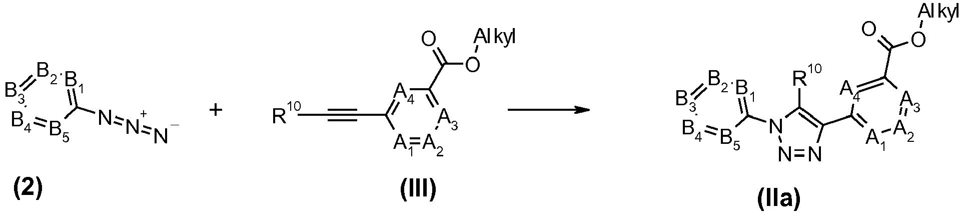

Erfindungsgemäße Verbindungen der allgemeinen Struktur **(IIa)** werden hergestellt indem man die Azide der Struktur **(2)** mit Acetylenen der Struktur **(III)** umsetzt.

Die Reste A¹-A⁴, B¹-B⁵, R¹⁰ und Alkyl haben die oben beschriebenen Bedeutungen.

Die Verbindungen der Strukturen **(2)** sind bekannt (z.B. WO2012175474) oder können nach literaturbekannten Methoden hergestellt werden. Die unbekannten Verbindungen der Struktur **(III)** können nach den für analoge Verbindungen beschriebenen Verfahren hergestellt werden (z.B. WO2012/175474 A1, S. 117-118). Als Beispiele für Verbindungen der Struktur **(2)** seien genannt: 5-Ethinyl-2-chlor-nicotinsäuremethylester, 5- Ethinyl -2-chlor-nicotinsäureethylester, 5-Ethinyl-2-methyl-nicotinsäuremethylester, 5- Ethinyl -2-methyl-nicotinsäureethylester. Als Beispiele der Verbindungen der Struktur **(III)** seien genannt: 2-Azido-1,3-dichlor-5-[1,2,2,2-tetrafluor-1-(trifluormethyl)ethyl]benzol, 2-Azido-1,3-dimethyl-5-[1,2,2,2-tetrafluor-1-(trifluormethyl)ethyl]benzol, 2-Azido-1-ethyl-3-methyl-5-[1,2,2,2-tetrafluor-1-(trifluormethyl)ethyl]benzol, 2-Azido-1-chlor-5-[1,2,2,2-tetrafluor-1-(trifluormethyl)ethyl]-3-(trifluormethyl)benzol, 2-Azido-1 -methyl-5-[1,2,2,2-tetrafluor-1-(trifluormethyl)ethyl]-3-(trifluormethyl)benzol, 2-Azido-1-chlor-5-[1,2,2,2-tetrafluor-1-(trifluormethyl)ethyl]-3-(trifluormethoxy)benzol, 2-Azido-1-methyl-5-[1,2,2,2-tetrafluor-1-(trifluormethyl)ethyl]-3-(trifluormethoxy)benzol. Die Reaktion wird unter den in der Literatur beschriebenen Bedingungen durchgeführt (z.B. WO2010008831, S. 52.; WO2012175474, S. 118).

### Stufen 2,3: Verseifung Amidierung

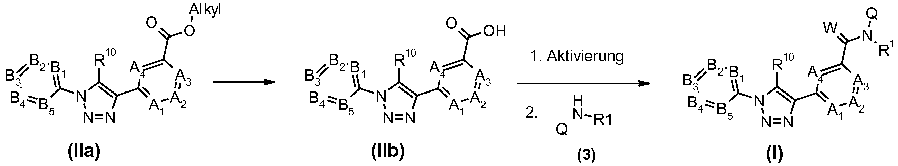

Erfindungsgemäße Verbindungen der allgemeinen Struktur **(I)** können in Analogie zu literaturbekannten Peptidkupplungsmethoden aus den Ausgangsmaterialien (**IIb**) und **(3)** hergestellt werden [WO2010-051926; WO2010-133312]. Verbindungen der allgemeinen Struktur (**IIb**) lassen sich in Analogie zu literaturbekannten Verfahren durch Esterspaltung aus Verbindungen der allgemeinen Struktur **(IIa)** herstellen [WO2010-051926; WO2010-133312]. Die Reste A¹-A⁴, B¹-B⁵, Alkyl, Q, R¹ und R¹⁰ haben die weiter oben beschriebenen Bedeutungen.

### Ringschluss mit Amid

Alternativ können erfindungsgemäße Verbindungen der allgemeinen Struktur **(I)** nach den in WO2012107434-A1 beschriebenen Verfahren aus Phenylaziden der Struktur **(2)** und Acetylen-Verbindungen der Strukturen **(3)** im Zuge einer [2+3]-Cycloaddition hergestellt werden. Verbindungen der Struktur **(2)** sind aus WO2012107434-A1 bekannt bzw. können nach literaturbekannten Verfahren hergestellt werden. Verbindungen der Struktur **(3)** können analog der für die Herstellung der Verbindungen der Struktur **(III)** verwendeten literaturbekannten Verfahren aus den jeweiligen Halogencarbonsäureamiden und Acetylenen hergestellt werden.

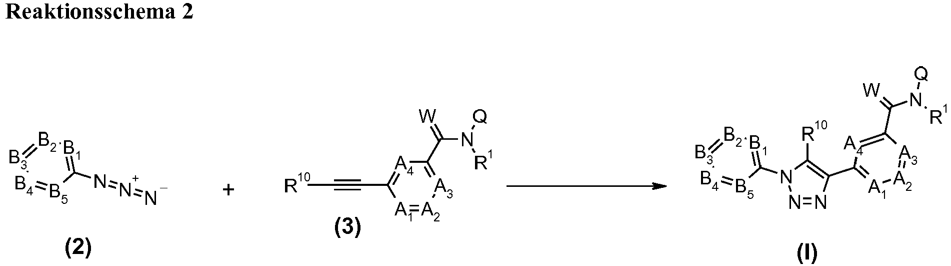

### Experimenteller Teil

### Beispiel 1

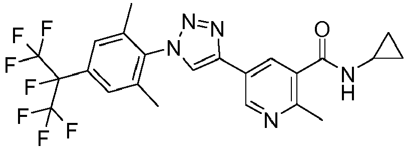

### Stufe 1

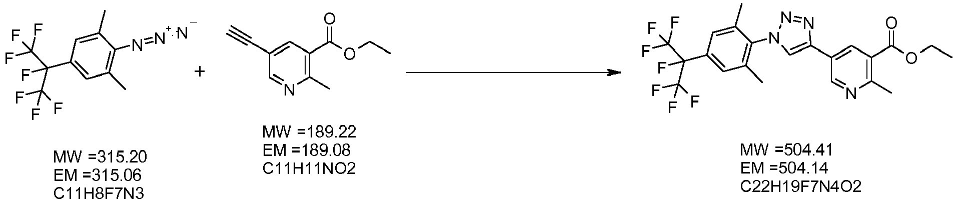

In einem 100 ml Dreihalskolben wurden 1,6 g (5,08 mmol) 2,6-Dimethyl-4-heptafluorisopropylphenylazid (hergestellt analog 2,6-Dichlor-4-heptafluorisopropyl-phenylazid, WO2012/175474, S. 117), 30 ml Acetonitril, 800 mg (4,23 mmol) 5-Ethinyl-2-methyl-pyridin-3-carbonsäureethylester, 0,65 g Diisopropylethylamin (5,03 mmol) und 0,1 g (0,525 mmol) Kupfer(I)iodid gemischt. Die entstandene Lösung wurde 12 Stunder bei Raumtemperatur unter Stickstoff als Schutzgas gerührt. Danach wurde im Vakuum eingedampft. Der Rückstand wurde dreimal mit je 20 ml Ethylacetat extrahiert. Die vereinigten organischen Phasen wurden dann einmal mit 20 ml gesättigter wässriger Kochsalzlösung gewaschen, mit wasserfreiem Natriumsulfat getrocknet und im Vakuum eingedampft. Der Rückstand an 5-[1-[2,6-Dimethyl-4-[1,2,2,2-tetrafluor-1-(trifluormethyl)ethyl]phenyl]triazol-4-yl]-2-methyl-pyridine-3-carbonsäureethylester wurde für die nächste Stufe verwendet.

Anaolog wurden folgende Verbindungen hergestellt:
2-Chlor-5-[1-[2,6-dimethyl-4-[1,2,2,2-tetrafluor-1-(trifluormethyl)ethyl]phenyl]triazol-4-yl]pyridine-3-carbonsäuremethylester
5-[1-[2,6-Dichlor-4-[1,2,2,2-tetrafluor-1-(trifluormethyl)ethyl]phenyl]triazol-4-yl]-2-methyl-pyridine-3-carbonsäureethylester
2-Chlor-5-[1-[2,6-dichlor-4-[1,2,2,2-tetrafluor-1-(trifluormethyl)ethyl]phenyl]triazol-4-yl]pyridine-3-carbonsäuremethylester

### Stufe 2

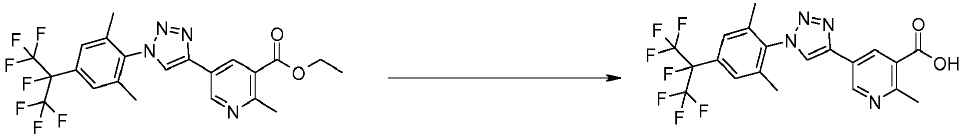

In einem 50 ml Kolben wurden 5-[1-[2,6-Dimethyl-4-[1,2,2,2-tetrafluor-1-(trifluormethyl)-ethyl]phenyl]triazol-4-yl]-2-methyl-pyridin-3-carbonsäureethylester (1,11 g, 2,2 mmol), Tetrahydrofuran (15 ml), Natriumhydroxid (300 mg, 7,5 mmol) und 5 ml Wasser zusammengegeben. Die entstandene Lösung wurde 12 Stunden bei Raumtemperatur gerührt. Danach wurde im Vakuum eingedampft. Der Rückstand wurde in etwas Wasser aufgenommen und der pH der Lösung durch Zugabe von 3 molarer wässriger Salzsäure auf einen Wert von 3-4 gebracht. Der Niederschlag wurde abfiltriert und getrocknet. Es wurden 849 mg 5-[1-[2,6-Dimethyl-4-[1,2,2,2-tetrafluor-1-(trifluormethyl)-ethyl]phenyl]triazol-4-yl]-2-methyl-pyridin-3-carbonsäure erhalten.

Anaolog wurden folgende Verbindungen hergestellt:
2-Chlor-5-[1-[2,6-dimethyl-4-[1,2,2,2-tetrafluor-1-(trifluormethyl)ethyl]phenyl]triazol-4-yl]pyridine-3-carbonsäure und als Nebenprodukt:
5-[1-[2,6-Dimethyl-4-[1,2,2,2-tetrafluor-1-(trifluormethyl)ethyl]phenyl]triazol-4-yl]-2-methoxy-pyridin-3-carbonsäure
5-[1-[2,6-Dichlor-4-[1,2,2,2-tetrafluor-1-(trifluormethyl)ethyl]phenyl]triazol-4-yl]-2-methyl-pyridine-3-carbonsäure
2-Chlor-5-[1-[2,6-dichlor-4-[1,2,2,2-tetrafluor-1-(trifluormethyl)ethyl]phenyl]triazol-4-yl]pyridine-3-carbonsäure und als Nebenprodukt:
   5-[1-[2,6-Dichlor-4-[1,2,2,2-tetrafluor-1-(trifluormethyl)ethyl]phenyl]triazol-4-yl]-2-methoxy-pyridin-3-carbonsäure

### Stufe 3

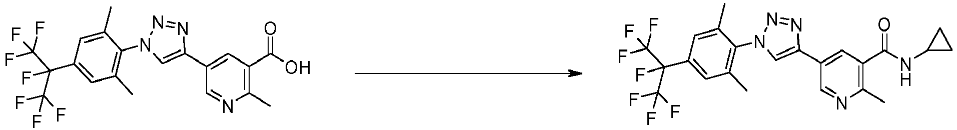

In einen 50 ml Dreihalskolben wurden 5-[1-[2,6-Dimethyl-4-[1,2,2,2-tetrafluor-1-(trifluormethyl)-ethyl]phenyl]triazol-4-yl]-2-methyl-pyridin-3-carbonsäure (481 mg, 1,01 mmol), Dioxan (20 ml), Cyclopropylamin (120 mg, 2,1 mmol), Diisopropylethylamin (390 mg, 3 mmol) und 2,4,6-Tripropyl-1,3,5,2,4,6-trioxatriphosphorinan-2,4,6-trioxid (1.0 ml einer 50 gewichtsprozentigen Lösung in Ethylacetat, 1,68 mmol) zusammengegeben. Die entstandene Lösung wurde 12 Stunden in einem Ölbad bei 50 °C gerührt. Danach wurde im Vakuum eingedampft. Der Rückstand wurde dreimal mit je 20 ml Ethylacetat extrahiert und die vereinigten organischen Phasen dann einmal mit gesättigter wässriger Kochsalzlösung gewaschen, mit wasserfreiem Natriumsulfat getrocknet und im Vakuum eingedampft. Das rohe Produkt wurde über präparative HPLC gereinigt. Es wurden 113,9 mg N-Cyclopropyl-5-[1-[2,6-dimethyl-4-[1,2,2,2-tetrafluor-1-(trifluormethyl)-ethyl]phenyl]triazol-4-yl]-2-methyl-pyridin-3-carboxamid erhalten.
HPLC-MS^{a)}: logP = 3.33, Masse (m/z) = 516 [M+H]+.
1H NMR (Bruker AV400, 400 MHz, d6-DMSO, ppm): δ 9,074 (s, 1H), 9,030-9,044 (d, 1H), 8,606-8,617 (d, 1H), 8,148-8,154 (d, 1H), 7,698 (s, 2H), 2,847-2,884 (m. 1H), 2,55 (s, 3H), 2,112 (s, 6H), 0,702-0,749 (m, 2H), 0.540-0.578 (m, 2H)

### Synthese der Ausgangsprodukte

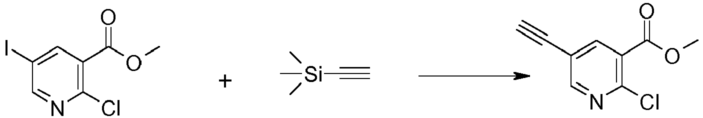

In einen 250 ml Kolben, entlüftet und unter Stickstoff Atmosphäre wurden 2-Chlor-5-iodpyridin-3-carbonsäuremethylester (20 g, 67,23 mmol), Triethylamin (200 ml), Ethinyltrimethylsilan (8,0 g, 81,45 mmol), Kupfer(I)iodid (3,0 g, 15,71 mmol), Triphenylphosphin (4,3 g, 16,39 mmol) und Bis-(triphenylphosphin)-palladium(II)-dichlorid (3,04 g, 4,33 mmol) gegeben. Die entstandene Lösung wurde 12 Stunden in einem Ölbad auf 50°C erhitzt. Danach wurden Methanol (40 ml) und Kaliumcarbonat (4,0 g, 28,9 mmol) unter Rühren zugefügt. Die entstande Lösung wurde dann für 2 Stunden bei Raumtemperatur gerührt. Ungelöste Feststoffe wurden dann abfiltriert. Das Filtrat wurde dreimal mit je 50 ml Ethylacetat extrahiert und die vereinigten organischen Phasen mit gesättigter wässriger Kochsalzlösung gewaschen. Die organische Phase wurde dann mit wasserfreiem Natriumsulfat getrocknet und im Vakuum eingedampft. Der Rückstand wurde über eine Säule mit Kieselgel und Petrolether/Ethylacetat 10:1 (v/v) als Laufmittel chromatographiert. Es wurden 7,5 g 2-Chlor-5-ethinylpyridin-3-carbonsäuremethylester erhalten.
¹H NMR (Bruker AV-III, 300 MHz, CDCl₃, 25°C): 8.70-8.71 (1H, d), 8.33-8.34 (1H, d), 4.68 (1H, s), 3.89 (3H, s).

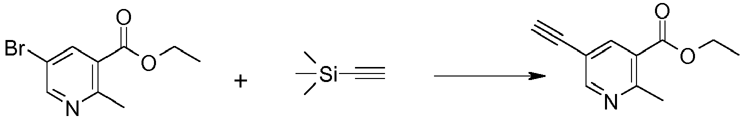

In einen 250 ml Kolben, entlüftet und unter Stickstoff Atmosphäre wurden 5-Brom-2-methylpyridin-3-carbonsäureethylester (20 g, 81,94 mmol), Triethylamin (150 ml), Ethinyltrimethylsilan (10,2 g, 103,85 mmol), Kupfer(I)iodid (3,3 g, 17,32 mmol), Triphenylphosphin (4,3 g, 16,39 mmol) und Bis-(triphenylphosphin)-palladium(II)-dichlorid (3,1 g, 4,42 mmol) gegeben. Die entstandene Lösung wurde 12 Stunden in einem Ölbad auf 50°C erhitzt. Danach wurden Methanol (100 ml) und Kaliumcarbonat (4,0 g, 28,9 mmol) unter Rühren zugefügt. Die entstande Lösung wurde dann für 2 Stunden bei Raumtemperatur gerührt. Ungelöste Feststoffe wurden dann abfiltriert. Das Filtrat wurde dreimal mit je 50 ml Ethylacetat extrahiert und die vereinigten organischen Phasen mit gesättigter wässriger Kochsalzlösung gewaschen. Die organische Phase wurde dann mit wasserfreiem Natriumsulfat getrocknet und im Vakuum eingedampft. Der Rückstand wurde über eine Säule mit Kieselgel und Petrolether/Ethylacetat 10:1 (v/v) als Laufmittel chromatographiert. Es wurden 3,5 g 5-Ethinyl-2-methyl-pyridin-3 -carbonsäureethylester erhalten.
¹H NMR (Bruker AV-III, 300 MHz, CDCl₃, 25°C): 8.70-8.71 (1H, d), 8.33-8.34 (1H, d), 4.68 (1H, s), 3.89 (3H, s).

### Herstellung des Ausgangsprodukts 4-Heptafluorisopropyl-2-methyl-6-trifluormethyl-anilin

### Verfahren 1:

4-Heptafluorisopropyl-2-methyl-6-trifluormethyl-anilin kann ausgehend von 2-Methyl-6-trifluormethyl-anilin nach dem in Reaktionsschema angegebenen Verfahren durch Umsetzung mit Heptafluorisopropyliodid in Gegenwart von Wasserstoffperoxid hergestellt werden. 2-Methyl-6-trifluormethyl-anilin ist literaturbekannt (John P. Chupp, Terry M. Balthazor, Michael J. Miller, and Mark J. PozzoJ. Org. Chem. 49 (1984),4711-4716 oder Thomas E. Nickson J. Org. Chem. 51 (1986) 3903-3904) und Heptafluorisopropyliodid ist kommerziell erhältlich.

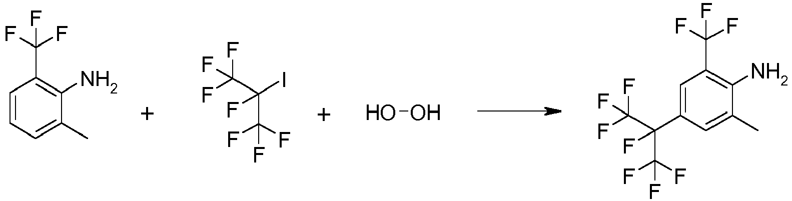

In einem Dreihalskolben wurden 17,48 g (100 mmol) 2-Methyl-6-trifluormethyl-anilin in 498 ml Dimethylsulfoxid vorgelegt und dann 44,3g (21,095 ml, 150 mmol) 2-Iodheptafluorpropan, 29,9 ml (29,9 mmol) 1 molare Eisen(II)-sulfatlösung in Wasser und 5,43 ml (104 mmol) 96 %ige Schwefelsäure zugegeben. Die Mischung wurde dann mit Argon entgast und dann mittels einer Spritzenpumpe 20,4 ml 30 %ige wässrige Wasserstoffperoxid Lösung innerhalb von 15 Minuten zugetropft. Die Temperatur stieg auf 54°C. Gegen Ende des Zutropfens wurde noch kurzzeitig auf 60°C erwärmt. Der Ansatz wurde 20 Minuten ohne Heizung nachgerührt, dabei sank die Temperatur auf 36 °C. Zur Aufarbeitung wurde der Ansatz auf gesättigte wässrige Natriumhydrogencarbonatlösung gegossen und das Produkt mit Essigester extrahiert. Die vereinigten Extrakte wurden erst mit Wasser und danach mit gesättigter wässriger Kochsalzlösung gewaschen, mit Natriumsulfat getrocknet und am Rotationsverdampfer im Vakuum eingedampft. Zur Reinigung wurde in zwei Portionen über eine Säule mit 120 g Kieselgel und einem Gradienten von reinem Cyclohexan bis Cyclohexan/Essigester 95:5 (v/v) chromatographiert. Es wurden 18,9 g 4-Heptafluorisopropyl-2-methyl-6-trifluormethyl-anilin erhalten.

Analog wurde auch 2-Chlor-4-heptafluorisopropyl-6-trifluormethyl-anilin ausgehend von 2-Chlor-6-trifluormethyl-anilin und 2-Iodheptafluorpropan erhalten:

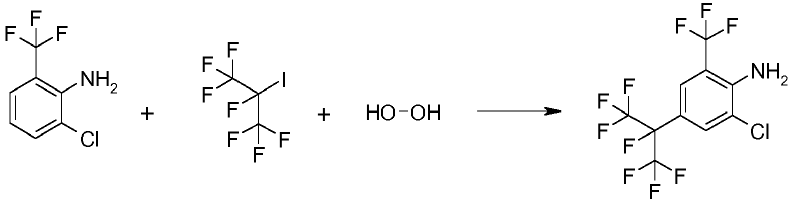

In einem Dreihalskolben wurden 30 g (0,153 mol) 2-Chlor-6-trifluormethylanilin (kommerziell erhältlich) in 765 ml Dimethylsulfoxid (DMSO) vorgelegt und dann 68,1 g (0,23 mol) 2-Iodheptafluorpropan, 46 ml einer 1 molaren wässrigen Eisen(II)-sulfatlösung und 15,4 g 98%ige Schwefelsäure zugegeben. Die Mischung wurde mit Argon entgast und dann mit einer Spritzenpumpe 34,8 g 30 %ige wässrige Wasserstoffperoxid Lösung innerhalb von 30 Minuten zugetropft. Dabei stieg die Temperatur auf 70°C. Der Ansatz wurde 20 Minuten nachgerührt, dabei sank die Temperatur auf 30 °C. Die Reaktionsmischung wurde dann auf gesättigte wässrige Natriumhydrogencarbonatlösung gegossen und mit Ethylacetat extrahiert. Die vereinigten Extrakte wurden zuerst mit Wasser, dann mit gesättigter wässriger Bisulfitlösung und gesättigter wässriger Kochsalz Lösung gewaschen, mit Natriumsulfat getrocknet und im Vakuum am Rotationsverdampfer eingedampft. Zur Reinigung wurde über eine Kartusche mit 330 g Kieselgel und einem Gradienten ausgehend von reinem Cyclohexan bis Cyclohexan/Ethylacetat 90:10 (v/v) chromatographiert. Es wurden 46,1 g 2-Chlor-4-heptafluorisopropyl-6-trifluormethyl-anilin erhalten.

### Herstellung des Ausgangsprodukts 4-Heptafluorisopropyl-2-methyl-6-trifluormethyl-anilin

### Verfahren 2:

Weiterhin kann 4-Heptafluorisopropyl-2-methyl-6-trifluormethyl-anilin ausgehend von literaturbekanntem 4-Heptafluorisopropyl-2-methyl-anilin (z.B. US2004/92762) nach dem imfolgenden Schema angegebenen Verfahren durch Umsetzung mit Natrium-trifluormethylsulfinat in Gegenwart von Oxidationsmitteln und Übergangsmetall Katalysatoren hergestellt werden.

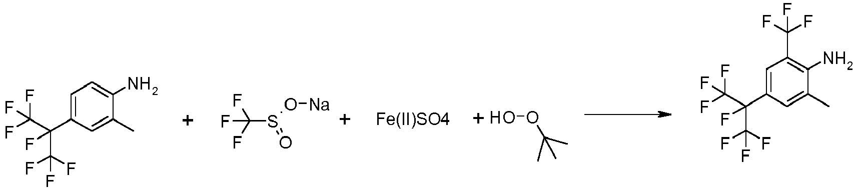

In einem 1000 ml Dreihalskolben wurden 25 g (91 mmol) 4-Heptafluorisopropyl-2-methylanilin zu einer Mischung aus 363,4 ml Wasser und 181,7 ml Acetonitril gegeben. Dann wurden 27,3 ml (27,3 mmol) wässrige 1 molare Eisen(II) sulfat-Lösung und 31,19g (200 mmol) Natrium-trifluormethylsulfinat zugefügt. Die Mischung wurde mit Argon entlüftet und anschließend 35,1 g (273 mmol) einer 70%igen wässrigen tert.-Butylhydroperoxid Lösung mit einer Spritzenpumpe innerhalb von 4,5 Stunden ohne Kühlung zudosiert. Die Temperatur stieg bis auf 34°C an. Nach beendeter Zugabe wurde 1 Stunde nachgerührt. Zur Aufarbeitung wurde der Ansatz auf 425 ml gesättigte wässrige Natriumhydrogensulfit-Lösung gegossen und 15 Minuten gerührt. Dann wurden 425 ml gesättigte Natriumhydrogencarbonat-Lösung zugegeben und dreimal mit Essigester extrahiert. Die vereinigten organischen Phasen wurden zuerst mit Wasser und dann mit gesättigter wässriger Kochsalzlösung gewaschen, mit Natriumsulfat getrocknet und im Vakuum am Rotationsverdampfer eingedampft. Das Rohprodukt wurde in zwei Portionen über eine Kartusche mit 120 g Kieselgel und einem Gradienten mit Cyclohexan/Essigester von 95:5 bis 85:15 (v/v) chromatographiert. Es wurden 19,5 g 4-Heptafluorisopropyl-2-methyl-6-trifluormethyl-anilin erhalten.
HPLC-MS^{a)}: logP = 4,67
GC/MS: Masse (m/z) = 343, Retentionszeit: 2,98 min, Kovacs Index: 1089
(Agilent 6890 GC, HP5979 MSD, 10m DB-1, iD=0.18mm, FILM=0.4µm, Inj.:250°C, const. flow: 1.6mm/min He, Det.:MSD:280°C, FID: 320°C, Oven:50°C(1 min) - 40°C/min - 320°C (3.25 min))
¹H-NMR (AV400, 400 MHz, d₃-Acetonitril): δ (ppm) = 7,50 (s, 1 H), 7,48 (s, 1H), 5,03 (s, 2H, breit), 2,23 (s, 3 H).

### Herstellung des Ausgangsprodukts 2-Chlor-6-ethyl-4-heptafluorisopropyl-anilin

Das Ausgangsprodukt 2-Chlor-6-ethyl-4-heptafluorisopropyl-anilin ist bisher in der Literatur noch nicht beschrieben. Die Herstellung kann aus literaturbekanntem 2-Ethyl-4-heptafluorisopropyl-anilin (z.B. US2002/198399) mittels bekannter chlorierender Verfahren erfolgen.

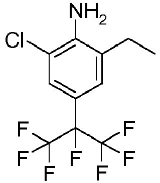

4,9 g (16,9 mmol) 2-Ethyl-4-heptafluorisopropyl-anilin (hergestellt nach US2002/198399) wurden in 100ml Chloroform vorgelegt, auf 45 - 50 °C erwärmt und dann 2,18ml, (26,7 mmol) Sulfurylchlorid, gelöst in in 400 ml Chloroform, langsam zugetropft. Den Ansatz wurde über Nacht bei 50°C gerührt danach wurden weitere 0,34 ml (4,2 mmol) Sulfurylchlorid gelöst in 2 ml Chloroform zugetropft und weitere 3 Stunden bei 50 °C gerührt. Danach wurde abgekühlt und das Lösungsmittel im Vakuum am Rotationsverdampfer abgezogen. Der Rückstand in Dichlormethan aufgenommen, erst mit Natriumhydrogensulfit und dann mit verdünnter Natronlauge gewaschen, mit Natriumsulfat getrocknet und das Lösungmittel im Vakuum am Rotationsverdampfer abdestilliert. Zur Reinigung wurde über eine Kartusche mit 120g Kieselgel mit einem Gradienten ausgehend von reinem Cyclohexan bis Cyclohexan/Ethylacetat 90:10 (v/v) chromatographiert. Es wurden 4,25 g 2-Chlor-6-ethyl-4-heptafluorisopropyl-anilin erhalten.
HPLC-MS^{a)}: logP = 4,67, Masse (m/z) = 324 [M+H]+.
¹H-NMR (AV400, 400 MHz, d₃-Acetonitril): δ (ppm) = 7,84 (s, 1 H), 7,82 (s, 1H), 7,53-7,56 (s, 2H, breit), 2,37 (q, J = 7,6 Hz, 2 H), 1,06 (t, J = 7,6 Hz, 3 H).

### Herstellung des Ausgangsprodukts 2-Brom-6-methyl-4-heptafluorisopropyl-anilin

Das Ausgangsprodukt 2-Brom-6-ethyl-4-heptafluorisopropyl-anilin ist bisher in der Literatur noch nicht beschrieben. Die Herstellung kann aus literaturbekanntem 2-Methyl-4-heptafluorisopropyl-anilin (z.B. US2004/92762) mittels bekannter bromierender Verfahren (z.B. EP2319830, S. 327) erfolgen.

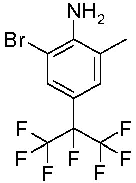

3,4g (12,356 mmol) 2-Methyl-4-heptafluorisopropyl-anilin wurden in 27 ml Dimethylformamid gelöst,dann 2,44g (13,6 mmol) N-Bromsuccinimid zugegeben und 1 Stunde bei 60°C gerührt. Der Ansatz wurde abgekühlt, mit Wasser versetzt und dreimal mit je 15 ml n-Hexan extrahiert. Die vereingten organischen Phasen wurden mit Wasser gewaschen, mit Natriumsulfat getrocknet und im Vakuum am Rotationsverdampfer eingedampft. Chromatographie über eine 120g Kartusche mit Kieselgel mit einen Gradienten beginnend mit reinem Cyclohexan nach Cyclohexan/Ethylacetat 90:10 (v/v) lieferte 2,44 g 2-Brom-6-ethyl-4-heptafluorisopropyl-anilin.
HPLC-MS^{a)}: logP = 4,38, Masse (m/z) = 354 [M+H]+.
¹H-NMR (AV400, 400 MHz, d₃-Acetonitril): δ (ppm) = 7,51 (s, 1 H), 7,23 (s, 1H), 4,86 (s, 2H, breit), 2,23 (s, 3 H).

Mit Hilfe der oben beschriebenen Darstellungsverfahren wurden die in der Tabelle 1 aufgeführten Verbindungen dargestellt.

**Tabelle 1**

| | | | | | | | | | | | | | |
|---|---|---|---|---|---|---|---|---|---|---|---|---|---|
| 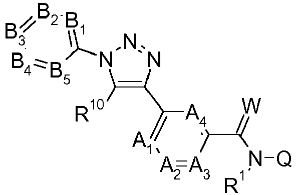 | | | | | | | | | | | | | |

| B2,B4 = C-H | | | | | | | | | | | | | |
|---|---|---|---|---|---|---|---|---|---|---|---|---|---|
| **Bsp.-Nr**. | **B₁** | **B₃** | **B₅** | **R¹** | **R¹⁰** | **A₁** | **A₂** | **A₃** | **A₄** | **W** | **Q** | **logP^{a)}** | **Masse [m/z] ^{a)1)}** |
| 1 | C-CH₃ | C-i-C₃F₇ | C-CH₃ | H | H | C-H | N | C-CH₃ | C-H | O | cyclopropyl | 3,3 | 516 |
| 2 | C-CH₃ | C-i-C₃F₇ | C-CH₃ | H | H | C-H | N | C-Cl | C-H | O | cyclopropyl | 3,8 | 536 |
| 3 | C-Cl | C-i-C₃F₇ | C-Cl | H | H | C-H | N | C-Cl | C-H | O | cyclopropyl | 3,9 | 576 |
| 4 | C-Cl | C-i-C₃F₇ | C-Cl | H | H | C-H | N | C-OCH₃ | C-H | O | 1-(cyano)cyclopropyl | 4,3 | 597 |
| 5 | C-Cl | C-i-C₃F₇ | C-Cl | H | H | C-H | N | C-CH₃ | C-H | O | cyclopropyl | 3,4 | 556 |
| 6 | C-Cl | C-i-C₃F₇ | C-Cl | H | H | C-H | N | C-CH₃ | C-H | O | 1-(cyano)cyclopropyl | 3,5 | 581 |
| 7 | C-CH₃ | C-i-C₃F₇ | C-CH₃ | H | H | C-H | N | C-OCH₃ | C-H | O | 1-(cyano)cyclopropyl | 4,2 | 557 |
| 8 | C-CH₃ | C-i-C₃F₇ | C-CH₃ | H | H | C-H | N | C-Cl | C-H | O | 1-(cyano)cyclopropyl | 3,8 | 561 |
| 9 | C-Cl | C-i-C₃F₇ | C-Cl | H | H | C-H | N | C-Cl | C-H | O | 1-(cyano)cyclopropyl | 3,8 | 601 |
| 10 | C-Cl | C-i-C₃F₇ | C-Cl | H | H | C-H | N | C-OCH₃ | C-H | O | cyclopropyl | 4,4 | 572 |
| 11 | C-CH₃ | C-i-C₃F₇ | C-CH₃ | H | H | C-H | N | C-OCH₃ | C-H | O | cyclopropyl | 4,3 | 532 |
| 12 | C-CH₃ | C-i-C₃F₇ | C-CH₃ | H | H | C-H | N | C-CH₃ | C-H | O | 1-(cyano)cyclopropyl | 3,4 | 541 |
| 13 | C-CF₃ | C-i-C₃F₇ | C-CH₃ | H | H | C-H | N | C-Cl | C-H | O | cyclopropyl | 3,9 | 590 |
| 14 | C-CF₃ | C-i-C₃F₇ | C-CH₃ | H | H | C-H | N | C-Cl | C-H | O | 1-(cyano)cyclopropyl | 3,9 | 615 |
| 15 | C-CF₃ | C-i-C₃F₇ | C-CH₃ | CH₃ | H | C-H | N | C-Cl | C-H | O | cyclopropyl | 4,3 | 604 |
| 16 | C-CF₃ | C-i-C₃F₇ | C-Cl | H | H | C-H | N | C-Cl | C-H | O | cyclopropyl | 4,0 | 610 |
| 17 | C-CF₃ | C-i-C₃F₇ | C-Cl | H | H | C-H | N | C-Cl | C-H | O | 1-(cyano)cyclopropyl | 3,9 | 635 |
| 18 | C-CH₃ | C-i-C₃F₇ | C-I | H | H | C-H | N | C-Cl | C-H | O | isopropyl | 4,2 | 602 |
| 19 | C-C₂H₅ | C-i-C₃F₇ | C-CH₃ | CH₃ | H | C-H | N | C-Cl | C-H | O | cyclopropyl | 4,6 | 564 |
| 20 | C-C₂H₅ | C-i-C₃F₇ | C-CH₃ | H | H | C-H | N | C-Cl | C-H | O | cyclopropyl | 4,1 | 550 |
| 21 | C-CH₃ | C-i-C₃F₇ | C-Br | CH₃ | H | C-H | N | C-Cl | C-H | O | cyclopropyl | 4,4 | 614 |
| 22 | C-CH₃ | C-i-C₃F₇ | C-Br | H | H | C-H | N | C-Cl | C-H | O | cyclopropyl | 3,9 | 600 |
| 23 | C-C₂H₅ | C-i-C₃F₇ | C-CH₃ | H | H | C-H | N | C-Cl | C-H | O | CH₂CF₃ | 4,5 | 592 |
| 24 | C-C₂H₅ | C-i-C₃F₇ | C-CH₃ | H | H | C-H | N | C-Cl | C-H | O | isopropyl | 4,4 | 552 |
| 25 | C-C₂H₅ | C-i-C₃F₇ | C-CH₃ | H | H | C-H | N | C-Cl | C-H | O | C₂H₅ | 4,1 | 538 |
| 26 | C-CH₃ | C-i-C₃F₇ | C-Br | H | H | C-H | N | C-Cl | C-H | O | CH₂CF₃ | 4,3 | 642 |
| 27 | C-CH₃ | C-i-C₃F₇ | C-Br | H | H | C-H | N | C-Cl | C-H | O | C₂H₅ | 3,9 | 588 |
| 28 | C-Cl | C-Ph | C-Cl | H | H | C-H | N | C-Cl | C-H | O | CH₂CF₃ | 3,9 | 526 |
| 29 | C-Cl | C-Ph | C-Cl | H | H | C-H | N | C-Cl | C-H | O | isopropyl | 3,8 | 486 |
| 30 | C-Cl | C-Ph | C-Cl | H | H | C-H | N | C-Cl | C-H | O | C₂H₅ | 3,5 | 472 |
| 31 | C-Cl | C-Ph | C-Cl | CH₃ | H | C-H | N | C-Cl | C-H | O | cyclopropyl | 4,0 | 498 |
| 32 | C-Cl | C-Ph | C-Cl | H | H | C-H | N | C-Cl | C-H | O | cyclopropyl | 3,5 | 484 |
| 33 | C-OCF₃ | C-i-C₃F₇ | C-Cl | H | H | C-H | N | C-Cl | C-H | O | cyclopropyl | 4,1 | 626 |
| 34 | C-OCF₃ | C-i-C₃F₇ | C-Cl | H | H | C-H | N | C-Cl | C-H | O | 1-(cyano)cyclopropyl | 4,0 | 651 |
| 35 | C-OCF₃ | C-i-C₃F₇ | C-Cl | CH₃ | H | C-H | N | C-Cl | C-H | O | cyclopropyl | 4,6 | 640 |
| 36 | C-OCHF₂ | C-i-C₃F₇ | C-Cl | H | H | C-H | N | C-Cl | C-H | O | cyclopropyl | 3,7 | 608 |
| 37 | C-OCHF₂ | C-i-C₃F₇ | C-Cl | CH₃ | H | C-H | N | C-Cl | C-H | O | cyclopropyl | 4,2 | 622 |
| 38 | C-OCHF₂ | C-i-C₃F₇ | C-Cl | H | H | C-H | N | C-Cl | C-H | O | 1-(cyano)cyclopropyl | 3,7 | 633 |

| | | | | | | | | | | | | | |
|---|---|---|---|---|---|---|---|---|---|---|---|---|---|
| ¹Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit² gekennzeichnet. ²Bei der angegebenen Masse handelt es sich um den Peak des Isotopenmusters des [M-H]⁻ Ions mit der höchsten Intensität. ^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50^{∗}4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System. | | | | | | | | | | | | | |

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾; ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Beispiel 1: ¹H-NMR(400.0 MHz, d₆-DMSO): δ= 9.074(4.6);9.044(2.0);9.039(2.1);8.617(1.0);8.606(1.0);8.154(2.0);8.148(2.0);7.698(3.7);4.44 8(0.4);3.904(4.4);3.388(9.7);3.371(0.7);3.364(0.8);3.333(42.6);3.168(0.4);2.884(0.4);2.875(0.7);2.865(0.7);2.856(0.4);2.847(0.3);2.676(0.4); 2.671(0.5);2.667(0.4);2.550(8.9);2.525(1.5);2.520(2.4);2.511 (31.1);2.507(63.6);2.502(84.9);2.498(64.1);2.493(32.7);2.334(0.4);2.329(0.5);2. 325(0.4);2.112(16.0);0.873(0.4);0.749(0.5);0.737(1.2);0.731 (1.8);0.719(1.6);0.714(1.4);0.702(0.6);0.578(0.6);0.568(1.7);0.562(1.5);0.558(1. 5);0.552(1.4);0.540(0.4);0.000(0.4) |
| Beispiel 2: ¹H-NMR(400.0 MHz, d₆-DMSO): δ= 9.128(4.1);9.018(2.2);9.012(2.2);8.769(1.2);8.758(1.2);8.329(2.2);8.323(2.2);7.704(4.0);4.45 1(0.4);3.904(2.7);3.395(1.0);3.388(0.9);3.381(1.1);3.335(151.7);2.873(0.5);2.864(0.7);2.854(0.7);2.845(0.5);2.836(0.4);2.676(0.5);2.672(0.6) ;2.668(0.5);2.507(75.3);2.502(98.1);2.498(76.8);2.329(0.6);2.325(0.5);2.114(16.0);0.765(0.4);0.752(1.3);0.748(1.8);0.735(1.7);0.730(1.4);0. 718(0.6);0.576(0.6);0.565(1.7);0.559(1.7);0.550(1.5);0.537(0.4) |
| Beispiel 3: ¹H-NMR(400.0 MHz, d₆-DMSO): δ= 9.272(11.9);9.040(6.2);9.034(6.1);8.769(3.4);8.758(3.4);8.364(6.4);8.358(6.1);8.219(16.0);3. 904(5.2);3.395(1.1);3.340(389.8);3.174(1.1);3.162(1.1);2.892(0.4);2.882(1.0);2.873(1.5);2.864(2.1);2.854(2.1);2.846(1.5);2.836(1.0);2.827(0. 4);2.672(1.2);2.507(155.4);2.503(191.9);2.499(144.9);2.334(0.9);2.330(1.2);0.766(1.3);0.752(4.1);0.748(5.2);0.735(5.0);0.730(4.2);0.718(1. 6);0.580(1.7);0.569(5.2);0.563(5.2);0.554(4.5);0.542(1.3);0.000(0.5) |
| Beispiel 4: ¹H-NMR(400.0 MHz, d₆-DMSO): δ= 9.175(7.0);9.158(3.3);8.889(3.4);8.883(3.5);8.548(3.7);8.542(3.6);8.211(7.9);4.465(0.4);4.45 2(1.2);4.438(0.4);4.024(16.0);3.904(5.1);3.395(3.0);3.388(2.1);3.382(3.1);3.336(204.7);2.676(0.5);2.672(0.7);2.667(0.5);2.542(0.4);2.525(2. 2);2.512(46.2);2.507(89.7);2.503(116.1);2.498(86.1);2.494(43.6);2.334(0.5);2.330(0.7);2.325(0.5);1.611(1.2);1.597(2.9);1.590(3.1);1.577(1. 4);1.336(1.5);1.323(3.0);1.316(3.2);1.301 (1.2);1.235(0.3);0.000(0.4) |
| Beispiel 5: ¹H-NMR(400.0 MHz, d₆-DMSO): δ= 9.213(9.3);9.056(3.7);9.051(3.9);8.630(1.9);8.619(2.0);8.214(9.9);8.165(3.7);8.160(3.8);4.46 5(0.5);4.451(1.3);4.437(0.5);3.904(4.0);3.395(3.0);3.388(2.0);3.382(3.0);3.336(129.7);3.175(0.6);3.162(0.6);2.894(0.6);2.885(0.8);2.876(1.3) ;2.866(1.3);2.857(0.8);2.848(0.6);2.676(0.4);2.672(0.6);2.667(0.4);2.562(16.0);2.539(0.4);2.525(1.8);2.520(2.6);2.512(34.1);2.507(69.7);2.5 03(92.9);2.498(70.4);2.494(36.1);2.334(0.4);2.329(0.6);2.325(0.4);0.750(0.8);0.737(2.2);0.732(3.2);0.720(2.9);0.714(2.5);0.703(1.1);0.582(1 .1);0.571(3.1);0.565(2.8);0.561 (2.7);0.555(2.6);0.543(0.8);0.000(0.5) |
| Beispiel 6: ¹H-NMR(400.0 MHz, d₆-DMSO): δ= 9.514(4.3);9.230(8.3);9.098(3.7);9.093(3.8);8.272(3.7);8.267(3.7);8.215(10.4);4.550(0.4);4.4 63(2.6);4.449(7.4);4.436(2.8);3.904(8.7);3.482(0.5);3.469(0.5);3.418(0.4);3.407(0.8);3.394(16.8);3.387(11.7);3.381(16.8);3.370(1.7);3.364(1 .3);3.332(178.3);3.174(0.9);3.161(0.9);2.676(0.9);2.671(1.3);2.667 (1.0);2.583(16.0);2.541(0.9);2.507(150.8);2.502(198.9);2.498(153.5);2.45 7(0.5);2.333(0.9);2.329(1.2);2.325(1.0);1.615(1.5);1.600(3.7);1.593(4.0);1.580(1.7);1.346(1.8);1.333(3.9);1.326(4.1);1.312(1.5);1.299(0.4);0. 891(0.6);0.873(1.2);0.855(0.6);0.000(0.8) |
| Beispiel 7: ¹H-NMR(400.0 MHz, d₆-DMSO): δ= 9.149(2.2);9.029(4.3);8.875(2.1);8.869(2.2);8.555(2.3);8.549(2.2);7.694(3.8);4.018(10.3);3.9 04(3.3);3.344(258.7);2.676(0.4);2.672(0.6);2.668(0.5);2.543(0.6);2.512(37.0);2.508(71.3);2.503(92.5);2.499(69.3);2.334(0.4);2.330(0.6);2.3 26(0.4);2.117(16.0);1.612(0.8);1.597(2.0);1.590(2.1);1.577(0.9);1.331(1.0);1.318(2.0);1.311(2.1);1.297(0.8) |
| Beispiel 8: ¹H-NMR(400.0 MHz, d₆-DMSO): δ= 9.677(2.3);9.143(4.8);9.058(2.4);9.052(2.5);8.441(2.5);8.435(2.4);7.704(3.7);4.467(1.6);4.45 3(4.5);4.440(1.7);3.904(6.2);3.469(0.4);3.419(0.3);3.407(0.7);3.395(11.2);3.388(7.4);3.382(10.9);3.370(1.8);3.340(224.0);3.175(0.4);3.162(0 .4);2.676(0.5);2.672(0.6);2.667(0.5);2.542(0.5);2.525(2.0);2.512(38.5);2.507(74.7);2.503(96.4);2.498(70.7);2.494(34.8);2.334(0.4);2.330(0.6 );2.325(0.4);2.115(16.0);1.643(0.8);1.629(1.9);1.622(2.0);1.609(0.9);1.332(1.0);1.318(2.1);1.312(2.1);1.297(0.9);0.892(0.4);0.873(0.7);0.855 (0.4) |
| Beispiel 9: ¹H-NMR(400.0 MHz, d₆-DMSO): δ=9.668(6.9);9.282(14.5);9.081(7.1);9.075(7.2);8.483(7.3);8.477(7.2);8.219(16.0);4.553(0.4);4.467(3.7);4.453(10.5);4.439(3.9);3.904(11.9);3.483(0.6);3.469(0.7);3.456(0.4);3.418(0.6);3.408(1.2);3.395(25.8);3.388(17.1);3.382(25.4);3.371(2.9);3.340(363.8);3.175(1.2);3.162(1.1);2.676(0.9);2.672(1.3);2.668(0.9);2.542(0.8);2.525(3.4);2.512(74.6);2.507(149.3);2.503(196.5);2.498(145.7);2.494(72.9);2.334(0.9);2.330(1.2);2.325(0.9);1.640(2.3);1.625(5.6);1.618(6.0);1.605(2.6);1.470(0.3);1.340(2.8);1.327(5.6);1.320(6.3);1.306(2.3);1.235(0.4);0.892(0.7);0.873(1.5);0.855(0.8);0.000(0.7) |
| Beispiel 10: ¹H-NMR(400.0 MHz, d₆-DMSO): δ=9.157(6.7);8.836(3.4);8.830(3.4);8.461(3.6);8.455(3.5);8.326(1.4);8.315(1.4);8.209(7.9);4.003(16.0);3.904(4.7);3.338(198.2);3.175(0.8);3.162(0.8);2.891 (0.5);2.881 (0.7);2.873(1.1);2.863(1.1);2.854(0.7);2.844(0.5);2.676(0.5);2.672(0.7);2.667(0.5);2.542(0.7);2.512(46.6);2.507(89.6);2.503(116.1);2.498(87.2);2.334(0.5);2.330(0.7);2.325(0.5);0.755(0.7);0.742(1.8);0.737(2.6);0.725(2.4);0.719(2.0);0.707(0.9);0.602(0.9);0.592(2.6);0.585(2.3);0.582(2.2);0.576(2.1);0.564(0.6);0.000(0.3) |
| Beispiel 11: ¹H-NMR(400.0 MHz, d₆-DMSO): δ= 9.012(4.7);8.822(2.4);8.816(2.5);8.471(2.5);8.465(2.4);8.315(0.9);8.304(1.0);7.692(3.8);3.998(11.2);3.904(2.7);3.332(55.2);3.175(0.8);3.162(0.8);2.891(0.3);2.881(0.5);2.872(0.7);2.862(0.7);2.854(0.5);2.844(0.4);2.672(0.3);2.525(1.1);2.512(20.3);2.507(40.0);2.503(52.5);2.498(39.3);2.494(19.9);2.329(0.3);2.115(16.0);0.754(0.5);0.741(1.2);0.736(1.8);0.724(1.6);0.718(1.4);0.706(0.6);0.600(0.6);0.590(1.8);0.583(1.5);0.579(1.5);0.574(1.4);0.561 (0.4) |
| Beispiel 12: ¹H-NMR(400.0 MHz, d₆-DMSO): δ= 9.509(2.3);9.094(4.4);9.085(2.0);9.080(2.0);8.255(1.9);8.250(1.9);7.700(3.8);4.463(1.1);4.4 50(3.3);4.436(1.2);3.904(4.3);3.395(6.9);3.388(5.0);3.381(6.7);3.370(0.8);3.364(0.6);3.332(105.1);3.174(0.4);3.161(0.4);2.675(0.5);2.671 (0. 6);2.667(0.5);2.570(8.5);2.542(0.5);2.524(1.9);2.507(77.0);2.502(98.2);2.498(72.1);2.333(0.4);2.329(0.6);2.325(0.4);2.114(16.0);1.617(0.8);1.63(2.0);1.596(2.1);1.583(0.9);1.341 (1.0);1.328(2.0);1.321 (2.1);1.306(0.8);0.873(0.5);0.000(0.4) |
| Beispiel 13: ¹H-NMR(400.0 MHz, CD3CN): δ= 9.017(0.4);9.011(0.4);8.988(11.8);8.982(11.7);8.484(16.0);8.334(13.5);8.328(13.1);8.077(8.4);8.022(8.6);7.080(3.0);5.447(0.4);4.086(1.6);4.068(4.8);4.050(4.8);4.032(1.6);2.895(0.7);2.885(2.1);2.876(3.1);2.867(4.6);2.857(4.7);2.849(3.1);2.839(2.2);2.830(0.8);2.467(0.4);2.462(0.5);2.457(0.4);2.147(122.9);2.124(51.3);2.107(1.5);2.101(1.0);2.095(0.6);2.086(1.0);1.971(21.5); 1.964(5.6);1.958(14.4);1.952(68.9);1.946(124.1);1.940(164.6);1.933(115.1);1.927(59.4);1.780(0.4);1.774(0.8);1.768(1.0);1.762(0.7);1.756(0.4);1.437(10.8);1.270(3.6);1.221(5.6);1.204(11.0);1.186(5.5);0.881(0.5);0.813(2.5);0.800(7.9);0.795(10.1);0.782(10.6);0.777(7.8);0.765(3.4);0.743(0.5);0.725(0.5);0.674(0.4);0.664(0.4);0.634(3.3);0.623(9.0);0.617(9.7);0.613(8.7);0.608(8.2);0.595(2.4);0.553(0.4);0.550(0.4);0.540(0.4);0.146(0.7);0.008(6.8);0.000(162.6);-0.008(8.8);-0.150(0.8) |
| Beispiel 14: ¹H-NMR(400.0 MHz, CD3CN): δ=9.033(11.1);9.027(11.0);8.497(16.0);8.404(12.1);8.398(11.6);8.079(8.2);8.024(8.4);7.737(4.8);5.447(0.7);4.086(2.3);4.068(6.9);4.051(7.0);4.033(2.4);2.355(0.4);2.330(0.5);2.287(0.9);2.278(0.7);2.150(197.9);2.126(56.0);2.107(3.1);2.101(2.2);2.095(1.6);2.087(3.5);1.972(30.8);1.964(6.3);1.958(16.3);1.952(75.5);1.946(134.9);1.940(178.1);1.934(124.2);1.928(64.1);1.808(0.3);1.781(0.5);1.774(0.9);1.768(1.1);1.762(0.8);1.756(0.5);1.651(0.3);1.613(4.7);1.598(12.6);1.592(12.5);1.578(6.3);1.538(0.8);1.437(1.4);1.432(0.9);1.392(6.4);1.378(12.6);1.371(12.8);1.357(4.7);1.319(0.4);1.285(0.4);1.271(1.2);1.222(8.1);1.204(15.8);1.186(7.9);1.152(3.1);1.140(9.4);1.132(9.5);1.121(4.1);1.081(0.6);0.980(0.5);0.940(4.1);0.929(9.3);0.922(9.3);0.909(3.0);0.146(0.6);0.000(140.9);-0.149(0.7) |
| Beispiel 15: ¹H-NMR(400.0 MHz, CD3CN): δ=8.979(3.2);8.974(3.0);8.473(5.0);8.290(3.0);8.285(2.8);8.229(0.4);8.077(3.3);8.023(3.3);4.068(0.6);4.050(0.6);3.077(16.0);2.821(2.7);2.809(0.4);2.797(0.8);2.791(0.9);2.782(1.4);2.771(0.9);2.764(0.7);2.754(0.4);2.464(0.4);2.410(0.3);2.149(221.2);2.128(15.8);2.107(1.1);2.101(0.7);2.095(0.5);2.086(0.4);2.026(0.4);1.972(3.6);1.952(60.1);1.946(102.5);1.940(127.2);1.934(90.5);1.928(46.9);1.780(0.3);1.774(0.6);1.768(0.7);1.762(0.5);1.437(0.5);1.349(1.2);1.271(0.6);1.222(0.7);1.204(1.3);1.186(0.7);0.857(0.5);0.843(0.5);0.801(0.7);0.591(2.5);0.549(2.6);0.533(2.3);0.000(72.0);-0.150(0.3) |
| Beispiel 16: ¹H-NMR(400.0 MHz, CD3CN): δ=8.997(1.5);8.991(1.5);8.530(2.0);8.345(1.8);8.339(1.6);8.290(1.1);8.128(1.1);7.052(0.4);2.876(0.4);2.867(0.6);2.857(0.6);2.849(0.4);2.134(65.1);2.113(0.9);2.106(1.0);2.100(0.6);2.094(0.4);1.963(4.7);1.957(12.0);1.951(59.2);1.945(106 .4);1.939(141.7);1.933(97.6);1.927(50.0);1.774(0.6);1.767(0.8);1.761(0.6);1.437(16.0);1.270(0.9);0.813(0.3);0.800(1.0);0.795(1.3);0.783(1.3);0.777(1.0);0.765(0.4);0.635(0.4);0.625(1.1);0.617(1.2);0.609(1.0);0.596(0.4);0.146(2.0);0.031(0.5);0.030(0.5);0.028(0.5);0.027(0.5);0.024(0.5);0.008(20.4);0.000(427.2);-0.009(18.9);-0.150(1.9) |
| Beispiel 17: ¹H-NMR(400.0 MHz, CD3CN): δ= 9.042(10.1);9.036(10.3);8.557(0.5);8.544(14.2);8.413(11.2);8.407(11.1);8.290(6.9);8.129(6.9);7.703(3.2);5.446(1.0);4.086(1.2);4.068(3.6);4.050(3.7);4.032(1.2);2.132(59.0);2.113(1.1);2.107(1.2);2.100(0.9);2.094(0.5);2.086(0.4);1.971(16.8);1.964(5.5);1.957(13.0);1.952(72.1);1.945(131.8);1.939(179.1);1.933(124.6);1.927(64.4);1.780(0.4);1.774(0.7);1.768(1.0);1.762(0.7);1.755(0.4);1.612(4.2);1.598(10.4);1.591(10.5);1.577(5.6);1.537(0.7);1.437(16.0);1.391(5.9);1.377(10.5);1.370(11.0);1.356(4.3);1.319(0.3);1.285(0.5);1.270(2.7);1.221(4.6);1.204(8.9);1.186(4.5);0.881(0.4);0.146(2.3);0.008(19.5);0.000(548.9);-0.009(23.0);-0.031(0.4);-0.150(2.4) |
| Beispiel 18: ¹H-NMR(400.0 MHz, d₆-DMSO): δ= 9.228(6.8);9.029(3.5);9.023(3.6);8.598(1.9);8.579(1.9);8.317(3.7);8.311(3.7);8.056(3.2);7.950(3.0);4.088(0.7);4.071(1.1);4.053(1.2);4.037(0.7);3.902(4.0);3.317(133.5);2.670(1.2);2.505(143.3);2.501(190.5);2.328(1.2);2.196(13.6);1.250(0.3);1.236(0.5);1.189(16.0);1.173(16.0);0.000(1.6) |
| Beispiel 19: ¹H-NMR(400.0 MHz, d₆-DMSO): δ= 9.146(1.2);9.131(7.2);9.021(0.7);9.015(0.8);9.009(3.7);9.003(3.8);8.446(3.8);8.440(3.8);8.348(0.6);8.342(0.6);7.706(3.0);7.668(3.0);3.902(7.5);3.317(134.1);3.037(16.0);2.819(0.3);2.810(0.7);2.802(0.8);2.793(1.3);2.782(3.5);2.766(0.4);2.675(0.8);2.670(1.1);2.666(0.9);2.506(127.3);2.501(172.2);2.497(135.2);2.414(0.9);2.396(2.8);2.377(2.8);2.358(1.0);2.332(0.8);2.328(1.0);2.324(0.8);2.103(13.1);1.249(0.4);1.236(0.5);1.058(3.9);1.048(1.2);1.039(8.5);1.029(1.9);1.020(3.9);1.011(0.9);0.834(0.5);0.824(0.4);0.815(0.5);0.782(0.5);0.610(0.4);0.589(2.1);0.581(3.1);0.571(1.1);0.551(1.4);0.538(2.1);0.522(2.0);0.504(0.4);0.000(1.9) |
| Beispiel 20: ¹H-NMR(400.0 MHz,d₆-DMSO): δ= 9.162(8.4);9.151(0.4);9.023(4.3);9.017(4.4);8.754(2.3);8.743(2.4);8.336(4.4);8.330(4.4);7.708(3.2);7.670(3.2);3.998(0.6);3.902(6.7);3.318(193.4);2.882(0.6);2.873(1.0);2.864(1.4);2.854(1.4);2.845(1.0);2.836(0.7);2.670(1.3);2.666(1.0);2.505(152.0);2.501(205.2);2.497(165.4);2.407(1.2);2.388(3.4);2.369(3.5);2.350(1.3);2.328(1.3);2.324(1.0);2.092(16.0);1.249(0.4);1.235(0 .7);1.041(4.8);1.022(10.0);1.003(4.5);0.764(0.8);0.751(2.6);0.747(3.4);0.734(3.3);0.729(2.8);0.717(1.1);0.577(1.1);0.566(3.3);0.559(3.3);0.550(2.9);0.538(0.9);0.000(1.4) |
| Beispiel 21: ¹H-NMR(400.0 MHz, d₆-DMSO): δ= 9.193(1.2);9.182(6.7);9.027(0.7);9.016(3.6);9.011(3.6);8.469(3.5);8.463(3.5);8.363(0.6);8.358(0.6);8.053(3.4);7.947(3.2);3.903(8.4);3.318(203.6);3.038(16.0);2.820(0.4);2.810(0.8);2.802(0.9);2.794(1.5);2.781(3.4);2.766(0.5);2.675(1.1);2.670(1.4);2.506(162.3);2.501(214.1);2.497(170.7);2.332(0.9);2.328(1.3);2.204(13.7);1.250(0.4);1.236(0.7);0.833(0.6);0.825(0.5);0.814(0.5);0.785(0.5);0.610(0.5);0.589(2.4);0.581(3.2);0.549(1.4);0.537(2.3);0.520(2.1);0.503(0.5);0.000(1.3) |
| Beispiel 22: ¹H-NMR(400.0 MHz, d₆-DMSO): δ= 9.208(7.9);9.030(4.1);9.024(4.1);8.759(2.4);8.749(2.5);8.344(4.2);8.338(4.2);8.055(3.6);7.949(3.4);3.902(2.7);3.378(0.5);3.322(383.3);3.281(0.7);2.882(0.7);2.873(1.0);2.864(1.4);2.855(1.4);2.846(1.0);2.837(0.7);2.670(1.4);2.501(223.7);2.328(1.4);2.192(16.0);1.248(0.4);1.236(0.6);0.765(0.9);0.752(2.7);0.747(3.5);0.735(3.4);0.729(2.9);0.718(1.1);0.578(1.1);0.567(3.5);0.561 (3.6);0.552(3.1);0.540(0.9);0.000(1.6) |
| Beispiel 23: ¹H-NMR(400.0 MHz, d₆-DMSO): δ= 9.459(1.2);9.443(2.4);9.428(1.2);9.200(9.1);9.071(4.6);9.065(4.7);8.373(4.7);8.367(4.7);8.313(0.6);7.707(3.1);7.669(3.1);4.200(0.6);4.175(1.9);4.159(1.9);4.151(2.1);4.135(1.9);4.127(0.8);4.111(0.6);3.316(112.0);2.675(0.6);2.671(0. 9);2.666(0.7);2.510(49.8);2.506(97.1);2.501(130.5);2.497(101.5);2.412(1.1);2.394(3.3);2.375(3.4);2.356(1.2);2.333(0.7);2.328(0.9);2.324(0.7);2.097(16.0);1.297(0.5);1.280(1.0);1.247(5.0);1.046(5.0);1.027(10.6);1.008(4.8);0.875(2.1);0.859(6.2);0.841(2.6);0.146(0.8);0.008(7.6);0.000(161.1);-0.008(8.4);-0.150(0.8) |
| Beispiel 24: ¹H-NMR(400.0 MHz, d₆-DMSO): δ= 9.178(7.1);9.021(3.6);9.015(3.7);8.592(1.5);8.572(1.5);8.311(4.1);8.305(3.7);7.706(2.3);7.667(2.3);4.088(0.6);4.072(0.9);4.053(1.0);4.037(0.6);3.314(63.3);3.291(0.5);2.675(0.6);2.670(0.8);2.666(0.6);2.523(2.6);2.510(43.3);2.506(87.1);2.501(118.3);2.497(90.4);2.492(46.3);2.411(0.8);2.392(2.5);2.373(2.6);2.355(0.9);2.332(0.6);2.328(0.8);2.324(0.6);2.096(12.2);1.246(1.0 );1.189(16.0);1.173(15.9);1.045(3.9);1.026(8.5);1.008(3.8);0.875(0.4);0.859(1.3);0.841(0.6);0.146(0.7);0.020(0.5);0.008(6.5);0.000(152.7);-0.008(6.9);-0.150(0.7) |
| Beispiel 25: ¹H-NMR(400.0 MHz, d₆-DMSO): δ= 9.171(9.7);9.029(5.0);9.023(5.1);8.713(1.1);8.700(2.1);8.686(1.1);8.343(5.2);8.337(5.2);8.313(1.7);7.706(3.1);7.668(3.0);4.033(0.3);3.334(1.3);3.315(93.8);3.302(3.9);3.298(3.6);3.284(2.9);3.266(0.9);2.679(0.3);2.675(0.7);2.670(0.9);2.666(0.7);2.524(2.9);2.510(51.8);2.506(104.0);2.501(141.4);2.497(110.7);2.492(58.7);2.411(1.1);2.392(3.4);2.373(3.5);2.354(1.2);2.337(0.4);2.333(0.7);2.328(1.0);2.324(0.8);2.096(16.0);1.246(0.7);1.169(5.9);1.151(12.5);1.133(5.7);1.045(5.1);1.026(11.1);1.007(4.9);0.859(0.9);0. 841(0.4);0.146(0.8);0.008(8.6);0.000(187.1);-0.008(10.0);-0.150(0.8) |
| Beispiel 26: ¹H-NMR(400.0 MHz, d₆-DMSO): δ= 9.463(1.1);9.448(2.4);9.432(1.1);9.249(9.0);9.080(4.5);9.074(4.6);8.381(4.7);8.375(4.6);8.313(0.5);8.053(3.3);7.949(3.1);4.200(0.6);4.176(1.9);4.160(1.9);4.152(2.0);4.136(1.9);4.128(0.8);4.112(0.6);3.315(96.3);2.675(0.8);2.670(1.1);2.666(0.8);2.506(126.2);2.501(168.9);2.497(129.8);2.332(0.8);2.328(1.1);2.324(0.8);2.198(16.0);1.279(0.4);1.247(1.9);0.875(0.8);0.859(2.3);0.841(1.0);0.146(0.9);0.008(9.5);0.000(197.7);-0.008(9.7);-0.150(1.0) |
| Beispiel 27: ¹H-NMR(400.0 MHz, d₆-DMSO): δ=9.219(10.0);9.035(4.9);9.029(5.0);8.718(1.1);8.704(2.1);8.690(1.0);8.350(5.2);8.344(5.1);8.313(1.1);8.051(3.2);7.948(3.0);5.753(0.4);3.334(1.4);3.315(99.7);3.303(3.7);3.299(3.4);3.285(2.8);3.267(0.9);2.675(0.7);2.670(1.0);2.666(0.7);2.523(3.1);2.510(52.3);2.506(104.9);2.501(142.4);2.497(109.4);2.492(55.8);2.337(0.3);2.333(0.7);2.328(0.9);2.324(0.7);2.196(16.0);1.169(5.8);1.151(12.5);1.133(5.7);0.859(0.4);0.146(0.9);0.008(8.4);0.000(191.4);-0.009(8.6);-0.150(0.8) |
| Beispiel 28: ¹H-NMR(400.0 MHz, d₆-DMSO): δ=9.463(0.9);9.448(2.0);9.432(0.9);9.292(8.0);9.108(4.0);9.102(4.1);8.416(4.1);8.410(4.1);8.3 13(0.5);8.166(16.0);7.908(2.5);7.905(3.4);7.888(3.7);7.885(3.3);7.582(0.9);7.578(1.3);7.573(0.7);7.561(3.8);7.542(3.4);7.536(1.7);7.532(2.4);7.522(0.7);7.515(1.6);7.497(0.5);4.204(0.5);4.180(1.6);4.164(1.6);4.156(1.7);4.140(1.6);4.132(0.7);4.115(0.5);3.315(81.0);2.675(0.7);2.670(1.0);2.666(0.8);2.510(56.3);2.506(110.3);2.501(148.5);2.497(114.4);2.492(59.3);2.333(0. 7);2.328(1.0);2.324(0.7);0.146(0.9);0.008(9.6);0.000(188.1);-0.008(9.1);-0.150(0.9) |
| Beispiel 29: ¹H-NMR(400.0 MHz, d₆-DMSO): δ=9.270(7.2);9.056(3.6);9.050(3.7);8.599(1.6);8.579(1.7);8.350(3.7);8.344(3.7);8.313(0.5);8.164(14.5);7.908(2.3);7.904(3.2);7.887(3.5);7.582(0.8);7.577(1.2);7.573(0.7);7.560(3.6);7.542(3.2);7.535(1.6);7.532(2.2);7.521(0.6);7.514(1.5) ;7.496(0.4);5.753(1.4);4.092(0.6);4.076(1.0);4.057(1.0);4.040(0.7);3.315(107.5);2.675(0.7);2.670(0.9);2.666(0.7);2.510(51.6);2.506(102.0);2.501(137.6);2.497(105.6);2.493(54.2);2.337(0.4);2.332(0.7);2.328(0.9);2.324(0.7);1.278(0.4);1.246(1.9);1.213(0.5);1.195(16.0);1.178(15.7);0.875(0.8);0.859(2.5);0.841(1.0);0.146(0.8);0.008(8.6);0.000(185.3);-0.008(8.5);-0.027(0.3);-0.150(0.9) |
| Beispiel 30: ¹H-NMR(400.0 MHz, d₆-DMSO): δ=9.262(8.0);9.064(4.1);9.058(4.5);8.720(1.3);8.706(2.6);8.693(1.3);8.384(4.4);8.378(4.6);8.313(0.4);8.163(16.0);7.904(4.6);7.885(5.3);7.577(1.7);7.560(4.9);7.541(4.5);7.532(3.2);7.521(1.0);7.514(2.1);7.496(0.6);5.753(0.4);4.039(0.5);4.021(0.4);3.338(1.2);3.315(82.7);3.289(3.1);3.271(1.0);2.671(1.0);2.501(147.5);2.328(1.0);1.988(1.6);1.193(0.5);1.174(6.1);1.156(11.4);1.138(5.3);0.146(0.7);0.000(138.7);-0.150(0.7) |
| Beispiel 31: ¹H-NMR(400.0 MHz, d₆-DMSO): δ=9.232(1.1);9.215(6.8);9.056(0.7);9.049(3.6);9.043(3.4);8.500(3.5);8.495(3.4);8.392(0.5);8.386(0.5);8.313(0.3);8.162(16.0);7.908(2.5);7.904(3.4);7.887(3.7);7.885(3.2);7.582(0.9);7.577(1.3);7.572(0.7);7.560(3.8);7.541(3.3);7.535(1.6);7.531(2.3);7.521(0.7);7.514(1.6);7.496(0.4);3.316(89.5);3.042(14.0);2.818(0.6);2.810(0.7);2.801(1.1);2.787(2.7);2.774(0.4);2.675(0.5);2.671(0.7);2.666(0.5);2.511(39.5);2.506(77.8);2.502(105.0);2.497(80.0);2.493(40.7);2.333(0.5);2.328(0.6);2.324(0.5);1.258(0.5);1.247(1.3);0.875(0.6);0.859(1.8);0.841(0.9);0.828(0.3);0.816(0.3);0.789(0.4);0.614(0.4);0.593(1.8);0.585(2.6);0.576(0.9);0.555(1.2);0.543(1.8);0.526(1.6);0. 508(0.4);0.146(0.6);0.008(6.4);0.000(136.3);-0.008(6.0);-0.150(0.6) |
| Beispiel 32: ¹H-NMR(400.0 MHz, d₆-DMSO): δ=9.249(7.9);9.058(3.9);9.052(4.1);8.759(2.4);8.748(2.4);8.378(4.1);8.372(4.2);8.313(0.5);8.162(16.0);7.903(4.0);7.885(4.5);7.577(1.5);7.560(4.5);7.541(4.0);7.532(2.7);7.521(0.8);7.514(1.9);7.496(0.5);4.038(0.4);4.021(0.4);3.315(76.7);2.887(0.6);2.877(0.9);2.868(1.4);2.859(1.4);2.850(1.0);2.841(0.7);2.670(1.1);2.501(165.4);2.497(135.3);2.328(1.1);1.988(1.6);1.193(0.4);1.175(0.8);1.158(0.4);0.768(0.8);0.754(2.6);0.750(3.4);0.737(3.3);0.732(2.8);0.720(1.1);0.585(1.1);0.574(3.4);0.568(3.4);0.559(3.0);0.547(0. 9);0.146(0.9);0.000(179.1);-0.150(0.9) |
| Beispiel 33: 1H-NMR(400,0 MHz, CD3CN): 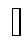 = 8,991(8,4);8,986(8,8);8,536(16,0);8,514(0,5);8,363(0,3);8,340(9,2);8,336(9,3);8,044(9,5);7,974(0,7);7,829(8,4);7,586(0,5);7,084(3,5);2,887(2,5);2,877(2,7);2,867(3,6);2,859(3,7);2,849(2,7);2,841(1,8);2,771(0,9);2,462(2,1);2,264(0,5);2,149(521,5);2,113(2,6);2,107(2,7);2,101(2,1);2,077(0,3);2,057(0,4);2,032(0,4);1,949(175,5);1,944(292,5);1,939(377,7);1,938(371,4);1,933(301,4);1,927(181,4);1,766(2,2);1,761(1,8);1,323(0,4);1,269(7,0);1,221(0,5);1,202(0,6);1,186(0,4);0,881(1,1);0,855(1,1);0,813(2,2);0,796(8,8);0,782(9,1);0,765(2,9);0,726(0,4);0,675(0,3);0,665(0,4);0,634(2,9);0,624(9,0);0,617(9,7);0,596(2,3);0,549(0,5);0,532(0,5);0,515(0,3);0,145(4,9 );0,048(0,7);-0,001(897,2);-0,002(880,8);-0,150(5,0) |
| Beispiel 34: 1H-NMR(400,0 MHz, d6-DMSO): 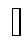 = 9,656(9,9);9,589(0,5);9,316(16,0);9,296(1,0);9,280(0,5);9,087(8,5);9,081(9,0);8,947(0,4);8,943(0,4);8,492(8,8);8,486(8,8);8,337(7,7);8,315(0,4);8,212(0,7);8,059(6,7);4,057(1,2);4,039(3,8);4,021(3,8);4,003(1,3);3,319(115,4);2,676(0,9);2,671(1,2);2,667(1,0);2,507(145,7);2,502(189,4);2,498(146,6);2,334(0,9);2,329(1,2);2,325(1,0);1,989(15,7);1,638(3,3);1,624(8,5);1,617(9,4);1,604(4,0);1,563(0,4);1,398(0,8);1,383(0,4);1,343(4,3);1,329(8,6);1,323(9,1);1,308(3,1);1,270(0,3);1,260(0,4);1,236(2,2);1,193(4,3);1,176(8,4); 1,158(4,1);0,854(0,4);0,000(48,0) |
| Beispiel 35: 1H-NMR(400,0 MHz, CD3CN): 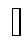 = 8,985(3,4);8,979(3,4);8,530(5,8);8,507(0,5);8,301(3,1);8,295(3,0);8,241(0,5);8,235(0,5);8,045(3,2);7,831(2,8);5,447(3,4);4,068(0,7);4,051(0,7);3,078(16,0);2,818(2,6);2,804(0,4);2,794(0,7);2,786(0,8);2,778(1,3);2,767(0,8);2,760(0,7);2,749(0,3);2,162(82,5);2,160(74,0);1,972(3,9);1,964(2,4);1,958(4,6);1,952(17,3);1,946(30,2);1,940(40,1);1,934(28,7);1,928(15,3);1,437(1,6);1,270(1,5);1,222(0,9);1,204(1,7);1,186(0,8);0,859(0,5);0,841(0,5);0,831(0,4);0,803(0,6);0,588(2,1);0,545(2,4);0,535(1,7);0,528(2,1);0,146(0,4);0,008(6,9);0,000(87,5);-0,150(0,4) |
| Beispiel 36: 1H-NMR(400,0MHz, CD3CN): 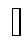 = 9,012(0,3);8,988(8,6);8,982(8,5);8,497(16,0);8,337(9,5);8,331(9,1);7,899(8,3);7,646(8,3);7,08 6(2,7);7,031(3,9);6,851 (8,0);6,671(4,0);2,895(0,6);2,886(1,7);2,877(2,5);2,868(3,5);2,859(3,5);2,850(2,4);2,840(1,6);2,831(0,6);2,464(1,3);2, 334(0,4);2,323(0,4);2,264(0,7);2,246(0,9);2,160(322,3);2,154(396,4);2,119(0,9);2,113(1,3);2,107(1,6);2,101(1,1);1,964(11,0);1,952(115,8);1,946(204,6);1,940(271,6);1,934(192,2);1,927(99,5);1,781(0,5);1,774(1,0);1,768(1,4);1,762(0,9);1,756(0,5);1,270(5,8);1,252(0,6);0,882(0,7);0,864(0,5);0,813(1,9);0,796(8,0);0,783(8,0);0,778(6,4);0,766(2,4);0,744(0,3);0,665(0,4);0,635(2,7);0,623(7,6);0,617(8,1);0,609(6,7);0,596(1,8);0,549(0,4);0,146(3,2);0,000(618,3);-0,150(3,1) |
| Beispiel 37: 1H-NMR(400,0 MHz, CD3CN): 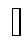 = 8,981 (3,3);8,975(3,2);8,495(5,3);8,470(0,4);8,298(2,8);8,292(2,7);8,236(0,4);8,230(0,4);7,900(2,8);7,647(2,8);7,041(1,4);6,861(2,7);6,681(1,4);4,086(0,6);4,068(1,8);4,051(1,8);4,033(0,6);3,077(16,0);2,817(2,5);2,804(0,3);2,794(0,6);2,787(0,6);2,778(1,1);2,767(0,7);2,760(0,6);2,466(1,0);2,461(0,5);2,182(181,0);2,114(0,3);2,107(0,4);1,972(8,0);1,964(2,2);1,958(5,1);1,952(28,6);1,946(51,7);1,940(69,9);1,934(47,8);1,928(24,3);1,768(0,4);1,437(8,7);1,270(0,9);1,222(2,1);1,204(4,1);1,186(2,0);0,858(0,4);0,841(0,4);0,802(0,5);0,587(1,8);0,554(1,2);0,545(2,0);0,536(1,3);0,529(1,8);0,515(0,4);0,146(0,9);0,007(9,0);0,000(193,1);-0,009(7,7);-0,150(0,9) |
| Beispiel 38: 1H-NMR(400,0 MHz, CD3CN): 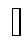 = 9,033(3,8);9,027(3,9);8,523(0,4);8,510(8,2);8,406(4,0);8,400(3,9);7,900(3,0);7,754(1,0);7,646(3,1);7,586(0,4);7,033(2,1);6,853(4,3);6,673(2,1);4,086(0,9);4,068(3,0);4,050(3,1);4,033(1,0);2,468(0,6);2,464(0,8);2,459(0,6);2,454(0,4);2,373(0,7);2,150(189,8);2,145(234,4);2,119(1,7);2,113(1,9);2,107(2,1);2,101(1,5);2,095(0,9);1,972(14,1);1,964(8,2);1,958(18,7);1,952(110,1);1,946(202,1);1,940(275,0);1,933(190,6);1,927(98,2);1,780(0,7);1,774(1,3);1,768(1,7);1,762(1,2);1,756(0,6);1,612(1,7);1,597(4,3);1,591(4,2);1,577(2,3);1,537(0,3);1,437(16,0);1,391(2,4);1,377(4,2);1,370(4,3);1,356(1,7);1,349(0,4);1,271(1,7);1,222(3,7);1,204(7,0);1,186(3,5);0,146(3,4);0,008(34,0);0,000(712,2);-0,008(31,3);-0,150(3,4) |

### Biologische Beispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 5 µg/cm² mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 500g/ha (5 µg/cm²): 1, 2, 5, 6, 8, 9, 12, 13, 15, 17

Bei diesem Test zeigen z.B. die folgenden Verbindungen der Herstellungsbeispiele eine Wirkung von 80% bei einer Aufwandmenge von 500g/ha (5 µg/cm²): 14

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus,* wenn in diesem Test bei einer Aufwandmenge von 5 µg/cm² mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 500g/ha (5 µg/cm²): 1, 2, 3, 5, 8, 13, 14

Bei diesem Test zeigen z.B. die folgenden Verbindungen der Herstellungsbeispiele eine Wirkung von 80% bei einer Aufwandmenge von 500g/ha (5 µg/cm²): 12

Bei diesem Test zeigen z.B. die folgenden Verbindungen der Herstellungsbeispiele eine Wirkung von 100 % bei einer Aufwandmenge von 20 g/ha (0,2 µg/cm²): 9

### Amblvomma hebaraeum -Test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckennymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine der Zecken abgetötet wurde.

Bei diesem Test zeigen z. B. die folgenden Verbindungen der Herstellungsbeispiele eine Wirkung von 100 % bei einer Aufwandmenge von 100 ppm: 2, 3, 5, 8, 9

Bei diesem Test zeigen z. B. die folgenden Verbindungen der Herstellungsbeispiele eine Wirkung von 80 % bei einer Aufwandmenge von 100 ppm: 1

### Boophilus microplus - Diptest

| | |
|---|---|
| Testtiere: | Rinderzecken (*Boophilus microplus*) Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100 % bei einer Aufwandmenge von 100 ppm: 1, 2, 3, 5, 6, 8, 9, 13, 14, 15, 16, 17, 19, 20, 21,22,25,27,33

Bei diesem Test zeigen z.B. die folgenden Verbindungen der Herstellungsbeispiele eine Wirkung von 80% bei einer Aufwandmenge von 100ppm: 12

### Boophilus microplus-Injektionstest

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken (Boophilus microplus) injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100 % bei einer Aufwandmenge von 20 µg/Tier: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35

### Ctenocephalides felis - Oraltest

### Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: 1, 2, 3, 4, 5, 6, 7, 8, 9, 10, 11, 12, 13, 14, 15, 16, 17, 18, 19, 20, 21, 22, 23, 24, 25, 26, 27, 28, 30, 31, 32, 33, 34, 35

### Lucilia cuprina - Test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: 1, 2, 3, 4, 5, 6, 7, 9, 11, 12, 13, 15, 16, 17, 18, 19, 20, 21, 22, 23, 25, 26, 27, 30, 31, 32, 33, 34, 35

Bei diesem Test zeigen z.B. die folgenden Verbindungen der Herstellungsbeispiele eine Wirkung von 95% bei einer Aufwandmenge von 100ppm: 10

Bei diesem Test zeigen z.B. die folgenden Verbindungen der Herstellungsbeispiele eine Wirkung von 90% bei einer Aufwandmenge von 100ppm: 8, 14

### Musca domestica-Test

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100ppm: 1, 2, 3, 4, 5, 6, 9, 11, 12, 13, 14, 15, 16, 17, 18, 21, 22, 26, 27, 33, 34, 35

Bei diesem Test zeigen z.B. die folgenden Verbindungen der Herstellungsbeispiele eine Wirkung von 95% bei einer Aufwandmenge von 100ppm: 8

Bei diesem Test zeigen z.B. die folgenden Verbindungen der Herstellungsbeispiele eine Wirkung von 80% bei einer Aufwandmenge von 100ppm: 19, 25

### Myzus persicae - Sprühtest

| | | |
|---|---|---|
| Lösungsmittel: | 78 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (Myzus *persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 100 g/ha: 1, 2, 12, 13, 15, 16, 35

### Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 100 g/ha: 1, 2, 3, 4, 5, 6, 7, 8, 9, 11, 12, 13, 14, 15, 16, 17, 34, 35

### Spodoptera frugiperda - Sprühtest

| | | |
|---|---|---|
| Lösungsmittel: | 78,0 | Gewichtsteile Aceton |
| | 1,5 | Gewichtsteile Dimethylformamid |
| Emulgator: | | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben *(Zea mays)* werden mit einer Wirkstoffzubereitung der gewünschten Konzentration gespritzt und nach dem Abtrocknen mit Raupen des Heerwurms *(Spodoptera frugiperda)* besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 100 g/ha: 1, 2, 3, 4, 5, 6, 7, 11, 12, 13, 14, 15, 16, 35

### Tetranychus urticae - Sprühtest, OP-resistent

| | | |
|---|---|---|
| Lösungsmittel: | 78,0 | GewichtsteileAceton |
| | 1,5 | Gewichtsteile Dimethylformamid |
| Emulgator : | | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100 % bei einer Aufwandmenge von 100 g/ha: 1, 5, 6, 8, 9, 12, 13, 14, 15, 16, 17, 34, 35

Bei diesem Test zeigen z. B. die folgenden Verbindungen der Herstellungsbeispiele eine Wirkung von 90 % bei einer Aufwandmenge von 100 g/ha: 2

Bei diesem Test zeigen z. B. die folgenden Verbindungen der Herstellungsbeispiele eine Wirkung von 90 % bei einer Aufwandmenge von 20 g/ha: 3, 11

### Anopheles Test (ANPHGB Oberflächenbehandlung)

### Lösungsmittel: Aceton + 2000ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Anopheles gambiae Stamm RSPH (homozygot kdr) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 40mg/m²: 16

### Anopheles Test (ANPHFU Oberflächenbehandlung)

### Lösungsmittel: Aceton + 2000ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Anopheles funestus Stamm FUMOZ-R (Hunt et al., Med Vet Entomol. 2005 Sep; 19(3):271-5) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 40mg/m²: 16

### Aedes Test (AEDSAE Oberflächenbehandlung)

### Lösungsmittel: Aceton + 2000ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Aedes aegypti Stamm MONHEIM auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 40mg/m²: 6, 9, 16

## Patentansprüche

1. Verbindung der Formel (I) 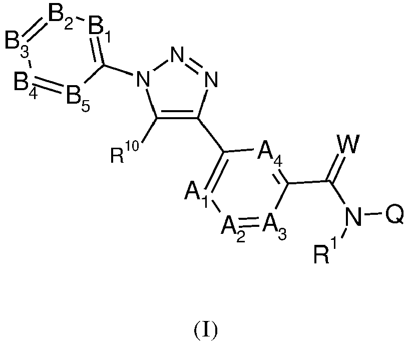 worin
R¹ für Wasserstoff, oder eine gegebenenfalls substituierte Gruppe ausgewählt aus C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl-C₁-C₆-alkyl, Heteroaryl-C₁-C₆-alkyl steht;
die chemischen Gruppierungen
A₁ für CR²
A₂ für Stickstoff,
A₃ für CR³ und
A₄ für CR⁴ stehen,
B₁ für CR⁵ oder N,
B₂ für CR⁶ oder N,
B₃ für CR⁷ oder N,
B₄ für CR⁸ oder N, und
B₅ für CR⁹ oder N stehen,
wobei aber nicht mehr als zwei der Gruppierungen B₁ bis B₅ gleichzeitig für Stickstoff stehen;
R² und R⁴ unabhängig voneinander für Wasserstoff oder C₁-C₄-Alkyl stehen,
R³ für Halogen, C₁-C₄-Alkyl, C₁-C₄-Halogenalkyl oder C₁-C₄-Alkoxy steht,
R⁵, R⁶, R⁸ und R⁹ unabhängig voneinander für Wasserstoff, Halogen, Cyano, Nitro, jeweils gegebenenfalls substituiertes C₁-C₆-Alkyl, C₃-C₇-Cycloalkyl, C₁-C₆-Alkoxy, *N*-(C₁-C,₆-Alkoxy)-imino-C₁-C₃-alkyl, C₁-C₆-Alkylsulfanyl, C₁-C₆-Alkylsulfinyl, C₁-C₆-Alkylsulfonyl, *N*-(C₁-C₆-Alkyl)amino oder *N,N*-Di-(C₁-C₆-alkyl)amino, C₁-C₆-Alkylsulfonyl-amino, *N*-(C₁-C₆-Alkyl)-C₁-C₆-alkylsulfonyl-amino, Phenyl stehen;
R⁷ für Halogen, Cyano, Nitro, jeweils gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, bevorzugt für Halogen, Cyano, Nitro oder C₁-C₆-Halogenalkyl, Phenyl steht;
R¹⁰ unabhängig voneinander für Wasserstoff, Halogen, Cyano, Nitro, Amino oder ein gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl steht;
W für Sauerstoff oder Schwefel steht;
Q für Wasserstoff, Formyl, Hydroxy, Amino oder eine der gegebenenfalls substituierten Gruppierungen C₁-C₆-Alkyl, C₁-C₆-Alkyloxy, C₃-C₆-Alkenyl, C₃-C₆-Alkinyl, C₃-C₇-Cycloalkyl, Hetero-C₁-C₆-cycloalkyl, C₃-C₆-Cycloalkyl-C₁-C₆-alkyl, Aryl-C₁-C₆-alkyl, Heteroaryl-C₁-C₆-alkyl oder für eine Gruppierung *N*-(C₁-C₆-Alkyl)amino, *N*-(C₁-C₆-Alkylcarbonyl)amino, *N,N*-Di(C₁-C₆-alkyl)amino steht; oder
Q für einen gegebenenfalls mehrfach mit V substituierten ungesättigten 6-gliedrigen Carbozyklus steht, oder für einen gegebenenfalls mehrfach mit V substituierten ungesättigten 5- bzw. 6-gliedrigen heterozyklischen Ring steht, wobei
V für Halogen, Cyano, Nitro, oder eine der gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkoxy, *N*-(C₁-C₆-Alkoxy)-imino-C₁-C₆-alkyl, C₁-C₆-Alkylsulfanyl, C₁-C₆-Alkylsulfinyl, C₁-C₆-Alkylsulfonyl, *N,N-*Di(C₁-C₆-alkyl)amino steht;
sowie Salze, N-Oxide und tautomere Formen der Verbindungen der Formel (I),
wobei die genannten substituierten Gruppen mit einem oder mehreren Substituent(en) M¹ , vorzugsweise 1, 2 oder 3 Resten M¹, substituiert sind und der/die Substituenten M¹ jeweils unabhängig voneinander ausgewählt aus der Gruppe bestehend aus Halogen, Hydroxy, Nitro, Formyl, Carboxy, Cyano, Amino, Isocyano, Azido, (C₁-C₄)-Alkyl, (C₁-C₄)-Halogenalkyl, (C₂-C₄)-alkenyl, (C₂-C₄)-alkinyl, (C₃-C₆)-Cycloalkyl (C₁-C₄)-Alkoxy, (C₁-C₄)-Halogenalkoxy, (C₁-C₄)-Alkoxy-(C₁-C₄)-alkoxy, (C₁-C₄)-Alkoxy-(C₁-C₆)-alkyl, *N*-(C₁-C₄)-Alkoxy-imino-(C₁-C₃)-alkyl, (C₁-C₄)-Alkylsulfanyl, (C₁-C₄)-Halogenalkylsulfanyl, (C₁-C₄)-Alkoxycarbonyl (C₁-C₄)-Alkylcarbonyl, Carbamoyl, C₁-C₄-Alkylcarbamoyl, C₃-C₇-Cycloalkylcarbamoyl, Mono- und *N,N*-Di(C₁-C₄)-alkylaminocarbonyl, Amino, (C₁-C₆)-Acylamino, Mono- und *N,N-*Di(C₁-C₄)-alkylamino, Tri(C₁-C₄)-alkylsilyl, (C₃-C₆)-Cycloalkyl, C₆-Aryl, Heterocyclyl mit 3 bis 6 Ringatomen, wobei jeder der letztgenannten cyclischen Gruppen auch über Heteroatome oder eine divalente funktionelle CH₂-, oder C₂H₄-Gruppe gebunden sein kann, (C₁-C₄)-Alkylsulfinyl, wobei beide Enantiomere der (C₁-C₄)-Alkylsulfinylgruppe umfasst sind, (C₁-C₄)-Alkylsulfonyl (C₁-C₄)-Alkylphosphinyl, (C₁-C₄)-Alkylsulfanyl-(C₁-C₄)-alkyl, (C₁-C₄)-Alkoxy-(C₁-C₄)-alkyl Mono- und *N,N*-Di(C₁-C₄)-alkyl-amino(C₁-C₄)-alkyl und Hydroxy(C₁-C₄)-a]kyl ist/sind und die genannten Reste M¹ unsubstituiert oder gegebenenfalls (wie z. B. Alkyl oder Amino), sofern sie kohlenwasserstoffhaltige oder stickstoffwasserstoffhaltige Anteile enthalten, mit einem oder mehreren, vorzugsweise 1, 2 oder 3 Resten M² substituiert sein können, wobei M² unabhängig voneinander ausgewählt ist aus der Gruppe bestehend aus Amino, Hydroxy, Halogen, Nitro, Cyano, Isocyano, Mercapto, Isothiocyanato, Carboxy und Carbonamid.

2. Verbindung der Formel (I) gemäß Anspruch 1, worin
R¹ für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, 2-Propin-1-yl, 2-Propen-1-yl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
die chemischen Gruppierungen
A₁ für CH,
A₂ für Stickstoff,
A₃ für CR³, und
A₄ für CH stehen;
B₁ für CR⁵ oder N,
B₂ für CH,
B₃ für CR⁷,
B₄ für CR⁸, und
B₅ für CR⁹ oder N stehen,
R³ für Fluor, Chlor, Brom, Iod, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy steht;
R⁵, R⁶, R⁸ und R⁹ unabhängig voneinander für Wasserstoff, Halogen, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, N-Methoxyiminomethyl, 1-(N-Methoxyimino)-ethyl, Methylsulfanyl, Trifluormethylsulfanyl, Methylsulfonyl, Methylsulfinyl, Trifluormethylsulfonyl, Trifluormethylsulfinyl, wobei aber R6 und R10 nicht gleichzeitig für Wasserstoff stehen;
R⁷ für Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Nonafluor-sec-butyl, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Trifluormethylsulfonyl, Trifluormethylsulfinyl, Trifluormethylsulfanyl, Phenyl steht;
R¹⁰ unabhängig voneinander für Wasserstoff, Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl steht;
W für Sauerstoff steht; und
Q für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-(Trifluormethyl)ethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N*-Cyclopropylcarbamoyl)-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, 2-Thienylmethyl, 3-Thienylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5- Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N*-Dimethylamino, *N,N*-Diethylamino, 2-oxo-2-(2,2,2-trifluoroethylamino)ethyl steht; oder
Q für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V unabhängig voneinander für Fluor, Chlor, Brom, Iod, Cyano, Nitro, Methyl, Ethyl, iso-Propyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht;
R⁵ für Wasserstoff, Methyl, Ethyl, 2-Methylethyl, tert.-Butyl, Fluor, Chlor, Brom, Iod, Nitro, Trifluormethyl, Difluormethoxy, Trifluormethoxy, Amino steht.

3. Verbindung der Formel (I) gemäß Anspruch 1, worin
W für Sauerstoff steht;
R¹⁰ für Wasserstoff und R¹ für Wasserstoff oder Methyl steht;
B₃ für CR⁷ steht und R⁷ für perhalogeniertes C₁-C₄-Alkyl steht; oder
B₃ für CR⁷ steht und R⁷ für perhalogeniertes C₁-C₄-Alkyl oder Phenyl steht;
Q für gegebenenfalls unabhängig voneinander mit F, Cl, Br, I oder CN substituiertes Cyclopropyl steht.

4. Verbindung der Formel (I) gemäß Anspruch 1 oder 3, worin
W für Sauerstoff steht;
R¹⁰ für Wasserstoff und R**¹** für Wasserstoff oder Methyl steht;
B₃ für CR⁷ steht und R⁷ für -C₃F₇ steht; oder
B₃ für CR⁷ steht und R⁷ für -C₃F₇ oder Phenyl steht; und
Q für Cyclopropyl oder 1 -Cyano-Cyclopropyl steht.

5. Verbindung der Formel (I) gemäß Anspruch 1, 3 oder 4, worin
B₁ für CR⁵ steht, und R⁵ für Halogen, C₁-C₆-Alkoxy oder C₁-C₆-Alkyl mit Halogen, bevorzugt mit F, substituiertes C₁-C₆-Alkoxy oder mit Halogen, bevorzugt mit F, substituiertes C₁-C₆-Alkyl steht;
B₂ für CR⁶ steht, wobei R⁶ für Wasserstoff oder C₁-C₆-Alkyl steht;
B₄ für CR⁸ oder N steht, wobei R⁸ für Wasserstoff oder C₁-C₆-Alkyl steht;
B₅ für CR⁹ oder N steht wobei R⁹ für Halogen, C₁-C₆-Alkoxy oder C₁-C₆-Alkyl steht.

6. Verbindung der Formel (I) gemäß Anspruch 5, worin
B₁ für CR⁵ steht, und R⁵ für Cl, Br, I, C₁-C₄-Alkyl, mit F substituiertes C₁-C₄-Alkyl, oder mit F substituiertes C₁-C₄-Alkoxy steht,
B₂ für CR⁶ steht, wobei R⁶ für Wasserstoff steht,
B₄ für CR⁸ oder N steht, wobei R⁸ für Wasserstoff steht,
B₅ für CR⁹ oder N steht wobei R⁹ für Halogen oder C₁-C₄-Alkyl steht.

7. Verbindung der Formel (I) gemäß einem der Ansprüche 1, 3, 4, 5 oder 6, worin
A₁ für CR² steht wobei R² für Wasserstoffsteht;
A₂ für Stickstoff steht;
A₃ für CR³ steht wobei R³ für Halogen, C₁-C₄-Alkyl, C₁-C₄-Halogenalkyl oder C₁-C₄-Alkoxy steht; und
A₄ für CR⁴ steht wobei R⁴ für Wasserstoff steht.

8. Verbindung der Formel (I) gemäß Anspruch 1, worin
W für Sauerstoff steht,
R¹⁰ für Wasserstoff und R¹ für Wasserstoff oder Methyl steht,
A₁ für CR² steht wobei R² für Wasserstoff oder C₁-C₄-Alkyl steht,
A₂ für Stickstoff steht,
A₃ für CR³ steht wobei R³ für Halogen, C₁-C₄-Alkyl, C₁-C₄-Halogenalkyl oder C₁-C₄-Alkoxy steht,
A₄ für CR⁴ wobei R⁴ für Wasserstoff oder C₁-C₄-Alkyl steht,
B₁ für CR⁵ steht, und R⁵ für Cl, Br, I, C₁-C₄-Alkyl, mit F substituiertes C₁-C₄-Alkyloder mit F substituiertes C₁-C₄-Alkoxy steht,
B₂ für CR⁶ steht, wobei R⁶ für Wasserstoff oder C₁-C₆-Alkyl steht,
B₃ für CR⁷ steht wobei R⁷ für perhalogeniertes C₁-C₄-Alkyl oder Phenyl steht,
B₄ für CR⁸ steht, wobei R⁸ für Wasserstoff oder C₁-C₆-Alkyl steht,
B₅ für CR⁹ steht wobei R⁹ für Halogen, C₁-C₆-Alkoxy oder C₁-C₆-Alkyl steht,
Q für Cyclopropyl oder 1-Cyano-Cyclopropyl, steht.

9. Verbindung der Formel (I) gemäß Anspruch 1, worin
W für Sauerstoff steht,
R¹⁰ für Wasserstoff und R¹ für Wasserstoff oder Methyl steht,
A₁ für CR² steht wobei R² für Wasserstoff steht,
A₂ für Stickstoff steht,
A₃ für CR³ steht wobei R³ für perfluoriertes C₁-C₄-Alkyl (-CF₃, -C₂F₅, -C₃F₇, -C₄F₉), C₁-C₄-Alkyl (-CH₃, -C₂H₅, -C₃H₇, -C₄H₉), Cl, F, I oder C₁-C₄-Alkoxy (-O-CH₃, -O-C₂H₅, -O-C₃H₇, -O-C₄H₉) steht, bevorzugt für C₁-C₄-Alkyl (-CH₃, -C₂H₅, -C₃H₇, -C₄H₉), Cl oder C₁-C₄-Alkoxy (-O-CH₃, -O-C₂H₅, -O-C₃H₇, -O-C₄H₉), mehr bevorzugt für Methyl, Methoxy oder Cl steht,
A₄ für CR⁴ wobei R⁴ für Wasserstoff steht,
B₁ für CR⁵ steht, wobei R⁵ für F, Cl, Br, C₁-C₄-Alkyl, mit F substituiertes C₁-C₄-Alkyl, oder mit F substituiertes C₁-C₄-Alkoxy steht, bevorzugt für -CF₃, -C₂F₅, -C₃F₇, -C₄F₉, -CH3,-C₂H₅, -C₃H₇, -C₄H₉, Cl, OCF₃, OCF₂H oder OCFH₂, mehr bevorzugt für Cl, CF₃, OCF₂H, OCF₃, Methyl oder Ethyl steht,
B₂ für CR⁶ steht, wobei R⁶ für Wasserstoff steht,
B₃ für CR⁷ steht wobei R⁷ für perfluoriertes C₁-C₄-Alkyl oder Phenyl, bevorzugt für -C₃F₇ oder Phenyl steht,
B₄ für CR⁸ steht, wobei R⁸ für Wasserstoff steht,
B₅ für CR⁹ steht wobei R⁹ für F, Cl, Br, I, C₁-C₄-Alkyl steht, bevorzugt für Cl, Br, I oder C₁-C₄-Alkylsteht, besonders bevorzugt für Cl, I, Br oder Methyl oder Ethyl steht,
Q für Cyclopropyl, 1-Cyano-Cyclopropyl, C₁-C₄-Alkyl (-CH₃, -C₂H₅, -C₃H₇, -C₄H₉), oder fluoriertes C₁-C₄-Alkyl (z. B. -CHF₂, CH₂F, -CF₃, -C₂H₄F, -C₂H₃F₂, -C₂H₂F₃, -C₂HF₄,-C₂F₅, -C₃F₇, -C₄F₉) steht, bevorzugt für Methyl, Ethyl, Propyl, C₂H₂F₃, Cyclopropyl oder 1-Cyano-Cyclopropyl, steht.

10. Pharmazeutische Zusammensetzungen, enthaltend wenigstens eine Verbindung gemäß einem der Ansprüche 1 bis 9.

11. Verbindungen gemäß einem der Ansprüche 1 bis 9 zur Verwendung als Arzneimittel.

12. Verbindungen gemäß einem der Ansprüche 1 bis 9 zur Verwendung zur Bekämpfung von Parasiten auf Tieren.

13. Verwendung von Verbindungen gemäß einem der Ansprüche 1 bis 9 zum Schutz des Vermehrungsmaterials von Pflanzen, bevorzugt zum Schutz von Saatgut.

## Claims

1. Compound of the formula (I) 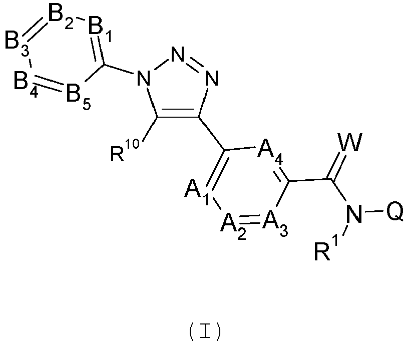 in which
R¹ represents hydrogen or an optionally substituted group selected from the group consisting of C₁-C₆-alkyl, C₃-C₆-alkenyl, C₃-C₆-alkynyl, C₃-C₇-cycloalkyl, C₁-C₆-alkylcarbonyl, C₁-C₆-alkoxy-carbonyl, aryl-C₁-C₆-alkyl, heteroaryl-C₁-C₆-alkyl;
the chemical moieties
A₁ represents CR²,
A₂ represents nitrogen,
A₃ represents CR³ and
A₄ represents CR⁴,
B₁ represents CR⁵ or N,
B₂ represents CR⁶ or N,
B₃ represents CR⁷ or N,
B₄ represents CR⁸ or N, and
B₅ represents CR⁹ or N,
but not more than two of the moieties B₁ to B₅ simultaneously represent nitrogen;
R² and R⁴ independently of one another represent hydrogen or C₁-C₄-alkyl,
R³ represents halogen, C₁-C₄-alkyl, C₁-C₄-haloalkyl or C₁-C₄-alkoxy,
R⁵, R⁶, R⁸ and R⁹ independently of one another represent hydrogen, halogen, cyano, nitro, in each case optionally substituted C₁-C₆-alkyl, C₃-C₇-cyclo-alkyl, C₁-C₆-alkoxy, *N*-(C₁-C₆-alkoxy)imino-C₁-C₃-alkyl, C₁-C₆-alkylsulfanyl, C₁-C₆-alkylsulfinyl, C₁-C₆-alkylsulfonyl, *N*-(C₁-C₆-alkyl)amino or *N,N*-di(C₁-C₆-alkyl)amino, C₁-C₆-alkylsulfonylamino, *N*-(C₁-C₆-alkyl)-C₁-C₆-alkylsulfonylamino, phenyl;
R⁷ represents halogen, cyano, nitro, in each case optionally substituted C₁-C₆-alkyl, C₃-C₆-cyclo-alkyl, C₁-C₆-alkoxy, *N*-C₁-C₆-alkoxyimino-C₁-C₃-alkyl, C₁-C₆-alkylsulfanyl, C₁-C₆-alkylsul finyl, C₁-C₆-alkylsulfonyl, *N*-C₁-C₆-alkylamino or *N*,*N*-di-C₁-C₆-alkylamino, preferably halogen, cyano, nitro or C₁-C₆-haloalkyl, phenyl;
R¹⁰ independently of one another represents hydrogen, halogen, cyano, nitro, amino or an optionally halogen-substituted C₁-C₆-alkyl, C₁-C₆-alkyloxy, C₁-C₆-alkylcarbonyl, C₁-C₆-alkylsulfanyl, C₁-C₆-alkyl-sulfinyl, C₁-C₆-alkylsulfonyl;
W represents oxygen or sulfur;
Q represents hydrogen, formyl, hydroxy, amino or one of the optionally substituted moieties C₁-C₆-alkyl, C₁-C₆-alkyloxy, C₃-C₆-alkenyl, C₃-C₆-alkynyl, C₃-C₇-cycloalkyl, hetero-C₁-C₆-cycloalkyl, C₃-C₆-cycloalkyl-C₁-C₆-alkyl, aryl-C₁-C₆-alkyl, hetero-aryl-C₁-C₆-alkyl or represents a moiety *N*-(C₁-C₆-alkyl)amino, *N*-(C₁-C₆-alkylcarbonyl)amino, *N,N-*di(C₁-C₆-alkyl)amino; or
Q represents an unsaturated 6-membered carbocycle which is optionally polysubstituted by V or represents an unsaturated 5- or 6-membered heterocyclic ring which is optionally polysubstituted by V, where
V represents halogen, cyano, nitro, or one of the optionally substituted moieties C₁-C₆-alkyl, C₂-C₆-alkenyl, C₂-C₆-alkynyl, C₃-C₇-cycloalkyl, C₁-C₆-alkoxy, *N*-(C₁-C₆-alkoxy)imino-C₁-C₆-alkyl, C₁-C₆-alkylsulfanyl, C₁-C₆-alkylsulfinyl, C₁-C₆-alkylsulfonyl, *N,N*-di(C₁-C₆-alkyl)amino;
and salts, N-oxides and tautomeric forms of the compounds of the formula (I),
where the substituted groups mentioned are substituted by one or more substituent(s) M¹, preferably 1, 2 or 3 radicals M¹, and the substituent(s) M¹ each independently is/are selected from the group consisting of halogen, hydroxyl, nitro, formyl, carboxyl, cyano, amino, isocyanato, azido, (C₁-C₄)-alkyl, (C₁-C₄)-haloalkyl, (C₂-C₄)-alkenyl, (C₂-C₄)-alkynyl, (C₃-C₆)-cycloalkyl, (C₁-C₄)-alkoxy, (C₁-C₄)-haloalkoxy, (C₁-C₄)-alkoxy-(C₁-C₄)-alkoxy, (C₁-C₄)-alkoxy-(C₁-C₆)-alkyl, *N*-(C₁-C₄)-alkoxy-imino-(C₁-C₃)-alkyl, (C₁-C₄)-alkylsulfanyl, (C₁-C₄)-haloalkylsulfanyl, (C₁-C₄)-alkoxycarbonyl, (C₁-C₄)-alkylcarbonyl, carbamoyl, C₁-C₄-alkylcarbamoyl, C₃-C₇-cycloalkylcarbamoyl, mono- and *N,N*-di(C₁-C₄)-alkylaminocarbonyl, amino, (C₁-C₆)-acylamino, mono- and *N,N*-di(C₁-C₄)-alkylamino, tri(C₁-C₄)-alkylsilyl, (C₃-C₆)-cycloalkyl, C₆-aryl, heterocyclyl having 3 to 6 ring atoms, where each of the last-mentioned cyclic groups may also be attached via heteroatoms or a divalent functional CH₂ or C₂H₄ group, (C₁-C₄)-alkylsulfinyl, comprising both enantiomers of the (C₁-C₄)-alkylsulfinyl group, (C₁-C₄)-alkylsulfonyl, (C₁-C₄)-alkylphosphinyl, (C₁-C₄) -alkylsulfanyl- (C₁-C₄)-alkyl, (C₁-C₄)-alkoxy-(C₁-C₄)-alkyl, mono- and *N,N*-di(C₁-C₄)-alkyl-amino-(C₁-C₄)-alkyl and hydroxy-(C₁-C₄)-alkyl and the radicals M¹ mentioned are unsubstituted or may optionally (such as, for example, alkyl or amino), if they contain hydrocarbon-containing or hydronitrogen-containing portions, be substituted by one or more, preferably 1, 2 or 3, radicals M², where M² independently of one another is selected from the group consisting of amino, hydroxyl, halogen, nitro, cyano, isocyanato, mercapto, isothiocyanato, carboxyl and carboxamide.

2. Compound of the formula (I) according to Claim 1 in which
R¹ represents hydrogen, methyl, ethyl, n-propyl, isopropyl, n-butyl, isobutyl, s-butyl, t-butyl, methoxymethyl, ethoxymethyl, propoxymethyl, 2-propyn-1-yl, 2-propen-1-yl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxy-carbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 4-chloropyrid-3-ylmethyl;
the chemical moieties
A₁ represents CH,
A₂ represents nitrogen,
A₃ represents CR³, and
A₄ represents CH;
B₁ represents CR⁵ or N,
B₂ represents CH,
B₃ represents CR⁷,
B₄ represents CR⁸, and
B₅ represents CR⁹ or N,
R³ represents fluorine, chlorine, bromine, iodine, methyl, ethyl, fluoromethyl, difluoromethyl, chlorodifluoromethyl, trifluoromethyl, 2,2,2-trifluoroethyl, methoxy, ethoxy, n-propoxy, 1-methylethoxy;
R⁵, R⁶, R⁸ and R⁹ independently of one another represent hydrogen, halogen, cyano, nitro, methyl, ethyl, fluoromethyl, difluoromethyl, chlorodifluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichloro-fluoromethoxy, trifluoromethoxy, 2,2,2-trifluoroethoxy, 2-chloro-2,2-difluoroethoxy, pentafluoroethoxy, N-methoxyiminomethyl, 1-(N-methoxyimino)ethyl, methylsulfanyl, trifluoro-methylsulfanyl, methylsulfonyl, methylsulfinyl, trifluoromethylsulfonyl, trifluoromethylsulfinyl, but where R⁶ and R¹⁰ do not simultaneously represent hydrogen;
R⁷ represents difluoromethyl, trichloromethyl, chlorodifluoromethyl, dichlorofluoromethyl, trifluoromethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoro-ethyl, 2,2,2-trichloroethyl, 2-chloro-2,2-difluoroethyl, 1,1-difluoroethyl, pentafluoroethyl, pentafluoro-tert-butyl, heptafluoro-n-propyl, heptafluoroisopropyl, nonafluoro-n-butyl, nonafluoro-sec-butyl, fluoromethoxy, difluoro-methoxy, chlorodifluoromethoxy, dichlorofluoro-methoxy, trifluoromethoxy, 2,2,2-trifluoroethoxy, 2-chloro-2,2-difluoroethoxy, pentafluoroethoxy, trifluoromethylsulfonyl, trifluoromethylsulfinyl trifluoromethylsulfanyl, phenyl;
R¹⁰ independently of one another represents hydrogen, halogen, cyano, nitro, amino, methyl, ethyl, 1-methylethyl, tert-butyl, trifluoromethyl, difluoromethyl, methoxy, ethoxy, trifluoromethoxy, 2,2-difluoroethoxy, 2,2,2-trifluoroethoxy, methylcarbonyl, ethylcarbonyl, trifluoromethylcarbonyl, methylsulfanyl, methylsulfinyl, methylsulfonyl, trifluoromethylsulfonyl, trifluoromethylsulfanyl, trifluoromethylsulfinyl;
W represents oxygen; and
Q represents hydrogen, methyl, ethyl, n-propyl, 1-methylethyl, 1,1-dimethylethyl, 1-methylpropyl, n-butyl, 2-methylpropyl, 2-methylbutyl, hydroxy-methyl, 2-hydroxypropyl, cyanomethyl, 2-cyanoethyl, 2-fluoroethyl, 2,2-difluoroethyl, 2,2,2-trifluoro-ethyl, 1-trifluoromethylethyl, 2,2-difluoropropyl, 3,3,3-trifluoropropyl, 2,2-dimethyl-3-fluoro-propyl, cyclopropyl, 1-cyanocyclopropyl, 1-methoxycarbonylcyclopropyl, 1-(*N*-methylcarbamoyl)-cyclopropyl, 1-(*N*-cyclopropylcarbamoyl)cyclo-propyl, cyclopropylmethyl, cyclobutyl, cyclopentyl, cyclohexyl, 1-cyclopropylethyl, bis(cyclopropyl)-methyl, 2,2-dimethylcyclopropylmethyl, 2-phenyl-cyclopropyl, 2,2-dichlorocyclopropyl, trans-2-chlorocyclopropyl, cis-2-chlorocyclopropyl, 2,2-difluorocyclopropyl, trans-2-fluorocyclopropyl, cis-2-fluorocyclopropyl, trans-4-hydroxycyclo-hexyl, 4-trifluoromethylcyclohexyl, prop-2-enyl, 2-methylprop-2-enyl, prop-2-ynyl, 1,1-dimethylbut-2-ynyl, 3-chloroprop-2-enyl, 3,3-dichloroprop-2-enyl, 3,3-dichloro-1,1-dimethylprop-2-enyl, phenyl, 2-chlorophenyl, 3-chlorophenyl, 4-chlorophenyl, oxetan-3-yl, thietan-3-yl, 1-oxidothietan-3-yl, 1,1-dioxidothietan-3-yl, isoxazol-3-ylmethyl, 1,2,4-triazol-3-ylmethyl, 3-methyloxetan-3-ylmethyl, 2-thienylmethyl, 3-thienylmethyl, benzyl, 2,6-difluorophenylmethyl, 3-fluorophenylmethyl, 2-fluorophenylmethyl, 2,5-difluorophenylmethyl, 1-phenylethyl, 4-chlorophenylethyl, 2-trifluoro-methylphenylethyl, 1-pyridin-2-ylethyl, pyridin-2-ylmethyl, 5-fluoropyridin-2-ylmethyl, (6-chloro-pyridin-3-yl)methyl, pyrimidin-2-ylmethyl, methoxy, 2-ethoxyethyl, 2-(methylsulfanyl)ethyl, 1-methyl-2-(ethylsulfanyl)ethyl, 2-methyl-1-(methylsulfanyl)-propan-2-yl, methoxycarbonyl, methoxycarbonyl-methyl, NH₂, *N*-ethylamino, *N*-allylamino, *N,N-*dimethylamino, *N,N*-diethylamino, 2-oxo-2-(2,2,2-trifluoroethylamino)ethyl; or
Q represents a phenyl, naphthyl, pyridazine, pyrazine, pyrimidine, triazine, pyridine, pyrazole, thiazole, isothiazole, oxazole, isoxazole, triazole, imidazole, furan, thiophene, pyrrole, oxadiazole, thiadiazole substituted by 0 - 4 substituents V, where
V independently of one another represents fluorine, chlorine, bromine, iodine, cyano, nitro, methyl, ethyl, isopropyl, difluoromethyl, trichloromethyl, chlorodifluoromethyl, dichlorofluoromethyl, trifluoromethyl, chloromethyl, bromomethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloro-ethyl, 2-chloro-2,2-difluoroethyl, 1,1-difluoro-ethyl, pentafluoroethyl, pentafluoro-tert-butyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoro-methoxy, dichlorofluoromethoxy, trifluoromethoxy, 2,2,2-trifluoroethoxy, 2-chloro-2,2-difluoro-ethoxy, pentafluoroethoxy, *N*-methoxyiminomethyl, 1-(*N*-methoxyimino)ethyl, methylsulfanyl, methylsulfonyl, methylsulfinyl, trifluoromethylsulfonyl, trifluoromethylsulfinyl, trifluoromethylsulfanyl, *N,N*-dimethylamino;
R⁵ represents hydrogen, methyl, ethyl, 2-methylethyl, tert-butyl, fluorine, chlorine, bromine, iodine, nitro, trifluoromethyl, difluoromethoxy, trifluoromethoxy, amino.

3. Compound of the formula (I) according to Claim 1 in which
W represents oxygen;
R¹⁰ represents hydrogen and R¹ represents hydrogen or methyl;
B₃ represents CR⁷ and R⁷ represents perhalogenated C₁-C₄-alkyl; or
B₃ represents CR⁷ and R⁷ represents perhalogenated C₁-C₄-alkyl or phenyl;
Q represents cyclopropyl optionally substituted independently of one another by F, Cl, Br, I or CN.

4. Compound of the formula (I) according to Claim 1 or 3 in which
W represents oxygen;
R¹⁰ represents hydrogen and R¹ represents hydrogen or methyl;
B₃ represents CR⁷ and R⁷ represents -C₃F₇; or
B₃ represents CR⁷ and R⁷ represents -C₃F₇ or phenyl; and
Q represents cyclopropyl or 1-cyanocyclopropyl.

5. Compound of the formula (I) according to Claim 1, 3 or 4 in which
B₁ represents CR⁵, and R⁵ represents halogen, C₁-C₆-alkoxy or C₁-C₆-alkyl, halogen-, preferably F-, substituted C₁-C₆-alkoxy or halogen-, preferably F-, substituted C₁-C₆-alkyl;
B₂ represents CR⁶, where R⁶ represents hydrogen or C₁-C₆-alkyl;
B₄ represents CR⁸ or N, where R⁸ represents hydrogen or C₁-C₆-alkyl;
B₅ represents CR⁹ or N, where R⁹ represents halogen, C₁-C₆-alkoxy or C₁-C₆-alkyl.

6. Compound of the formula (I) according to Claim 5 in which
B₁ represents CR⁵, and R⁵ represents Cl, Br, I, C₁-C₄-alkyl, F-substituted C₁-C₄-alkyl or F-substituted C₁-C₄-alkoxy,
B₂ represents CR⁶, where R⁶ represents hydrogen,
B₄ represents CR⁸ or N, where R⁸ represents hydrogen,
B₅ represents CR⁹ or N, where R⁹ represents halogen or C₁-C₄-alkyl.

7. Compound of the formula (I) according to any of Claims 1, 3, 4, 5 and 6 in which
A₁ represents CR², where R² represents hydrogen;
A₂ represents nitrogen;
A₃ represents CR³, where R³ represents halogen, C₁-C₄-alkyl, C₁-C₄-haloalkyl or C₁-C₄-alkoxy; and
A₄ represents CR⁴, where R⁴ represents hydrogen.

8. Compound of the formula (I) according to Claim 1 in which
W represents oxygen,
R¹⁰ represents hydrogen and R¹ represents hydrogen or methyl,
A₁ represents CR², where R² represents hydrogen or C₁-C₄-alkyl,
A₂ represents nitrogen,
A₃ represents CR³, where R³ represents halogen, C₁-C₄-alkyl, C₁-C₄-haloalkyl or C₁-C₄-alkoxy,
A₄ represents CR⁴, where R⁴ represents hydrogen or C₁-C₄-alkyl,
B₁ represents CR⁵, and R⁵ represents Cl, Br, I, C₁-C₄-alkyl, F-substituted C₁-C₄-alkyl or F-substituted C₁-C₄-alkoxy,
B₂ represents CR⁶, where R⁶ represents hydrogen or C₁-C₆-alkyl,
B₃ represents CR⁷, where R⁷ represents perhalogenated C₁-C₄-alkyl or phenyl,
B₄ represents CR⁸, where R⁸ represents hydrogen or C₁-C₆-alkyl,
B₅ represents CR⁹, where R⁹ represents halogen, C₁-C₆-alkoxy or C₁-C₆-alkyl,
Q represents cyclopropyl or 1-cyanocyclopropyl.

9. Compound of the formula (I) according to Claim 1 in which
W represents oxygen,
R¹⁰ represents hydrogen and R¹ represents hydrogen or methyl,
A₁ represents CR², where R² represents hydrogen,
A₂ represents nitrogen,
A₃ represents CR³, where R³ represents perfluorinated C₁-C₄-alkyl (-CF₃, -C₂F₅, -C₃F₇, -C₄F₉), C₁-C₄-alkyl (-CH₃, -C₂H₅, -C₃H₇, -C₄H₉), Cl, F, I or C₁-C₄-alkoxy (-O-CH₃, -O-C₂H₅, -O-C₃H₇, -O-C₄H₉), preferably C₁-C₄-alkyl (-CH₃, -C₂H₅, -C₃H₇, -C₄H₉), Cl or C₁-C₄-alkoxy (-O-CH₃, -O-C₂H₅, -O-C₃H₇, -O-C₄H₉), more preferably methyl, methoxy or Cl,
A₄ represents CR⁴, where R⁴ represents hydrogen,
B₁ represents CR⁵, where R⁵ represents F, Cl, Br, C₁-C₄-alkyl, F-substituted C₁-C₄-alkyl or F-substituted C₁-C₄-alkoxy, preferably -CF₃, -C₂F₅, -C₃F₇, -C₄F₉, -CH₃, -C₂H₅, -C₃H₇, -C₄H₉, Cl, OCF₃, OCF₂H, or OCFH₂, more preferably Cl, CF₃, OCF₂H, OCF₃, methyl or ethyl,
B₂ represents CR⁶, where R⁶ represents hydrogen,
B₃ represents CR⁷, where R⁷ represents perfluorinated C₁-C₄-alkyl or phenyl, preferably -C₃F₇ or phenyl,
B₄ represents CR⁸, where R⁸ represents hydrogen,
B₅ represents CR⁹, where R⁹ represents F, Cl, Br, I, C₁-C₄-alkyl, preferably Cl, Br, I or C₁-C₄-alkyl, particularly preferably Cl, I, Br or methyl or ethyl,
Q represents cyclopropyl, 1-cyanocyclopropyl, C₁-C₄-alkyl (-CH₃, -C₂H₅, -C₃H₇, -C₄H₉) or fluorinated C₁-C₄-alkyl (e.g. -CHF₂, CH₂F, -CF₃, -C₂H₄F, -C₂H₃F₂, -C₂H₂F₃, -C₂HF₄, -C₂F₅, -C₃F₇, -C₄F₉), preferably methyl, ethyl, propyl, C₂H₂F₃, cyclopropyl or 1-cyanocyclopropyl.

10. Pharmaceutical compositions comprising at least one compound according to any of Claims 1 to 9.

11. Compounds according to any of Claims 1 to 9 for use as medicaments.

12. Compounds according to any of Claims 1 to 9 for use for controlling parasites on animals.

13. Use of compounds according to any of Claims 1 to 9 for protecting the propagation material of plants, preferably for protecting seed.

## Revendications

1. Composé de la formule (I) : 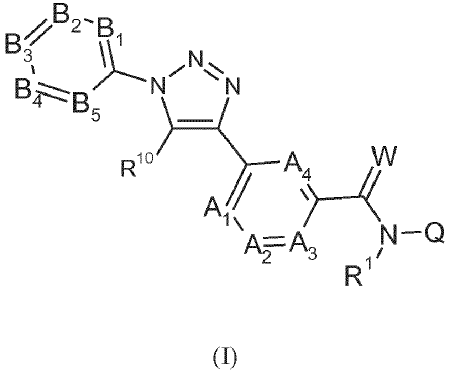 dans lequel
R¹ représente hydrogène, ou un groupe éventuellement substitué choisi parmi alkyle en C₁-C₆, alcényle en C₃-C₆, alcynyle en C₃-C₆, cycloalkyle en C₃-C₇, alkylcarbonyle en C₁-C₆, alcoxycarbonyle en C₁-C₆, aryl-alkyle en C₁-C₆, hétéroaryl-alkyle en C₁-C₆ ;
les groupements chimiques ont les significations suivantes :
A₁ représente CR²,
A₂ représente azote,
A₃ représente CR³ et
A₄ représente CR⁴,
B₁ représente CR⁵ ou N,
B₂ représente CR⁶ ou N,
B₃ représente CR⁷ ou N,
B₄ représente CR⁸ ou N, et
B₅ représente CR⁹ ou N,
pas plus de deux des groupements B₁ à B₅ ne représentant toutefois simultanément azote ;
R² et R⁴ représentent indépendamment l'un de l'autre hydrogène ou alkyle en C₁-C₄,
R³ représente halogène, alkyle en C₁-C₄, halogénoalkyle en C₁-C₄ ou alcoxy en C₁-C₄,
R⁵, R⁶, R⁸ et R⁹ représentent indépendamment les uns des autres hydrogène, halogène, cyano, nitro ; alkyle en C₁-C₆, cycloalkyle en C₃-C₇, alcoxy en C₁-C₆, *N-*(alcoxy C₁-C₆)-imino-alkyle en C₁-C₃, alkylsulfanyle en C₁-C₆, alkylsulfinyle en C₁-C₆, alkylsulfonyle en C₁-C₆, *N*-(alkyle en C₁-C₆)amino ou *N,N*-di(alkyle en C₁-C₆)amino, alkylsulfonyl-amino en C₁-C₆, *N*-(alkyle en C₁-C₆)-alkylsulfonyl-amino en C₁-C₆, phényle, chacun éventuellement substitué ;
R⁷ représente halogène, cyano, nitro ; alkyle en C₁-C₆, cycloalkyle en C₃-C₆, alcoxy en C₁-C₆, *N*-alcoxy-imino en C₁-C₆-alkyle en C₁-C₃, alkylsulfanyle en C₁-C₆, alkylsulfinyle en C₁-C₆, alkylsulfonyle en C₁-C₆, *N*-alkylamino en C₁-C₆ ou *N,N*-di-alkylamino en C₁-C₆, chacun éventuellement substitué, de préférence halogène, cyano, nitro ou halogénoalkyle en C₁-C₆, phényle ;
R¹⁰ représentent indépendamment les uns des autres hydrogène, halogène, cyano, nitro, amino ou un alkyle en C₁-C₆, alkyloxy en C₁-C₆, alkylcarbonyle en C₁-C₆, alkylsulfanyle en C₁-C₆, alkylsulfinyle en C₁-C₆, alkylsulfonyle en C₁-C₆ éventuellement substitué avec halogène ;
W représente oxygène ou soufre ;
Q représente hydrogène, formyle, hydroxy, amino ou un des groupements éventuellement substitués alkyle en C₁-C₆, alkyloxy en C₁-C₆, alcényle en C₃-C₆, alcynyle en C₃-C₆, cycloalkyle en C₃-C₇, hétérocycloalkyle en C₁-C₆, cycloalkyle en C₃-C₆-alkyle en C₁-C₆, aryl-alkyle en C₁-C₆, hétéroaryl-alkyle en C₁-C₆, ou un groupement *N-*(alkyle en C₁-C₆)amino, *N-*(alkylcarbonyle en C₁-C₆)amino, *N,N*-di(alkyle en C₁-C₆)amino ; ou
Q représente un carbocycle insaturé à 6 chaînons éventuellement substitué plusieurs fois avec V, ou un cycle hétérocyclique insaturé à 5 ou 6 chaînons éventuellement substitué plusieurs fois avec V,
V représentant halogène, cyano, nitro ou un des groupements éventuellement substitués alkyle en C₁-C₆, alcényle en C₂-C₆, alcynyle en C₂-C₆, cycloalkyle en C₃-C₇, alcoxy en C₁-C₆, *N*-(alcoxy en C₁-C₆)-imino-alkyle en C₁-C₆, alkylsulfanyle en C₁-C₆, alkylsulfinyle en C₁-C₆, alkylsulfonyle en C₁-C₆, *N,N*-di(alkyle en C₁-C₆)amino ;
ainsi que sels, N-oxydes et formes tautomères des composés de la formule (I),
les groupes substitués mentionnés étant substitués avec un ou plusieurs substituant(s) M¹, de préférence 1, 2 ou 3 radicaux M¹, et le ou les substituants M¹ étant chacun choisis indépendamment les uns des autres dans le groupe constitué par halogène, hydroxy, nitro, formyle, carboxy, cyano, amino, isocyano, azido, alkyle en (C₁-C₄), halogénoalkyle en (C₁-C₄), alcényle en (C₂-C₄), alcynyle en (C₂-C₄), cycloalkyle en (C₃-C₆), alcoxy en (C₁-C₄), halogénoalcoxy en (C₁-C₄), alcoxy en (C₁-C₄)-alcoxy en (C₁-C₄), alcoxy en (C₁-C₄)-alkyle en (C₁-C₆), *N*-alcoxy en (C₁-C₄)-imino-alkyle en (C₁-C₃), alkylsulfanyle en (C₁-C₄), halogénoalkylsulfanyle en (C₁-C₄), alcoxycarbonyle en (C₁-C₄), alkylcarbonyle en (C₁-C₄), carbamoyle, alkylcarbamoyle en C₁-C₄, cycloalkylcarbamoyle en C₃-C₇, mono- et *N,N-*di-alkylaminocarbonyle en (C₁-C₄), amino, acylamino en (C₁-C₆), mono- et *N,N*-di-alkylamino en (C₁-C₄), tri-alkylsilyle en (C₁-C₄), cycloalkyle en (C₃-C₆), aryle en C₆, hétérocyclyle comportant 3 à 6 atomes de cycle, chacun des derniers groupes cycliques mentionnés pouvant également être relié par des hétéroatomes ou un groupe CH₂ ou C₂H₄ fonctionnel divalent, alkylsulfinyle en (C₁-C₄), les deux énantiomères du groupe alkylsulfinyle en (C₁-C₄) étant compris, alkylsulfonyle en (C₁-C₄), alkylphosphinyle en (C₁-C₄), alkylsulfanyle en (C₁-C₄)-alkyle en (C₁-C₄), alcoxy en (C₁-C₄)-alkyle en (C₁-C₄), mono- et *N,N*-di-alkylamino en (C₁-C₄)-alkyle en (C₁-C₄) et hydroxy-alkyle en (C₁-C₄), et les radicaux M¹ mentionnés pouvant être non subtitués ou éventuellement (tels que p. ex. alkyle ou amino), dans la mesure où ils contiennent des fractions hydrocarbonées ou hydroazotées, substitués avec un ou plusieurs, de préférence 1, 2 ou 3 radicaux M², les M² étant choisis indépendamment les uns des autres dans le groupe constitué par amino, hydroxy, halogène, nitro, cyano, isocyano, mercapto, isothiocyanato, carboxy et carbonamide.

2. Composé de la formule (I) selon la revendication 1, dans laquelle
R¹ représente hydrogène, méthyle, éthyle, n-propyle, isopropyle, n-butyle, i-butyle, s-butyle, t-butyle, méthoxyméthyle, éthoxyméthyle, propoxyméthyle, 2-propyn-1-yle, 2-propén-1-yle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, cyanométhyle, 2-cyanoéthyle, benzyle, 4-méthoxybenzyle, pyridin-2-yl-méthyle, pyridin-3-yl-méthyle, pyridin-4-yl-méthyle, 4-chloro-pyridin-3-yl-méthyle ;
les groupements chimiques ont les significations suivantes :
A₁ représente CH,
A₂ représente azote,
A₃ représente CR³ et
A₄ représente CH ;
B₁ représente CR⁵ ou N,
B₂ représente CH,
B₃ représente CR⁷,
B₄ représente CR⁸, et
B₅ représente CR⁹ ou N,
R³ représente fluor, chlore, brome, iode, méthyle, éthyle, fluorométhyle, difluorométhyle, chlorodifluorométhyle, trifluorométhyle, 2,2,2-trifluoroéthyle, méthoxy, éthoxy, n-propoxy, 1-méthyléthoxy ;
R⁵, R⁶, R⁸ et R⁹ représentent indépendamment les uns des autres hydrogène, halogène, cyano, nitro, méthyle, éthyle, fluorométhyle, difluorométhyle, chlorodifluorométhyle, trifluorométhyle, 2,2,2-trifluoroéthyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N-*méthoxyiminométhyle, 1-(*N*-méthoxyimino)-éthyle, méthylsulfanyle, trifluorométhylsulfanyle, méthylsulfonyle, méthylsulfinyle, trifluorométhylsulfonyle, trifluorométhylsulfinyle, R6 et R10 ne représentant toutefois pas simultanément hydrogène ;
R⁷ représente difluorométhyle, trichlorométhyle, chlorodifluorométhyle, dichlorofluorométhyle, trifluorométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-tert-butyle, heptafluoro-n-propyle, heptafluoro-isopropyle, nonafluoro-n-butyle, nonafluoro-sec-butyle, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, trifluorométhylsulfonyle, trifluorométhylsulfinyle, trifluorométhylsulfanyle, phényle ;
R¹⁰ représentent indépendamment les uns des autres hydrogène, halogène, cyano, nitro, amino, méthyle, éthyle, 1-méthyléthyle, tert-butyle, trifluorométhyle, difluorométhyle, méthoxy, éthoxy, trifluorométhoxy, 2,2-difluoroéthoxy, 2,2,2-trifluoroéthoxy, méthylcarbonyle, éthylcarbonyle, trifluorométhylcarbonyle, méthylsulfanyle, méthylsulfinyle, méthylsulfonyle, trifluorométhylsulfonyle, trifluorométhylsulfanyle, trifluorométhylsulfinyle ;
W représente oxygène ; et
Q représente hydrogène, méthyle, éthyle, n-propyle, 1-méthyléthyle, 1,1-diméthyléthyle, 1-méthylpropyle, n-butyle, 2-méthylpropyle, 2-méthylbutyle, hydroxyméthyle, 2-hydroxypropyle, cyanométhyle, 2-cyanoéthyle, 2-fluoroéthyle, 2,2-difluoroéthyle, 2,2,2-trifluoroéthyle, 1-(trifluorométhyl)éthyle, 2,2-difluoropropyle, 3,3,3-trifluropropyle, 2,2-diméthyl-3-fluoropropyle, cyclopropyle, 1-cyano-cyclopropyle, 1-méthoxycarbonyl-cyclopropyle, 1-(*N-*méthylcarbamoyl)cyclopropyle, 1-(*N-*cyclopropylcarbamoyl)-cyclopropyle, cyclopropyl-méthyle, cyclobutyle, cyclopentyle, cyclohexyle, 1-cyclopropyléthyle, bis(cyclopropyl)méthyle, 2,2-diméthylcyclopropyl-méthyle, 2-phénylcyclopropyle, 2,2-dichlorocyclopropyle, trans-2-chlorocyclopropyle, cis-2-chlorocyclopropyle, 2,2-difluorocyclopropyle, trans-2-fluorocyclopropyle, cis-2-fluorocyclopropyle, trans-4-hydroxycyclohexyle, 4-trifluorométhylcyclohexyle, prop-2-ényle, 2-méthylprop-2-ényle, prop-2-inyle, 1,1-diméthylbut-2-inyle, 3-chloro-prop-2-ényle, 3,3-dichloro-prop-2-ényle, 3,3-dichloro-1,1-diméthylprop-2-ényle, phényle, 2-chlorophényle, 3-chlorophényle, 4-chlorophényle, oxétan-3-yle, thiétan-3-yle, 1-oxido-thiétan-3-yle, 1,1-dioxydo-thiétan-3-yle, isoxazol-3-ylméthyle, 1,2,4-triazol-3-ylméthyle, 3-méthyloxétan-3-ylméthyle, 2-thiénylméthyle, 3-thiénylméthyle, benzyle, 2,6-difluorophénylméthyle, 3-fluorophénylméthyle, 2-fluorophénylméthyle, 2,5-difluorophénylméthyle, 1-phényléthyle, 4-chlorophényléthyle, 2-trifluorométhylphényléthyle, 1-pyridin-2-yléthyle, pyridin-2-ylméthyle, 5-fluoropyridin-2-ylméthyle, (6-chloro-pyridin-3-yl)méthyle, pyrimidin-2-ylméthyle, méthoxy, 2-éthoxyéthyle, 2-(méthylsulfanyl)éthyle, 1-méthyl-2-(éthylsulfanyl)éthyle, 2-méthyl-1-(méthylsulfanyl)propan-2-yle, méthoxycarbonyle, méthoxycarbonylméthyle, NH₂, N-éthylamino, *N-*allylamino, *N,N*-diméthylamino, *N,N*-diéthylamino, 2-oxo-2-(2,2,2-trifluoroéthylamino)éthyle ; ou
Q représente un phényle, un naphtyle, une pyridazine, une pyrazine, une pyrimidine, une triazine, une pyridine, un pyrazole, un thiazole, un isothiazole, un oxazole, un isoxazole, un triazole, un imidazole, un furanne, un thiophène, un pyrrole, un oxadiazole, un thiadiazole substitué avec 0 à 4 substituants V,
V représentant indépendamment les uns des autres fluor, chlore, brome, iode, cyano, nitro, méthyle, éthyle, isopropyle, difluorométhyle, trichlorométhyle, chlorodifluorométhyle, dichlorofluorométhyle, trifluorométhyle, chlorométhyle, bromométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-tert-butyle, heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N-*méthoxyiminométhyle, 1-(*N*-méthoxyimino)-éthyle, méthylsulfanyle, méthylsulfonyle, méthylsulfinyle, trifluorométhylsulfonyle, trifluorométhylsulfinyle, trifluorométhylsulfanyle, *N,N*-diméthylamino ;
R⁵ représente hydrogène, méthyle, éthyle, 2-méthyléthyle, tert.-butyle, fluor, chlore, brome, iode, nitro, trifluorométhyle, difluorométhoxy, trifluorométhoxy, amino.

3. Composé de la formule (I) selon la revendication 1, dans laquelle
W représente oxygène ;
R¹⁰ représente hydrogène et R¹ représente hydrogène ou méthyle ;
B₃ représente CR⁷ et R⁷ représente alkyle en C₁-C₄ perhalogéné ; ou
B₃ représente CR⁷ et R⁷ représente alkyle en C₁-C₄ perhalogéné ou phényle ;
Q représente cyclopropyle éventuellement substitué indépendamment les uns des autres avec F, Cl, Br, I ou CN.

4. Composé de la formule (I) selon la revendication 1 ou 3, dans laquelle
W représente oxygène ;
R¹⁰ représente hydrogène et R¹ représente hydrogène ou méthyle ;
B₃ représente CR⁷ et R⁷ représente -C₃F₇ ; ou
B₃ représente CR⁷ et R⁷ représente -C₃F₇ ou phényle ; et
Q représente cyclopropyle ou 1-cyano-cyclopropyle.

5. Composé de la formule (I) selon la revendication 1, 3 ou 4, dans laquelle
B₁ représente CR⁵, et R⁵ représente halogène, alcoxy en C₁-C₆ ou alkyle en C₁-C₆, alcoxy en C₁-C₆ substitué avec halogène, de préférence avec F, ou alkyle en C₁-C₆ substitué avec halogène, de préférence avec F ;
B₂ représente CR⁶, R⁶ représentant hydrogène ou alkyle en C₁-C₆ ;
B₄ représente CR⁸ ou N, R⁸ représentant hydrogène ou alkyle en C₁-C₆ ;
B₅ représente CR⁹ ou N, R⁹ représentant halogène, alcoxy en C₁-C₆ ou alkyle en C₁-C₆.

6. Composé de la formule (I) selon la revendication 5, dans laquelle
B₁ représente CR⁵, et R⁵ représente Cl, Br, I, alkyle en C₁-C₄, alkyle en C₁-C₄ substitué avec F, ou alcoxy en C₁-C₄ substitué avec F,
B₂ représente CR⁶, R⁶ représentant hydrogène,
B₄ représente CR⁸ ou N, R⁸ représentant hydrogène,
B₅ représente CR⁹ ou N, R⁹ représentant halogène ou alkyle en C₁-C₄.

7. Composé de la formule (I) selon l'une quelconque des revendications 1, 3, 4, 5 et 6, dans laquelle
A₁ représente CR², R² représentant hydrogène ;
A₂ représente azote ;
A₃ représente CR³, R³ représentant halogène, alkyle en C₁-C₄, halogénoalkyle en C₁-C₄ ou alcoxy en C₁-C₄ ; et
A₄ représente CR⁴, R⁴ représentant hydrogène.

8. Composé de la formule (I) selon la revendication 1, dans laquelle
W représente oxygène,
R¹⁰ représente hydrogène et R¹ représente hydrogène ou méthyle,
A₁ représente CR², R² représentant hydrogène ou alkyle en C₁-C₄,
A₂ représente azote,
A₃ représente CR³, R³ représentant halogène, alkyle en C₁-C₄, halogénoalkyle en C₁-C₄ ou alcoxy en C₁-C₄,
A₄ représente CR⁴, R⁴ représentant hydrogène ou alkyle en C₁-C₄,
B₁ représente CR⁵, et R⁵ représente Cl, Br, I, alkyle en C₁-C₄, alkyle en C₁-C₄ substitué avec F, ou alcoxy en C₁-C₄ substitué avec F,
B₂ représente CR⁶, R⁶ représentant hydrogène ou alkyle en C₁-C₆,
B₃ représente CR⁷, R⁷ représentant alkyle en C₁-C₄ perhalogéné ou phényle,
B₄ représente CR⁸, R⁸ représentant hydrogène ou alkyle en C₁-C₆,
B₅ représente CR⁹, R⁹ représentant halogène, alcoxy en C₁-C₆ ou alkyle en C₁-C₆,
Q représente cyclopropyle ou 1-cyano-cyclopropyle.

9. Composé de la formule (I) selon la revendication 1, dans laquelle
W représente oxygène,
R¹⁰ représente hydrogène et R¹ représente hydrogène ou méthyle,
A₁ représente CR², R² représentant hydrogène,
A₂ représente azote,
A₃ représente CR³, R³ représentant alkyle en C₁-C₄ perfluoré (-CF₃, -C₂F₅, -C₃F₇, -C₄F₉), alkyle en C₁-C₄ (-CH₃, -C₂H₅, -C₃H₇, -C₄H₉), Cl, F, I ou alcoxy en C₁-C₄ (-O-CH₃, -O-C₂H₅, -O-C₃H₇, -O-C₄H₉), de préférence alkyle en C₁-C₄ (-CH₃, -C₂H₅, -C₃H₇,-C₄H₉), Cl ou alcoxy en C₁-C₄ (-O-CH₃, -O-C₂H₅, -O-C₃H₇, -O-C₄H₉), de manière davantage préférée méthyle, méthoxy ou Cl,
A₄ représente CR⁴, R⁴ représentant hydrogène,
B₁ représente CR⁵, et R⁵ représente F, Cl, Br, alkyle en C₁-C₄, alkyle en C₁-C₄ substitué avec F, ou alcoxy en C₁-C₄ substitué avec F, de préférence-CF₃, -C₂F₅, -C₃F₇, -C₄F₉, -CH₃, -C₂H₅, -C₃H₇, -C₄H₉, Cl, OCF₃, OCF₂H, ou OCFH₂, de manière davantage préférée Cl, CF₃, OCF₂H, OCF₃, méthyle ou éthyle,
B₂ représente CR⁶, R⁶ représentant hydrogène,
B₃ représente CR⁷, R⁷ représentant alkyle en C₁-C₄ perfluoré ou phényle, de préférence -C₃F₇ ou phényle,
B₄ représente CR⁸, R⁸ représentant hydrogène,
B₅ représente CR⁹, R⁹ représentant F, Cl, Br, I, alkyle en C₁-C₄, de préférence Cl, Br, I ou alkyle en C₁-C₄, de manière particulièrement préférée Cl, I, Br ou méthyle ou éthyle,
Q représente cyclopropyle, 1-cyano-cyclopropyle, alkyle en C₁-C₄ (-CH₃, -C₂H₅, -C₃H₇, -C₄H₉), ou alkyle en C₁-C₄ fluoré (p. ex. -CHF₂, CH₂F, -CF₃,-C₂H₄F, -C₂H₃F₂, -C₂H₂F₃, -C₂HF₄, -C₂F₅, -C₃F₇, -C₄F₉), de préférence méthyle, éthyle, propyle, C₂H₂F₃, cyclopropyle ou 1-cyano-cyclopropyle.

10. Compositions pharmaceutiques, contenant au moins un composé selon l'une quelconque des revendications 1 à 9.

11. Composés selon l'une quelconque des revendications 1 à 9 pour une utilisation en tant que médicament.

12. Composés selon l'une quelconque des revendications 1 à 9 pour une utilisation pour lutter contre des parasites sur des animaux.

13. Utilisation de composés selon l'une quelconque des revendications 1 à 9 pour la protection du matériel de reproduction de plantes, de préférence pour la protection de semences.
